(19) Europäisches Patentamt
European Patent Office
Office européen des brevets

(11) EP 2 224 912 B1

(12) **EUROPEAN PATENT SPECIFICATION**

(45) Date of publication and mention of the grant of the patent:
**11.05.2016 Bulletin 2016/19**

(21) Application number: **08866209.3**

(22) Date of filing: **31.12.2008**

(51) Int Cl.:
*A61K 9/127* (2006.01) *A61K 31/7088* (2006.01)
*C07D 317/28* (2006.01) *C07C 229/30* (2006.01)

(86) International application number:
**PCT/US2008/088676**

(87) International publication number:
**WO 2009/086558 (09.07.2009 Gazette 2009/28)**

(54) **IMPROVED COMPOSITIONS AND METHODS FOR THE DELIVERY OF NUCLEIC ACIDS**

VERBESSERTE ZUSAMMENSETZUNGEN UND VERFAHREN ZUR FREISETZUNG VON NUKLEINSÄUREN

COMPOSITIONS ET PROCÉDÉS AMÉLIORÉS POUR LA DÉLIVRANCE D'ACIDES NUCLÉIQUES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority:
**02.01.2008 US 18616**
**02.01.2008 US 18627**
**26.03.2008 US 39748**
**01.05.2008 US 49568**

(43) Date of publication of application:
**08.09.2010 Bulletin 2010/36**

(60) Divisional application:
**16163003.3**

(73) Proprietors:
- **TEKMIRA Pharmaceuticals Corporation**
  **Burnaby, BC V5J 5J8 (CA)**
- **THE UNIVERSITY OF BRITISH COLUMBIA**
  **Vancouver, British Columbia V6T 1Z3 (CA)**
- **Alnylam Pharmaceuticals**
  **Cambridge, Massachusetts 02142 (US)**

(72) Inventors:
- **HOPE, Michael, J.**
  **Vancouver**
  **British Columbia V6R 2K2 (CA)**
- **SEMPLE, Sean, C.**
  **Delta**
  **British Columbia V4E 3L5 (CA)**
- **CHEN, Jianxin**
  **Vancouver**
  **British Columbia V6S 1B7 (CA)**
- **MADDEN, Thomas, D.**
  **Vancouver**
  **British Columbia V6L 2A1 (CA)**
- **MUI, Barbara**
  **Vancouver**
  **British Columbia V5T 2G3 (CA)**
- **CULLIS, Pieter, R.**
  **Vancouver**
  **British Columbia V6R 1H4 (CA)**
- **CIUFOLINI, Marco, A.**
  **Vancouver**
  **British Columbia V6T 1Z1 (CA)**
- **WONG, Kim, F.**
  **Vancouver**
  **British Columbia V6R 2V5 (CA)**
- **MANOHARAN, Muthiah**
  **Weston**
  **Massachusetts 02493 (US)**
- **RAJEEV, Kallanthottathil, G.**
  **Wayland**
  **Massachusetts 01778 (US)**
- **SRINIVASULU, Masunu**
  **Vancouver, British Columbia V5W 3E7 (CA)**

(74) Representative: **Walker, Ross Thomson et al**
**Forresters**
**Skygarden**
**Erika-Mann-Strasse 11**
**80636 München (DE)**

(56) References cited:
**WO-A1-2005/026372**
**WO-A1-2006/074546**
**US-B1- 6 235 310**
**US-B2- 6 858 225**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- DAVID A. JAEGER, JANUSZ JAMROZIK, TIMOTHY G. GOLICH, MALGORZATA WEGRZYN CLENNAN, JAMSHID MOHEBALIAN: "Preparation and characterization of glycerol-based cleavable surfactants and derived vesicles", J. AM. CHEM. SOC., vol. 111, 1989, pages 3001-3006, XP002664188,
- ROBERT A. MOSS, SHOVAN GANGULI, YUKIHISA OKUMURA, TSUNEHISA FUJITA: "Relation of surfactant monomer structure to Flip-Flop dynamics in surface-differntiated synthetic bilayer membranes", J. AM. CHEM. SOC., vol. 112, 1990, pages 6391-6392, XP002664189,
- DONGLIANG LIU, JIANJUN HU, WEIHONG QIAO, ZONGSHI LI, SHUBIAO ZHANG, LUBO CHENG: "Synthesis of carbamate-linked lipids for gene delivery", BIOORGANIC & MEDICINAL CHEMISTRY LETTERS, vol. 15, 3 May 2005 (2005-05-03), pages 3147-3150, XP002664190,

## Description

STATEMENT REGARDING SEQUENCE LISTING

The Sequence Listing associated with this application is provided in text format in lieu of a paper copy, and is hereby incorporated by reference into the specification. The name of the text file containing the Sequence Listing is 480208_457PC_SEQUENCE_LISTING.txt. The text file is 8 KB, was created on December 31, 2008, and is being submitted electronically via EFS-Web.

BACKGROUND

Technical Field

The present invention relates to the field of therapeutic agent delivery using lipid particles. In particular, the present invention provides cationic lipids and lipid particles comprising these lipids, which are advantageous for the *in vivo* delivery of nucleic acids, as well as nucleic acid-lipid particle compositions suitable for *in vivo* therapeutic use. Additionally, the present invention provides methods of making these compositions, as well as methods of introducing nucleic acids into cells using these compositions, e.g., for the treatment of various disease conditions.

Description of the Related Art

Therapeutic nucleic acids include, e.g., small interfering RNA (siRNA), micro RNA (miRNA), antisense oligonucleotides, ribozymes, plasmids, and immune stimulating nucleic acids. These nucleic acids act via a variety of mechanisms. In the case of siRNA or miRNA, these nucleic acids can down-regulate intracellular levels of specific proteins through a process termed RNA interference (RNAi). Following introduction of siRNA or miRNA into the cell cytoplasm, these double-stranded RNA constructs can bind to a protein termed RISC. The sense strand of the siRNA or miRNA is displaced from the RISC complex providing a template within RISC that can recognize and bind mRNA with a complementary sequence to that of the bound siRNA or miRNA. Having bound the complementary mRNA the RISC complex cleaves the mRNA and releases the cleaved strands. RNAi can provide down-regulation of specific proteins by targeting specific destruction of the corresponding mRNA that encodes for protein synthesis.

The therapeutic applications of RNAi are extremely broad, since siRNA and miRNA constructs can be synthesized with any nucleotide sequence directed against a target protein. To date, siRNA constructs have shown the ability to specifically down-regulate target proteins in both *in vitro* and *in vivo* models. In addition, siRNA constructs are currently being evaluated in clinical studies.

However, two problems currently faced by siRNA or miRNA constructs are, first, their susceptibility to nuclease digestion in plasma and, second, their limited ability to gain access to the intracellular compartment where they can bind RISC when administered systemically as the free siRNA or miRNA. These double-stranded constructs can be stabilized by incorporation of chemically modified nucleotide linkers within the molecule, for example, phosphothioate groups. However, these chemical modifications provide only limited protection from nuclease digestion and may decrease the activity of the construct. Intracellular delivery of siRNA or miRNA can be facilitated by use of carrier systems such as polymers, cationic liposomes or by chemical modification of the construct, for example by the covalent attachment of cholesterol molecules [reference]. However, improved delivery systems are required to increase the potency of siRNA and miRNA molecules and reduce or eliminate the requirement for chemical modification.

Antisense oligonucleotides and ribozymes can also inhibit mRNA translation into protein. In the case of antisense constructs, these single stranded deoxynucleic acids have a complementary sequence to that of the target protein mRNA and can bind to the mRNA by Watson-Crick base pairing. This binding either prevents translation of the target mRNA and/or triggers RNase H degradation of the mRNA transcripts. Consequently, antisense oligonucleotides have tremendous potential for specificity of action (*i.e.,* down-regulation of a specific disease-related protein). To date, these compounds have shown promise in several *in vitro* and *in vivo* models, including models of inflammatory disease, cancer, and HIV (reviewed in Agrawal, Trends in Biotech. 14:376-387 (1996)). Antisense can also affect cellular activity by hybridizing specifically with chromosomal DNA. Advanced human clinical assessments of several antisense drugs are currently underway. Targets for these drugs include the bcl2 and apolipoprotein B genes and mRNA products.

Immune-stimulating nucleic acids include deoxyribonucleic acids and ribonucleic acids. In the case of deoxyribonucleic acids, certain sequences or motifs have been shown to illicit immune stimulation in mammals. These sequences or motifs include the CpG motif, pyrimidine-rich sequences and palindromic sequences. It is believed that the CpG motif in deoxyribonucleic acids is specifically recognized by an endosomal receptor, toll-like receptor 9 (TLR-9), which then triggers both the innate and acquired immune stimulation pathway. Certain immune stimulating ribonucleic acid sequences have also been reported. It is believed that these RNA sequences trigger immune activation by binding to toll-like receptors 6 and 7 (TLR-6 and TLR-7). In addition, double-stranded RNA is also reported to be immune stimulating and is believe to activate via binding to TLR-3.

One well known problem with the use of therapeutic nucleic acids relates to the stability of the phosphodiester internucleotide linkage and the susceptibility of this linker to nucleases. The presence of exonucleases and endonucleases in serum results in the rapid digestion of nucleic acids possessing phosphodiester linkers and, hence, therapeutic nucleic acids can have very short half-lives in the presence of serum or within cells. (Zelphati, O., et al., Antisense. Res. Dev. 3:323-338 (1993); and Thierry, A.R., et al., pp147-161 in Gene Regulation: Biology of Antisense RNA and DNA (Eds. Erickson, RP and Izant, JG; Raven Press, NY (1992)). Therapeutic nucleic acid being currently being developed do not employ the basic phosphodiester chemistry found in natural nucleic acids, because of these and other known problems.

This problem has been partially overcome by chemical modifications that reduce serum or intracellular degradation. Modifications have been tested at the internucleotide phosphodiester bridge (*e.g.,* using phosphorothioate, methylphosphonate or phosphoramidate linkages), at the nucleotide base (*e.g.,* 5-propynyl-pyrimidines), or at the sugar (*e.g.,* 2'-modified sugars) (Uhlmann E., et al. Antisense: Chemical Modifications. Encyclopedia of Cancer, Vol. X., pp 64-81 Academic Press Inc. (1997)). Others have attempted to improve stability using 2'-5' sugar linkages *(see, e.g.,* US Pat. No. 5,532,130). Other changes have been attempted. However, none of these solutions have proven entirely satisfactory, and *in vivo* free therapeutic nucleic acids still have only limited efficacy.

In addition, as noted above relating to siRNA and miRNA, problems remain with the limited ability of therapeutic nucleic acids to cross cellular membranes (see, Vlassov, et al., Biochim. Biophys. Acta 1197:95-1082 (1994)) and in the problems associated with systemic toxicity, such as complement-mediated anaphylaxis, altered coagulatory properties, and cytopenia (Galbraith, et al., Antisense Nucl. Acid Drug Des. 4:201-206 (1994)).

To attempt to improve efficacy, investigators have also employed lipid-based carrier systems to deliver chemically modified or unmodified therapeutic nucleic acids. In Zelphati, O and Szoka, F.C., J. Contr. Rel. 41:99-119 (1996), the authors refer to the use of anionic (conventional) liposomes, pH sensitive liposomes, immunoliposomes, fusogenic liposomes, and cationic lipid/antisense aggregates. Similarly siRNA has been administered systemically in cationic liposomes, and these nucleic acid-lipid particles have been reported to provide improved down-regulation of target proteins in mammals including non-human primates (Zimmermann et al., Nature 441: 111-114 (2006)).

In spite of this progress, there remains a need in the art for improved lipid-therapeutic nucleic acid compositions that are suitable for general therapeutic use. Preferably, these compositions would encapsulate nucleic acids with high-efficiency, have high drug:lipid ratios, protect the encapsulated nucleic acid from degradation and clearance in serum, be suitable for systemic delivery, and provide intracellular delivery of the encapsulated nucleic acid. In addition, these lipid-nucleic acid particles should be well-tolerated and provide an adequate therapeutic index, such that patient treatment at an effective dose of the nucleic acid is not associated with significant toxicity and/or risk to the patient. The present invention provides such compositions, methods of making the compositions, and methods of using the compositions to introduce nucleic acids into cells, including for the treatment of diseases.

WO 2006/074546 A1 discloses lipid-based formulations for delivering, for example nucleic acid-lipid particles comprising an interfering RNA molecule to a cell, and methods of making, delivering, and administrating such lipid-based formulations.

According to the present invention, there is provided an amino lipid having the following structure (I):

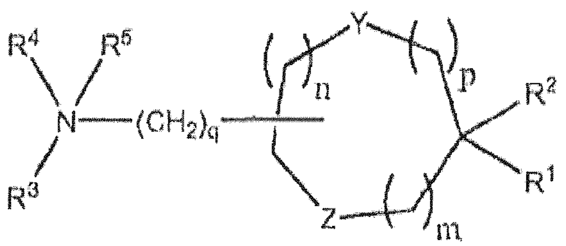

(I)

wherein

$R^1$ and $R^2$ are either the same or different and independently substituted $C_{12}$-$C_{24}$ alkyl, optionally substituted $C_{12}$-$C_{24}$ alkenyl, optionally substituted $C_{12}$-$C_{24}$ alkynyl, or optionally substituted $C_{12}$-$C_{24}$ acyl;

$R^3$ and $R^4$ are either the same or different and independently optionally substituted $C_1$-$C_6$ alkyl, optionally substituted $C_1$-$C_6$ alkenyl, or optionally substituted $C_1$-$C_6$ alkynyl or $R^3$ and $R^4$ may join to form an optionally substituted heterocyclic ring of 4 to 6 carbon atoms and 1 or 2 heteroatoms chosen from nitrogen end oxygen;

$R^5$ is either absent or present and when present is hydrogen $C_1$-$C_6$ alkyl; m, n and p are either the same or different

and independently either 0 or 1 with the proviso that m, n, and p are not simultaneously 0;
q is 0, 1, 2, 3, or 4; and
Y and Z are either the same or different and independently O, S or NH; wherein the optional substituents are chosen from oxo, halogen, heterocycle, -CN, $-OR^x$, $-NR^xR^y$, $-NR^xC(=O)R^y$, $-NR^xSO_2R^y$, $-C(=O)R^x$, $-C(=O)OR^x$, $-C(=O)NR^xR^y$,$-SO_nR^x$ and $-SO_nNR^xR^y$ wherein n is 0, 1 or 2, $R^x$ and $R^y$ are the same or different and independently hydrogen, alkyl or heterocycle, and each or said alkyl and heterocycle substituents may be further substituted with one or more of oxo, halogen, -OH, -CN, alkyl, $-OR^x$, heterocycle, $-NR^xR^y$, $NR^xC(=O)R^y$, $NR^xSO_2R^y$, $-C(=O)R^x$, $-C(=O)OR^x$,$-C(=O)NR^xR^y$, $-SO_nR^x$ and $-SO_nNR^xR^y$;

wherein halogen is chosen from fluoro, chloro, bromo and iodo.

Preferably, the amino lipid has the structure:

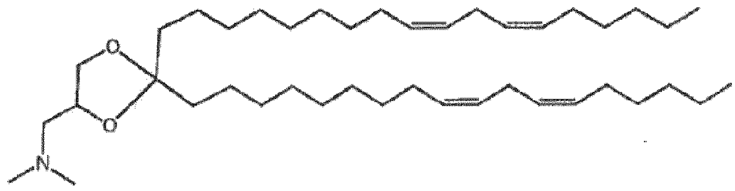

(DLin-K-DMA).

Conveniently, the invention provides a lipid particle comprising an amino lipid, preferably the amino lipid has the structure:

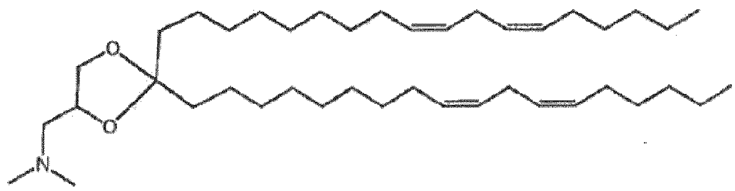

Advantageously, the particle further comprises a neutral lipid and a lipid capable of reducing particle aggregation, wherein preferably the particle further comprises essentially of:

(i) DLin-K-DMA;
(ii) a neutral lipid selected from DSPC, POPC, DOPE, and SM;
(iii) cholesterol; and
(iv) PEG-S-DMG, PEG-C-DOMG or PEG-DMA;

in a molar ratio of about 20-60% DLin-K-DMA : 5-25% neutral lipid : 25-55% Chol: 0.5-1 5% PEG-S-DMG, PEG-C-DOMG or PEG-DMA.

Conveniently, the lipid particle comprises:

(i) one or more amino lipids;
(ii) one or more neutral lipids selected from DSPC, POPC, DOPE, and SM;
(iii) cholesterol; and
(iv) PEG-C-DOMG,

in a molar ratio of about 20-60% amino lipid : 5-25% neutral lipid : 25-55% cholesterol : 0.5-15% PEG-C-DOMG, wherein the amino lipid is an amino lipid of the invention.

Advantageously, the lipid particle further comprises a therapeutic agent, wherein preferably the therapeutic agent is a nucleic acid, which is further preferably a plasmid, an imunostimulatory oligonucleotide, or is selected from the group constisting of: a siRNA, a microRNA, an antisense oligonucleotide, and a ribozyme, preferably a siRNA.

According to the present invention there is also provided a pharmaceutical composition comprising a lipid particle and a pharmaceutically acceptable excipient, carrier, or diluent.

Preferably, the pharmaceutical composition is for use in therapy.

Conveniently, the pharmaceutical composition for use in a method of treating a disease or disorder characterized

by overexpression of a poypeptide in a subject, the method comprising providing to the subject the pharmaceutical composition, wherein the therapeutic agent is selected from a siRNA, a microRNA, an antisense oligonucleotide, a plasmid capable of expressing a siRNA, a microRNA, and an antisense oligonucleotide, and wherein the siRNA, microRNA, or antisense RNA comprises a polynucleotide that specifically binds to a polynucleotide that encodes the polypeptide, or a complement thereof.

Preferably, the pharmaceutical composition for use in a method of treating a disease or disorder characterized by underexpression of a polypeptide in a subject, the method comprising providing to the subject the pharmaceutical composition, wherein the therapeutic agent is a pasmid that encodes the polypeptide or a functional variant or fragment thereof.

Advantageously, the pharmaceutical composition for use in a method of inducing an immune response in a subject comprising providing to the subject the pharmaceutical composition, wherein the therapeutic agent is an immunostimulatory oligonucleotide.

Conveniently, the pharmaceutical composition is suitable to be provided to the patient in combination with a vaccine or antigen.

According to the present invention there is also provided a vaccine comprising the lipid particle,wherein the therapeutic agent is an immunostimulatory oligonucleotide and an antigen associated with a disease or pathogen.

Advantageously, said antigen is a tumor antigen, a viral antigen, a bacterial antigen, or a parasitic antigen.

Preferably, the lipid particle is for use in therapy, preferably for use in modulating the expression of a polypeptide by a cell, wherein optionally and preferably:

a) the therapeutic agent is selected from a siRNA, a microRNA, an antisense oligonucleotide, a plasmid capable of expressing a siRNA, a microRNA, and an antisense oligonucleotide, and wherein the siRNA, microRNA, or antisense RNA comprises a polynucleotide that specifically binds to a polynucleotide that encodes the polypeptide, or a complement thereof, such that the expression of the polypeptide is reduced; or

b) the nucleic acid is a plasmid that encodes the polypeptide or a functional variant or fragment thereof, such that expression of the polypeptide or the functional variant or fragment thereof is increased.

The present invention further includes, in other related embodiments, a method of modulating the expression of a polypeptide by a cell, comprising providing to a cell a lipid particle or pharmaceutical composition of the present invention. In certain embodiments, the lipid particle comprises, consists essentially of, or consists of one or more of the above amino lipids of the present invention, one or more neutral lipids, one or more lipids capable of reducing particle aggregation, and one or more siRNAs capable of reducing the expression of a selected polypeptide. In one particular embodiment, the lipid particle consists essentially of or consists of: (i) DLin-K-DMA; (ii) a neutral lipid selected from DSPC, POPC, DOPE, and SM; (iii) cholesterol; and (iv) PEG-S-DMG, PEG-C-DOMG or PEG-DMA, in a molar ratio of about 20-60% DLin-K-DMA:5-25% neutral lipid:25-55% Chol:0.5-15% PEG-S-DMG, PEG-C-DOMG or PEG-DMA. In particular embodiments, the lipid paticle comprises a therapeutic agent selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof, such that the expression of the polypeptide is reduced. In another embodiment, the nucleic acid is a plasmid that encodes the polypeptide or a functional variant or fragment thereof, such that expression of the polypeptide or the functional variant or fragment thereof is increased.

In yet a further related embodiment, the present invention includes a composition for use in a method of treating a disease or disorder characterized by overexpression of a polypeptide in a subject, comprising providing to the subject a lipid particle or pharmaceutical composition of the present invention, wherein the therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof.

In another related embodiment, the present invention includes a composition for use in a method of treating a disease or disorder characterized by underexpression of a polypeptide in a subject, comprising providing to the subject the pharmaceutical composition of the present invention, wherein the therapeutic agent is a plasmid that encodes the polypeptide or a functional variant or fragment thereof.

In a further embodiment, the present invention includes a composition for use in a method of inducing an immune response in a subject, comprising providing to the subject the pharmaceutical composition of the present invention, wherein the therapeutic agent is an immunostimulatory oligonucleotide. In particular embodiments, the pharmaceutical composition is provided to the patient in combination with a vaccine or antigen.

In a related embodiment, the present invention includes a vaccine comprising the lipid particle of the present invention and an antigen associated with a disease or pathogen. In one embodiment, the lipid particle comprises an

immunostimulatory nucleic acid or oligonucleotide. In a particular embodiment, the antigen is a tumor antigen. In another embodiment, the antigen is a viral antigen, a bacterial antigen, or a parasitic antigen.

The present invention further includes methods of preparing the lipid particles and pharmaceutical compositions of the present invention, as well as kits usedful in the preparation of these lipid particle and pharmaceutical compositions.

The present invention also includes a lipid particle comprising: a cationic lipid or an amino lipid, including any of those of the present invention; a neutral lipid, which may optionally be selected from DSPC, POPC, DOPE, and SM; cholesterol; and PEG-C-DOMG, in a molar ratio of about 20-60% amino lipid:5-25% neutral lipid:25-55% Chol:0.5-15% PEG-C-DOMG. In one embodiment, the lipid paticle comprises the amino lipid DLin-K-DMA. In related embodiments, the lipid particle further comprises a therapeutic agent. In one embodiment, the

## BRIEF DESCRIPTION OF THE SEVERAL VIEWS OF THE DRAWINGS

Figure 1 illustrates the effects of various ethanol concentrations on nucleic acid encapsulation and resulting vesicle stability. Figure 1A is a graph showing the amount of encapsulation of a 16mer phosphodiester oligonucleotide in DLinDMA/DSPC/CH/PEG-S-DMG (40:10:48:2 mole ratio) vesicles in the presence of 32, 34, and 36% ethanol. Figure 1 B is a bar graph illustrating vesicle size before loading and 30 min and 60 min after loading in 32, 34, and 36% ethanol.

Figure 2 depicts the effect of time and temperature on nucleic acid encapsulation. Figure 2A is a graph showing the amount of encapsulation of a 16mer phosphodiester oligonucleotide in DLinDMA/DSPC/CH/PEG-S-DMG vesicles at 30° C and 40° C at the indicated incubation time points. Figure 2B is a bar graph showing vesicle size before incubation and after 15 min, 30 min, and 60 min of incubation at 40° C.

Figure 3 is a graph depicting the ability of various lipid formulations of nucleic acid-lipid particles containing Factor VII siRNA to reduce Factor VII expression *in vivo.* Factor VII levels following treatment with various Factor VII siRNA dosages in particles comprising either DLin-K-DMA, DLinDMA, or DLinDAP are shown.

Figure 4 is a graph comparing the amount of residual FVII following administration of various concentrations of DLin-DMA lipid particle formulations comprising the different indicated PEG-lipids.

Figure 5 is a graph comparing the amount of residual FVII following administration of various concentrations of DLin-K-DMA lipid particle formulations comprising the different indicated PEG-lipids.

Figure 6 is a graph depicting the serum ALT levels present following administration of the indicated lipid formulations at various siRNA dosages.

Figure 7A and Figure 7B demonstrate the relative tolerability of DLin-K-DMA lipid particles comprising either PEG-C-DOMG or PEG-S-DMG. Figure 7A shows serum ALT levels following treatment with the lipid particles at various siRNA dosages, and Figure 7B shows the change in weight of animals following treatment with the lipid particles at various siRNA dosages.

## DETAILED DESCRIPTION

The present invention is based, in part, upon the discovery of cationic lipids that provide advantages when used in lipid particles for the *in vivo* delivery of an active agent, such as a therapeutic agent. In particular, as illustrated by the accompanying Examples, the present invention provides nucleic acid-lipid particle compositions comprising a cationic lipid according to the present invention that provide increased activity of the nucleic acid and improved tolerability of the compositions *in vivo,* resulting in a significant increase in therapeutic index as compared to lipid-nucleic acid particle compositions previously described. Additionally, compositions and methods of use are disclosed that provided for amelioration of the toxicity observed with certain therapeutic nucleic acid-lipid particles.

In certain embodiments, the present invention specifically provides for improved compositions for the delivery of siRNA molecules. It is shown herein that these compositions are effective in down-regulating the protein levels and/or mRNA levels of target proteins. Furthermore, it is shown that the activity of these improved compositions is dependent on the presence of a certain cationic lipids and that the molar ratio of cationic lipid in the formulation can influence activity.

The lipid particles and compositions of the present invention may be used for a variety of purposes, including the delivery of associated or encapsulated therapeutic agents to cells, both *in vitro* or *in vivo.* Accordingly, the present invention provides methods of treating diseases or disorders in a subject in need thereof, by contacting the subject with a lipid particle of the present invention associated with a suitable therapeutic agent.

As described herein, the lipid particles of the present invention are particularly useful for the delivery of nucleic acids, including, *e.g.,* siRNA molecules and plasmids. Therefore, the lipid particles and compositions of the present invention may be used to modulate the expression of target genes and proteins both in vitro and in vivo by contacting cells with a lipid particle of the present in vention associated with a nucleic acid that reduces target gene expression

(*e.g.,* a siRNA) or a nucleic acid that may be used to increase expression of a desired protein (*e.g.,* a plasmid encoding the desired protein).

Various exemplary embodiments of the cationic lipids of the present invention, as well as lipid particles and compositions comprising the same, and their use to deliver therapeutic agents and modulate gene and protein expression are described in further detail below.

A. Amino Lipids

The present invention provides novel amino lipids that are advantageously used in lipid particles of the present invention for the *in vivo* delivery of therapeutic agents to cells, including but not limited to amino lipids having the following structures, including (R) and (S) enantiomers thereof:

**1,2-Dilinoleylcarbamoyloxy-3-dimethylaminopropane (DLin-C-DAP)**

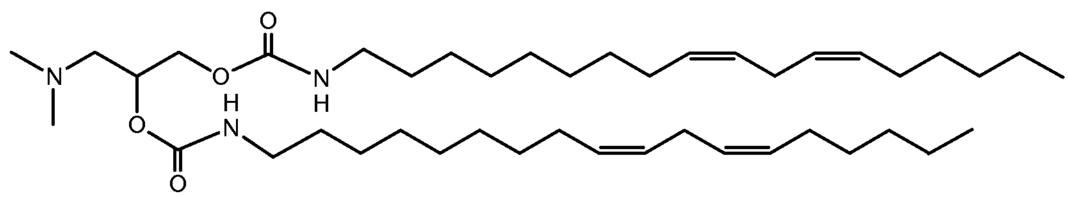

$C_{43}H_{79}N_3O_4$
Exact Mass: 701.61
Mol. Wt.: 702.11
C, 73.56; H, 11.34; N, 5.98; O, 9.12

**1,2-Dilinoleyoxy-3-(dimethylamino)acetoxypropane (DLin-DAC)**

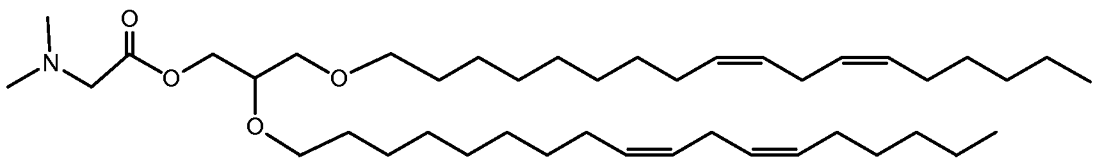

$C_{43}H_{79}NO_4$
Exact Mass: 673.60
Mol. Wt.: 674.09
C, 76.62; H, 11.81; N, 2.08; O, 9.49

**1,2-Dilinoleyoxy-3-morpholinopropane (DLin-MA)**

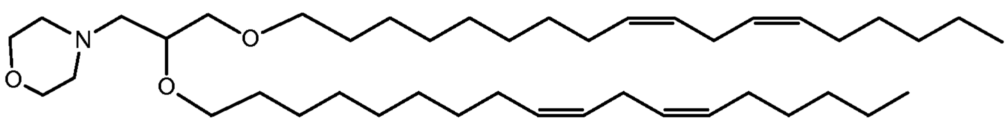

$C_{43}H_{79}NO_3$
Exact Mass: 657.61
Mol. Wt.: 658.09
C, 78.48; H, 12.10; N, 2.13; O, 7.29

**1,2-Dilinoleoyl-3-dimethylaminopropane (DLinDAP)**

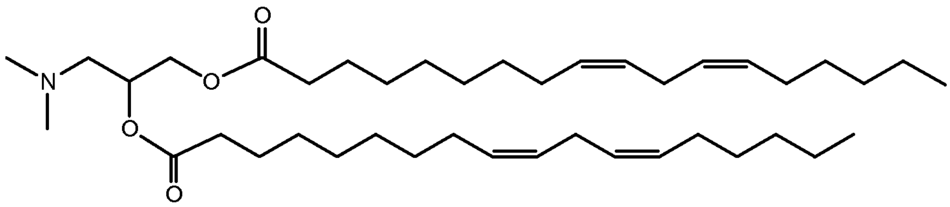

$C_{41}H_{73}NO_4$
Exact Mass: 643.55
Mol. Wt.: 644.02
C, 76.46; H, 11.43; N, 2.17; O, 9.94

**1,2-Dilinoleylthio-3-dimethylaminopropane (DLin-S-DMA)**

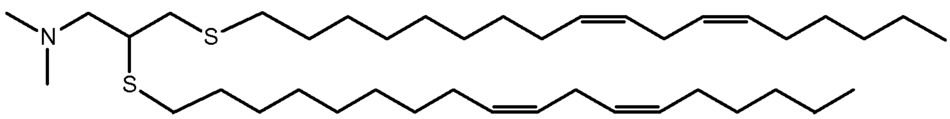

$C_{41}H_{77}NS_2$
Exact Mass: 647.55
Mol. Wt.: 648.19
C, 75.97; H, 11.97; N, 2.16; S, 9.89

**1-Linoleoyl-2-linoleyloxy-3-dimethylaminopropane (DLin-2-DMAP)**

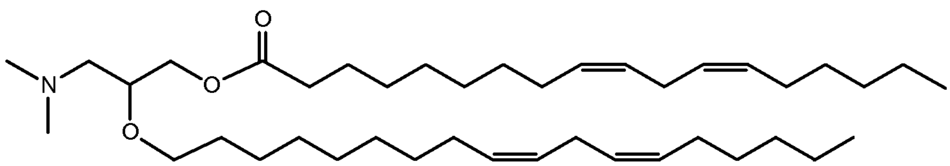

$C_{41}H_{75}NO_3$
Exact Mass: 629.57
Mol. Wt.: 630.04
C, 78.16; H, 12.00; N, 2.22; O, 7.62

**1,2-Dilinoleyloxy-3-trimethylaminopropane chloride salt (DLin-TMA.Cl)**

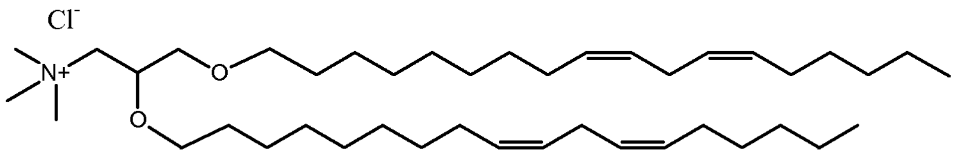

$C_{42}H_{80}ClNO_2$
Exact Mass: 665.59
Mol. Wt.: 666.54
C, 75.68; H, 12.10; Cl, 5.32; N, 2.10; O, 4.80

**1,2-Dilinoleoyl-3-trimethylaminopropane chloride salt (DLin-TAP.Cl)**

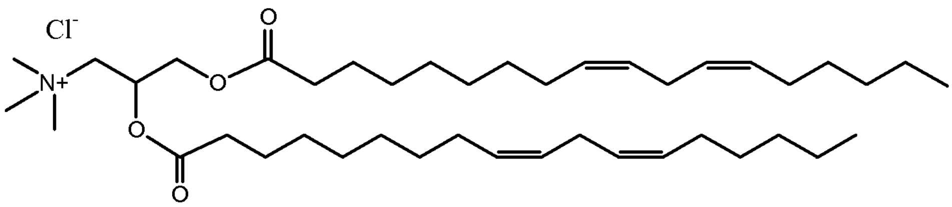

$C_{42}H_{76}ClNO_4$
Exact Mass: 693.55
Mol. Wt.: 694.51
C, 72.63; H, 11.03; Cl, 5.10; N, 2.02; O, 9.21

**1,2-Dilinoleyloxy-3-(N-methylpiperazino)propane (DLin-MPZ)**

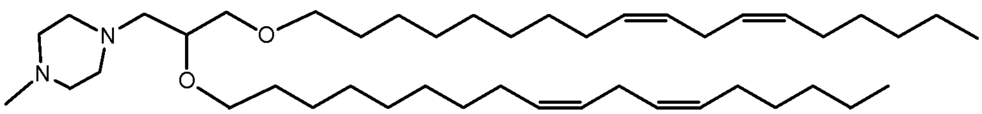

$C_{44}H_{82}N_2O_2$
Exact Mass: 670.64
Mol. Wt.: 671.13
C, 78.74; H, 12.32; N, 4.17; O, 4.77

**3-(N,N-Dilinoleylamino)-1,2-propanediol (DLinAP)**

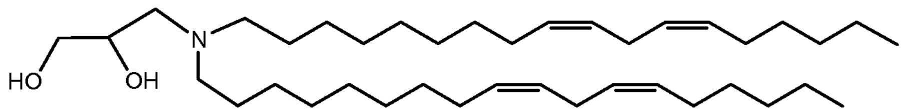

$C_{39}H_{73}NO_2$
Exact Mass: 587.56
Mol. Wt.: 588.00
C, 79.66; H, 12.51; N, 2.38; O, 5.44

**3-(N,N-Dioleylamino)-1,2-propanedio (DOAP)**

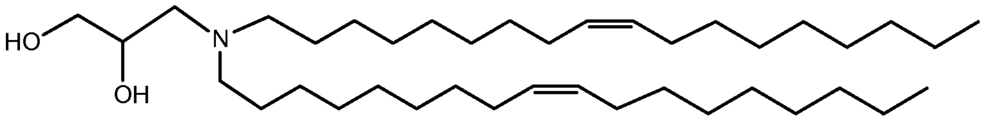

$C_{39}H_{77}NO_2$
Exact Mass: 591.60
Mol. Wt.: 592.03
C, 79.12; H, 13.11; N, 2.37; O, 5.40

**1,2-Dilinoleyloxo-3-(2-N,N-dimethylamino)ethoxypropane (DLin-EG-DMA)**

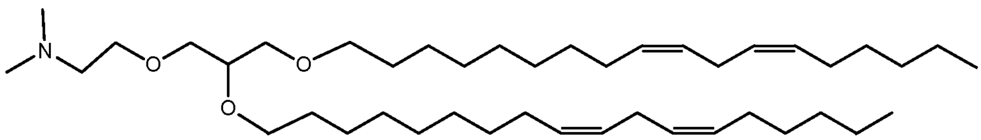

$C_{43}H_{81}NO_3$
Exact Mass: 659.62
Mol. Wt.: 660.11
C, 78.24; H, 12.37; N, 2.12; O, 7.27

**2,2-Dilinoleyl-4-dimethylaminomethyl-[1,3]-dioxolane (DLin-K-DMA)**

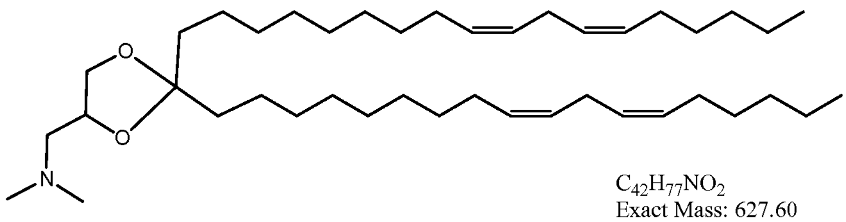

$C_{42}H_{77}NO_2$
Exact Mass: 627.60
Mol. Wt.: 628.07
C, 80.32; H, 12.36; N, 2.23; O, 5.09

In one embodiment of the invention, the amino lipid has the following structure (I):

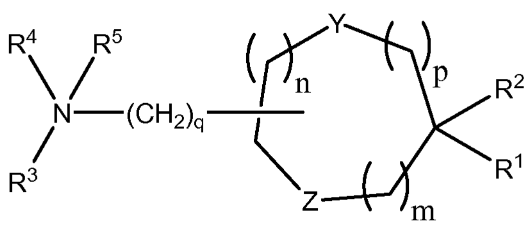

(I)

or salts thereof, wherein

$R^1$ and $R^2$ are either the same or different and independently optionally substituted $C_{12}$-$C_{24}$ alkyl, optionally substituted $C_{12}$-$C_{24}$ alkenyl, optionally substituted $C_{12}$-$C_{24}$ alkynyl, or optionally substituted $C_{12}$-$C_{24}$ acyl;

$R^3$ and $R^4$ are either the same or different and independently optionally substituted $C_1$-$C_6$ alkyl, optionally substituted $C_1$-$C_6$ alkenyl, or optionally substituted $C_1$-$C_6$ alkynyl or $R^3$ and $R^4$ may join to form an optionally substituted heterocyclic ring of 4 to 6 carbon atoms and 1 or 2 heteroatoms chosen from nitrogen and oxygen;

$R^5$ is either absent or hydrogen or $C_1$-$C_6$ alkyl to provide a quaternary amine;

m, n, and p are either the same or different and independently either 0 or 1 with the proviso that m, n, and p are not simultaneously 0;

q is 0, 1, 2, 3, or 4; and

Y and Z are either the same or different and independently O, S, or NH.

"Alkyl" means a straight chain or branched, noncyclic or cyclic, saturated aliphatic hydrocarbon containing from 1 to 24 carbon atoms. Representative saturated straight chain alkyls include methyl, ethyl, n-propyl, n-butyl, n-pentyl, n-hexyl, and the like; while saturated branched alkyls include isopropyl, *sec*-butyl, isobutyl, *tert*-butyl, isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like.

"Alkenyl" means an alkyl, as defined above, containing at least one double bond between adjacent carbon atoms. Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Acyl" means any alkyl, alkenyl, or alkynyl wherein the carbon at the point of attachment is substituted with an oxo group, as defined below. For example, -C(=O)alkyl, -C(=O)alkenyl, and -C(=O)alkynyl are acyl groups.

"Heterocycle" means a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined below. Heterocycles include morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" means that, when substituted, at least one hydrogen atom is replaced with a substituent. In the case of an oxo substituent (=O) two hydrogen atoms are replaced. In this regard, substituents include oxo, halogen, heterocycle, -CN, $-OR^x$, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^Y$, wherein n is 0, 1 or 2, $R^x$ and $R^y$ are the same or different and independently hydrogen, alkyl or heterocycle, and each of said alkyl and heterocycle substituents may be further substituted with one or more of oxo, halogen, -OH, -CN, alkyl, $-OR^x$, heterocycle, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^Y$.

"Halogen" means fluoro, chloro, bromo and iodo.

In some embodiments, the methods of the invention may require the use of protecting groups. Protecting group methodology is well known to those skilled in the art *(see, for example,* PROTECTIVE GROUPS IN ORGANIC SYNTHESIS, Green, T.W. et. al., Wiley-Interscience, New York City, 1999). Briefly, protecting groups within the context of this invention are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

The compounds of the present invention may be prepared by known organic synthesis techniques, including the methods described in more detail in the Examples. In general, the compounds of structure (I) above may be made by the following Reaction Schemes 1 or 2, wherein all substituents are as defined above unless indicated otherwise.

Compounds of structure (I) wherein m is 1 and p is 0 can be prepared according to Reaction Scheme 1. Ketone **1** and Grignard reagent **2,** wherein P is an alcohol protecting group such as trityl, can be purchased or prepared according to methods known to those of ordinary skill in the art. Reaction of **1** and **2** yields alcohol **3.** Deprotection of **3,** for example by treatment with mild acid, followed by bromination with an appropriate bromination reagent, for example phosphorous tribromide, yields **4** and **5** respectively. Treatment of bromide **5** with **6** yields the heterocyclic compound **7.** Treatment

of **7** with amine **8** then yields a compound of structure (I) wherein m is 1 and $R^5$ is absent **(9).** Further treatement of **9** with chloride **10** yields compounds of structure (I) wherein m is 1 and $R^5$ is present.

## 1. Reaction Scheme 1

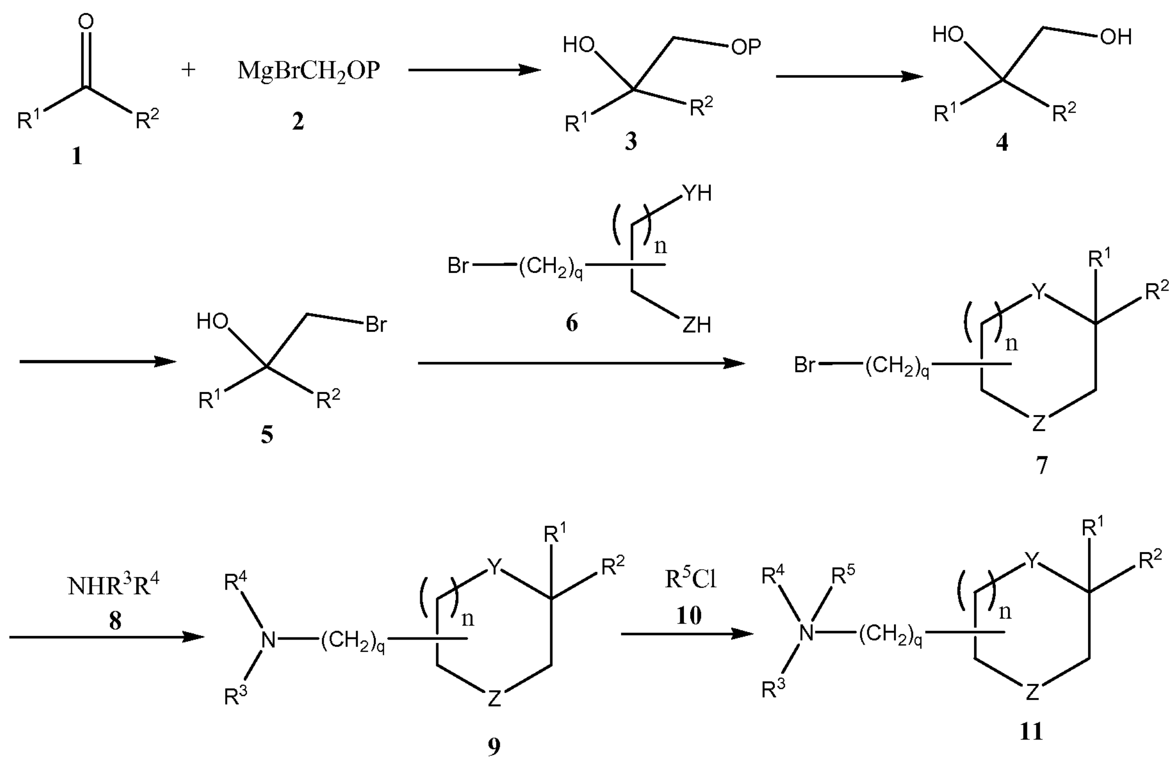


Compounds of structure (I) wherein m and p are 0 can be prepared according to Reaction Scheme 2. Ketone **1** and bromide **6** can be purchased or prepared according to methods known to those of ordinary skill in the art. Reaction of **1** and **6** yields heterocycle **12.** Treatment of **12** with amine **8** yields compounds of structure (I) wherein m is 0 and $R^5$ is absent **(13).** Further treatment of **13** with **10** produces compounds of structure (I) wherein w is 0 and $R^5$ is present.

## 2. Reaction Scheme 2

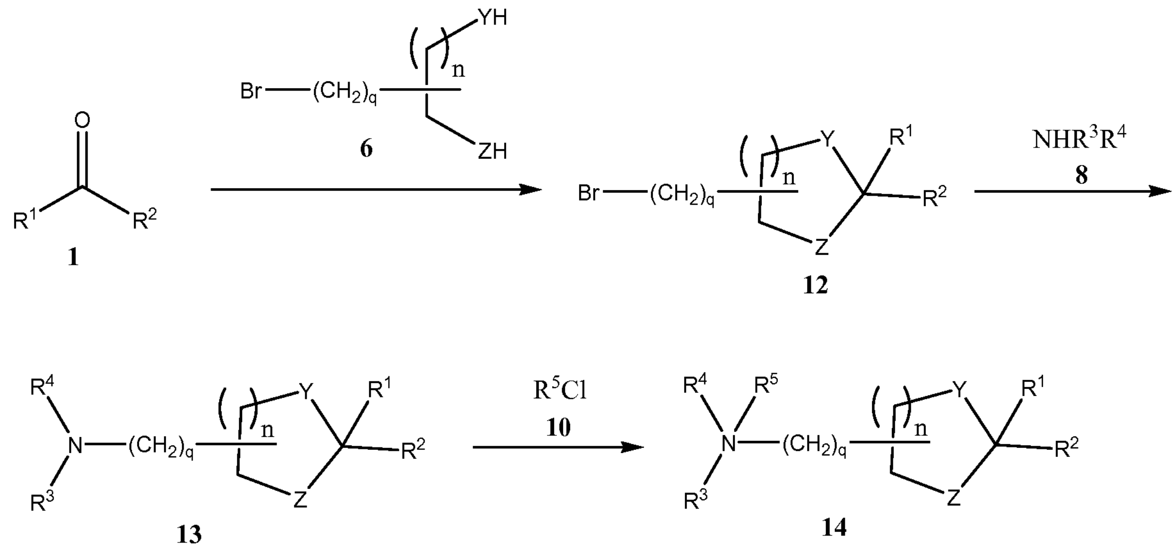

In certain embodiments where m and p are 1 and n is 0, compounds of this invention can be prepared according to Reaction Scheme 3. Compounds **12** and **13** can be purchased or prepared according to methods know to those of ordinary skill in the art. Reaction of **12** and **13** yields a compound of structure (I) where $R^5$ is absent **(14).** In other embodiments where $R^5$ is present, **13** can be treated with **10** to obtain compounds of structure **15.**

3. Reaction Scheme 3

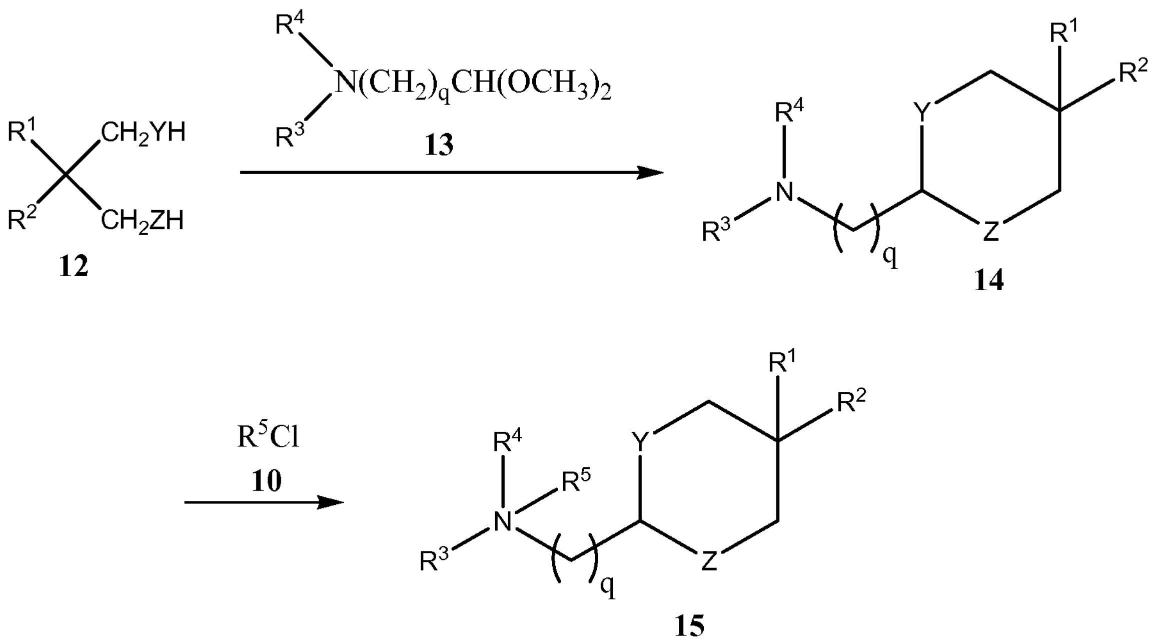


12 14 15

In certain other embodiments where either m or p is 1 and n is 0, compounds of this invention can be prepared according to Reaction Scheme 4. Compound **16** can be purchased or prepared according to methods know to those of ordinary skill in the art and reacted with **13** to yield a compound of structure (I) where $R^5$ is absent **(17).** Other embodiments of structure (I) where $R^5$ is present can be prepared by treatment of **17** with **10** to yield compounds of structure **18.**

4. Reaction Scheme 4

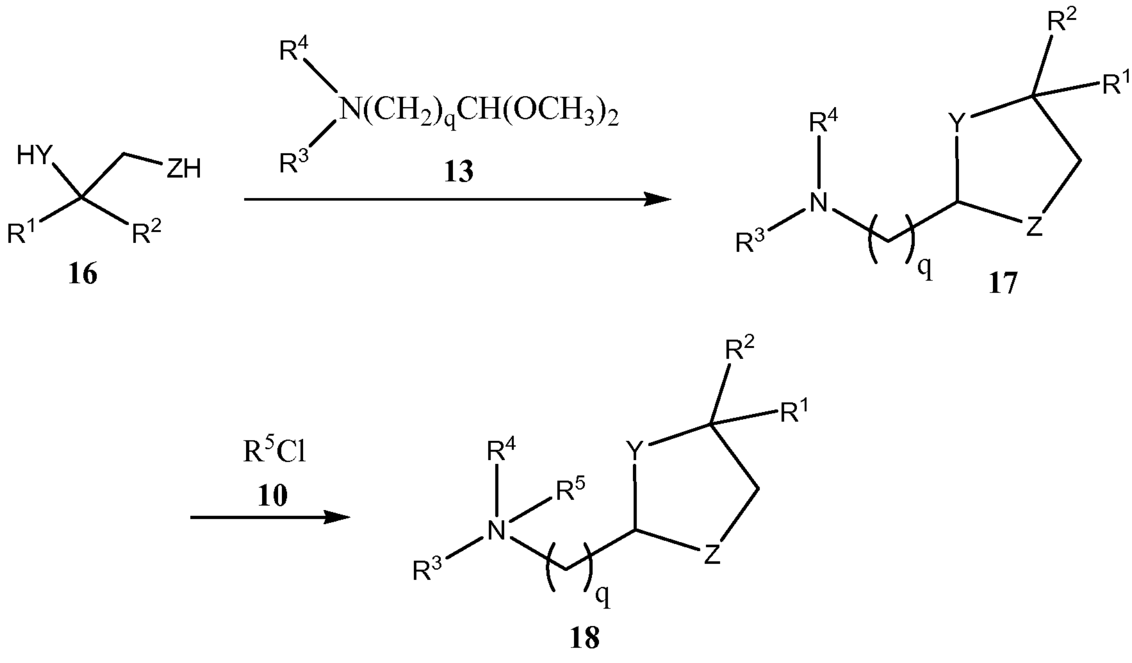


16 17 18

In certain specific embodiments of structure (I) where n is 1 and m and p are 0, compounds of this invention

can be prepared according to Reaction Scheme 5. Compound **19** can be purchased or prepared according to methods known to those of ordinary skill in the art. Reaction of **19** with formaldehyde followed by removal of an optional alcohol protecting group (P), yields alcohol **20.** Bromination of **20** followed by treatment with amine **8** yields **22.** Compound **22** can then be treated with n-butyl lithium and $R^1I$ followed by further treatment with n-butyl lithium and $R^2I$ to yield a compound of structure (I) where $R^5$ is absent **(23).** Further treatment of **23** with **10** yields a compound of structure (I) where $R^5$ is present **(24).**

5. Reaction Scheme 5

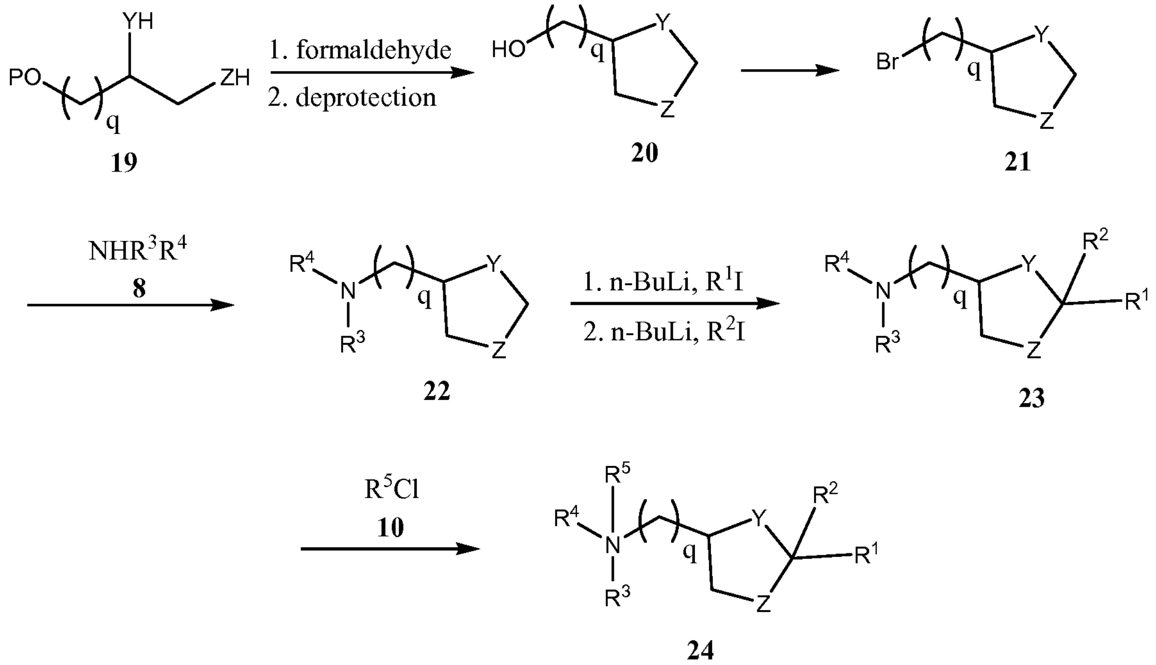


In particular embodiments, the amino lipids are of the present invention are cationic lipids. As used herein, the term "amino lipid" is meant to include those lipids having one or two fatty acid or fatty alkyl chains and an amino head group (including an alkylamino or dialkylamino group) that may be protonated to form a cationic lipid at physiological pH.

Other amino lipids would include those having alternative fatty acid groups and other dialkylamino groups, including those in which the alkyl substituents are different (*e.g.,* N-ethyl-N-methylamino-, N-propyl-N-ethylamino-and the like). For those embodiments in which $R^{11}$ and $R^{12}$ are both long chain alkyl or acyl groups, they can be the same or different. In general, amino lipids having less saturated acyl chains are more easily sized, particularly when the complexes must be sized below about 0.3 microns, for purposes of filter sterilization. Amino lipids containing unsaturated fatty acids with carbon chain lengths in the range of $C_{14}$ to $C_{22}$ are preferred. Other scaffolds can also be used to separate the amino group and the fatty acid or fatty alkyl portion of the amino lipid. Suitable scaffolds are known to those of skill in the art.

In certain embodiments, amino or cationic lipids of the present invention have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of the lipid be present in the charged or neutral form. Lipids that have more than one protonatable or deprotonatable group, or which are zwiterrionic, are not excluded from use in the invention.

In certain embodiments, protonatable lipids according to the invention have a pKa of the protonatable group in the range of about 4 to about 11. Most preferred is pKa of about 4 to about 7, because these lipids will be cationic at a lower pH formulation stage, while particles will be largely (though not completely) surface neutralized at physiological pH around pH 7.4. One of the benefits of this pKa is that at least some nucleic acid associated with the outside surface of the particle will lose its electrostatic interaction at physiological pH and be removed by simple dialysis; thus greatly reducing the particle's susceptibility to clearance.

B. Lipid Particles

The present invention also provides lipid particles comprising one or more of the amino lipids described above. Lipid particles include, but are not limited to, liposomes. As used herein, a liposome is a structure having lipid-containing membranes enclosing an aqueous interior. Liposomes may have one or more lipid membranes. The invention contemplates both single-layered liposomes, which are referred to as unilamellar, and multi-layered liposomes, which are referred to as multilamellar. When complexed with nucleic acids, lipid particles may also be lipoplexes, which are composed of cationic lipid bilayers sandwiched between DNA layers, as described, *e.g.,* in Felgner, *Scientific American.*

The lipid particles of the present invention may further comprise one or more additional lipids and/or other components such as cholesterol. Other lipids may be included in the liposome compositions of the present invention for a variety of purposes, such as to prevent lipid oxidation or to attach ligands onto the liposome surface. Any of a number of lipids may be present in liposomes of the present invention, including amphipathic, neutral, cationic, and anionic lipids. Such lipids can be used alone or in combination. Specific examples of additional lipid components that may be present are described below.

Additional components that may be present in a lipid particle of the present invention include bilayer stabilizing components such as polyamide oligomers (*see, e.g.,* U.S. Patent No. 6,320,017), peptides, proteins, detergents, lipid-derivatives, such as PEG coupled to phosphatidylethanolamine and PEG conjugated to ceramides (*see,* U.S. Patent No. 5,885,613).

In particular embodiments, the lipid particles include one or more of a second amino lipid or cationic lipid, a neutral lipid, a sterol, and a lipid selected to reduce aggregation of lipid particles during formation, which may result from steric stabilization of particles which prevents charge-induced aggregation during formation.

Examples of lipids that reduce aggregation of particles during formation include polyethylene glycol (PEG)-modified lipids, monosialoganglioside Gm1, and polyamide oligomers ("PAO") such as (described in US Pat. No. 6,320,017). Other compounds with uncharged, hydrophilic, steric-barrier moieties, which prevent aggregation during formulation, like PEG, Gm1 or ATTA, can also be coupled to lipids for use as in the methods and compositions of the invention. ATTA-lipids are described, *e.g.,* in U.S. Patent No. 6,320,017, and PEG-lipid conjugates are described, *e.g.,* in U.S. Patent Nos. 5,820,873, 5,534,499 and 5,885,613. Typically, the concentration of the lipid component selected to reduce aggregation is about 1 to 15% (by mole percent of lipids).

Specific examples of PEG-modified lipids (or lipid-polyoxyethylene conjugates) that are useful in the present invention can have a variety of "anchoring" lipid portions to secure the PEG portion to the surface of the lipid vesicle. Examples of suitable PEG-modified lipids include PEG-modified phosphatidylethanolamine and phosphatidic acid, PEG-ceramide conjugates (*e.g.,* PEG-CerC14 or PEG-CerC20) which are described in co-pending USSN 08/486,214, incorporated herein by reference, PEG-modified dialkylamines and PEG-modified 1,2-diacyloxypropan-3-amines. Particularly preferred are PEG-modified diacylglycerols and dialkylglycerols.

In particular embodiments, a PEG-lipid is selected from:

**PEG-C-DOMG**

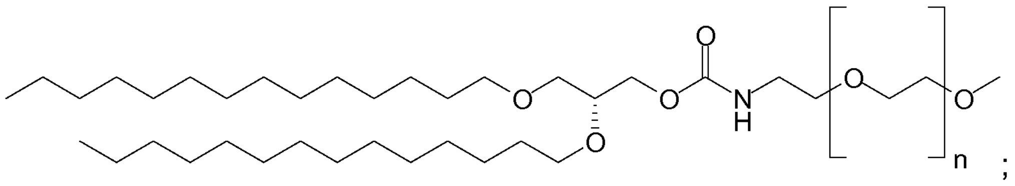

;

**PEG- DMA**

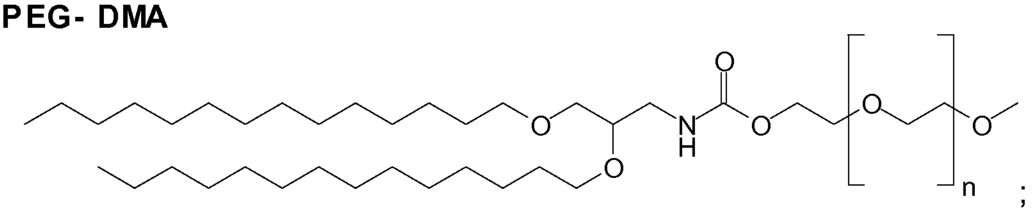

;

and

**PEG-S-DMG**

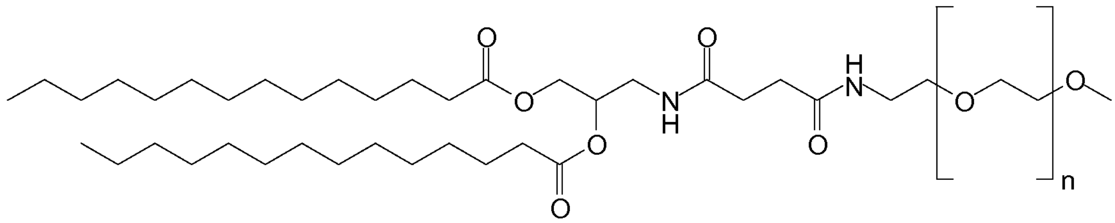

In embodiments where a sterically-large moiety such as PEG or ATTA are conjugated to a lipid anchor, the selection of the lipid anchor depends on what type of association the conjugate is to have with the lipid particle. It is well known that mePEG (mw2000)-diastearoylphosphatidylethanolamine (PEG-DSPE) will remain associated with a liposome until the particle is cleared from the circulation, possibly a matter of days. Other conjugates, such as PEG-CerC20 have similar staying capacity. PEG-CerC14, however, rapidly exchanges out of the formulation upon exposure to serum, with a $T_{1/2}$ less than 60 mins. in some assays. As illustrated in US Pat. Application SN 08/486,214, at least three characteristics influence the rate of exchange: length of acyl chain, saturation of acyl chain, and size of the steric-barrier head group. Compounds having suitable variations of these features may be useful for the invention. For some therapeutic applications it may be preferable for the PEG-modified lipid to be rapidly lost from the nucleic acid-lipid particle *in vivo* and hence the PEG-modified lipid will possess relatively short lipid anchors. In other therapeutic applications it may be preferable for the nucleic acid-lipid particle to exhibit a longer plasma circulation lifetime and hence the PEG-modified lipid will possess relatively longer lipid anchors.

It should be noted that aggregation preventing compounds do not necessarily require lipid conjugation to function properly. Free PEG or free ATTA in solution may be sufficient to prevent aggregation. If the particles are stable after formulation, the PEG or ATTA can be dialyzed away before administration to a subject.

Neutral lipids, when present in the lipid particle, can be any of a number of lipid species which exist either in an uncharged or neutral zwitterionic form at physiological pH. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the particles described herein is generally guided by consideration of, *e.g.,* liposome size and stability of the liposomes in the bloodstream. Preferably, the neutral lipid component is a lipid having two acyl groups, (*i.e.,* diacylphosphatidylcholine and diacylphosphatidylethanolamine). Lipids having a variety of acyl chain groups of varying chain length and degree of saturation are available or may be isolated or synthesized by well-known techniques. In one group of embodiments, lipids containing saturated fatty acids with carbon chain lengths in the range of $C_{14}$ to $C_{22}$ are preferred. In another group of embodiments, lipids with mono or diunsaturated fatty acids with carbon chain lengths in the range of $C_{14}$ to $C_{22}$ are used. Additionally, lipids having mixtures of saturated and unsaturated fatty acid chains can be used. Preferably, the neutral lipids used in the present invention are DOPE, DSPC, POPC, or any related phosphatidylcholine. The neutral lipids useful in the present invention may also be composed of sphingomyelin, dihydrosphingomyeline, or phospholipids with other head groups, such as serine and inositol.

The sterol component of the lipid mixture, when present, can be any of those sterols conventionally used in the field of liposome, lipid vesicle or lipid particle preparation. A preferred sterol is cholesterol.

Other cationic lipids, which carry a net positive charge at about physiological pH, in addition to those specifically described above, may also be included in lipid particles of the present invention. Such cationic lipids include, but are not limited to, N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"); 3β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, e.g., LIPOFECTIN (including DOTMA and DOPE, available from GIBCO/BRL), and LIPOFECTAMINE (comprising DOSPA and DOPE, available from GIBCO/BRL). In particular embodiments, a cationic lipid is an amino lipid.

Anionic lipids suitable for use in lipid particles of the present invention include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

In numerous embodiments, amphipathic lipids are included in lipid particles of the present invention. "Amphipathic lipids" refer to any suitable material, wherein the hydrophobic portion of the lipid material orients into a hydrophobic

phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine, dioleoylphosphatidylcholine, distearoylphosphatidylcholine, or dilinoleoylphosphatidylcholine. Other phosphorus-lacking compounds, such as sphingolipids, glycosphingolipid families, diacylglycerols, and β-acyloxyacids, can also be used. Additionally, such amphipathic lipids can be readily mixed with other lipids, such as triglycerides and sterols.

Also suitable for inclusion in the lipid particles of the present invention are programmable fusion lipids. Such lipid particles have little tendency to fuse with cell membranes and deliver their payload until a given signal event occurs. This allows the lipid particle to distribute more evenly after injection into an organism or disease site before it starts fusing with cells. The signal event can be, for example, a change in pH, temperature, ionic environment, or time. In the latter case, a fusion delaying or "cloaking" component, such as an ATTA-lipid conjugate or a PEG-lipid conjugate, can simply exchange out of the lipid particle membrane over time. By the time the lipid particle is suitably distributed in the body, it has lost sufficient cloaking agent so as to be fusogenic. With other signal events, it is desirable to choose a signal that is associated with the disease site or target cell, such as increased temperature at a site of inflammation.

In certain embodiments, it is desirable to target the lipid particles of this invention using targeting moieties that are specific to a cell type or tissue. Targeting of lipid particles using a variety of targeting moieties, such as ligands, cell surface receptors, glycoproteins, vitamins (*e.g.,* riboflavin) and monoclonal antibodies, has been previously described *(see, e.g.,* U.S. Patent Nos. 4,957,773 and 4,603,044). The targeting moieties can comprise the entire protein or fragments thereof. Targeting mechanisms generally require that the targeting agents be positioned on the surface of the lipid particle in such a manner that the target moiety is available for interaction with the target, for example, a cell surface receptor. A variety of different targeting agents and methods are known and available in the art, including those described, *e.g.,* in Sapra, P. and Allen, TM, Prog. Lipid Res. 42(5):439-62 (2003); and Abra, RM et al., J. Liposome Res. 12:1-3, (2002).

The use of lipid particles, *i.e.,* liposomes, with a surface coating of hydrophilic polymer chains, such as polyethylene glycol (PEG) chains, for targeting has been proposed (Allen, et al., Biochimica et Biophysica Acta 1237: 99-108 (1995); DeFrees, et al., Journal of the American Chemistry Society 118: 6101 - 6104 (1996); Blume, et al., Biochimica et Biophysica Acta 1149: 180-184 (1993); Klibanov, et al., Journal of Liposome Research 2: 321-334 (1992); U.S. Patent No. 5,013556; Zalipsky, Bioconjugate Chemistry 4: 296-299 (1993); Zalipsky, FEBS Letters 353: 71-74 (1994); Zalipsky, in Stealth Liposomes Chapter 9 (Lasic and Martin, Eds) CRC Press, Boca Raton Fl (1995). In one approach, a ligand, such as an antibody, for targeting the lipid particle is linked to the polar head group of lipids forming the lipid particle. In another approach, the targeting ligand is attached to the distal ends of the PEG chains forming the hydrophilic polymer coating (Klibanov, et al., Journal of Liposome Research 2: 321-334 (1992); Kirpotin et al., FEBS Letters 388: 115-118 (1996)).

Standard methods for coupling the target agents can be used. For example, phosphatidylethanolamine, which can be activated for attachment of target agents, or derivatized lipophilic compounds, such as lipid-derivatized bleomycin, can be used. Antibody-targeted liposomes can be constructed using, for instance, liposomes that incorporate protein A (*see,* Renneisen, et al., J. Bio. Chem., 265:16337-16342 (1990) and Leonetti, et al., Proc. Natl. Acad. Sci. (USA), 87:2448-2451 (1990). Other examples of antibody conjugation are disclosed in U.S. Patent No. 6,027,726, the teachings of which are incorporated herein by reference. Examples of targeting moieties can also include other proteins, specific to cellular components, including antigens associated with neoplasms or tumors. Proteins used as targeting moieties can be attached to the liposomes via covalent bonds (see, Heath, Covalent Attachment of Proteins to Liposomes, 149 Methods in Enzymology 111-119 (Academic Press, Inc. 1987)). Other targeting methods include the biotin-avidin system.

In one exemplary embodiment, the lipid particle comprises a mixture of an amino lipid of the present invention, neutral lipids (other than an amino lipid), a sterol (*e.g.,* cholesterol) and a PEG-modified lipid (*e.g.,* a PEG-S-DMG, PEG-C-DOMG or PEG-DMA). In certain embodiments, the lipid mixture consists of or consists essentially of an amino lipid of the present invention, a neutral lipid, cholesterol, and a PEG-modified lipid. In further preferred embodiments, the lipid particle consists of or consists essentially of the above lipid mixture in molar ratios of about 20-70% amino lipid: 5-45% neutral lipid:20-55% cholesterol:0.5-15% PEG-modified lipid.

In particular embodiments, the lipid particle consists of or consists essentially of DLin-K-DMA, DSPC, Chol, and either PEG-S-DMG, PEG-C-DOMG or PEG-DMA, *e.g.,* in a molar ratio of about 20-60% DLin-K-DMA: 5-25% DSPC:25-55% Chol:0.5-15% PEG-S-DMG, PEG-C-DOMG or PEG-DMA. In particular embodiments, the molar lipid ratio is approximately 40/10/40/10 (mol% DLin-K-DMA/DSPC/Chol/PEG-S-DMG or DLin-K-DMA/DSPC/Chol/PEG-C-DOMG or DLin-K-DMA/DSPC/Chol/PEG-DMA) or 35/15/40/10 mol% DLin-K-DMA/DSPC/Chol/PEG-S-DMG or DLin-K-DMA/DSPC/Chol/PEG-C-DOMG or DLin-K-DMA/DSPC/Chol/PEG-DMA. In another group of embodiments, the neutral lipid in these compositions is replaced with POPC, DOPE or SM.

C. Therapeutic Agent-Lipid Particle Compositions and Formulations

The present invention includes compositions comprising a lipid particle of the present invention and an active agent, wherein the active agent is associated with the lipid particle. In particular embodiments, the active agent is a therapeutic agent. In particular embodiments, the active agent is encapsulated within an aqueous interior of the lipid particle. In other embodiments, the active agent is present within one or more lipid layers of the lipid particle. In other embodiments, the active agent is bound to the exterior or interior lipid surface of a lipid particle.

"Fully encapsulated" as used herein indicates that the nucleic acid in the particles is not significantly degraded after exposure to serum or a nuclease assay that would significantly degrade free DNA or RNA. In a fully encapsulated system, preferably less than 25% of particle nucleic acid is degraded in a treatment that would normally degrade 100% of free nucleic acid, more preferably less than 10% and most preferably less than 5% of the particle nucleic acid is degraded. Alternatively, full encapsulation may be determined by an Oligreen® assay. Oligreen® is an ultra-sensitive fluorescent nucleic acid stain for quantitating oligonucleotides and single-stranded DNA or RNA in solution (available from Invitrogen Corporation, Carlsbad, CA). Fully encapsulated also suggests that the particles are serum stable, that is, that they do not rapidly decompose into their component parts upon *in vivo* administration.

Active agents, as used herein, include any molecule or compound capable of exerting a desired effect on a cell, tissue, organ, or subject. Such effects may be biological, physiological, or cosmetic, for example. Active agents may be any type of molecule or compound, including *e.g.,* nucleic acids, peptides and polypeptides, including, *e.g.,* antibodies, such as, *e.g.,* polyclonal antibodies, monoclonal antibodies, antibody fragments; humanized antibodies, recombinant antibodies, recombinant human antibodies, and Primatized™ antibodies, cytokines, growth factors, apoptotic factors, differentiation-inducing factors, cell surface receptors and their ligands; hormones; and small molecules, including small organic molecules or compounds.

In one embodiment, the active agent is a therapeutic agent, or a salt or derivative thereof. Therapeutic agent derivatives may be therapeutically active themselves or they may be prodrugs, which become active upon further modification. Thus, in one embodiment, a therapeutic agent derivative retains some or all of the therapeutic activity as compared to the unmodified agent, while in another embodiment, a therapeutic agent derivative lacks therapeutic activity.

In various embodiments, therapeutic agents include any therapeutically effective agent or drug, such as anti-inflammatory compounds, antidepressants, stimulants, analgesics, antibiotics, birth control medication, antipyretics, vasodilators, anti-angiogenics, cytovascular agents, signal transduction inhibitors, cardiovascular drugs, *e.g.,* anti-arrhythmic agents, vasoconstrictors, hormones, and steroids.

In certain embodiments, the therapeutic agent is an oncology drug, which may also be referred to as an anti-tumor drug, an anti-cancer drug, a tumor drug, an antineoplastic agent, or the like. Examples of oncology drugs that may be used according to the invention include, but are not limited to, adriamycin, alkeran, allopurinol, altretamine, amifostine, anastrozole, araC, arsenic trioxide, azathioprine, bexarotene, biCNU, bleomycin, busulfan intravenous, busulfan oral, capecitabine (Xeloda), carboplatin, carmustine, CCNU, celecoxib, chlorambucil, cisplatin, cladribine, cyclosporin A, cytarabine, cytosine arabinoside, daunorubicin, cytoxan, daunorubicin, dexamethasone, dexrazoxane, dodetaxel, doxorubicin, doxorubicin, DTIC, epirubicin, estramustine, etoposide phosphate, etoposide and VP-16, exemestane, FK506, fludarabine, fluorouracil, 5-FU, gemcitabine (Gemzar), gemtuzumab-ozogamicin, goserelin acetate, hydrea, hydroxyurea, idarubicin, ifosfamide, imatinib mesylate, interferon, irinotecan (Camptostar, CPT-111), letrozole, leucovorin, leustatin, leuprolide, levamisole, litretinoin, megastrol, melphalan, L-PAM, mesna, methotrexate, methoxsalen, mithramycin, mitomycin, mitoxantrone, nitrogen mustard, paclitaxel, pamidronate, Pegademase, pentostatin, porfimer sodium, prednisone, rituxan, streptozocin, STI-571, tamoxifen, taxotere, temozolamide, teniposide, VM-26, topotecan (Hycamtin), toremifene, tretinoin, ATRA, valrubicin, velban, vinblastine, vincristine, VP16, and vinorelbine. Other examples of oncology drugs that may be used according to the invention are ellipticin and ellipticin analogs or derivatives, epothilones, intracellular kinase inhibitors and camptothecins.

1. Nucleic Acid-Lipid Particles

In certain embodiments, lipid particles of the present invention are associated with a nucleic acid, resulting in a nucleic acid-lipid particle. In particular embodiments, the nucleic acid is fully encapsulated in the lipid particle. As used herein, the term "nucleic acid" is meant to include any oligonucleotide or polynucleotide. Fragments containing up to 50 nucleotides are generally termed oligonucleotides, and longer fragments are called polynucleotides. In particular embodiments, oligonucletoides of the present invention are 20-50 nucleotides in length.

In the context of this invention, the terms "polynucleotide" and "oligonucleotide" refer to a polymer or oligomer of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often preferred over native forms because of properties such as, for example, enhanced cellular uptake and increased stability in the

presence of nucleases.

Oligonucleotides are classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this invention includes any form of nucleic acid that is known. The nucleic acids used herein can be single-stranded DNA or RNA, or double-stranded DNA or RNA, or DNA-RNA hybrids. Examples of double-stranded DNA include structural genes, genes including control and termination regions, and self-replicating systems such as viral or plasmid DNA. Examples of double-stranded RNA include siRNA and other RNA interference reagents. Single-stranded nucleic acids include, *e.g.,* antisense oligonucleotides, ribozymes, microRNA, and triplex-forming oligonucleotides.

Nucleic acids of the present invention may be of various lengths, generally dependent upon the particular form of nucleic acid. For example, in particular embodiments, plasmids or genes may be from about 1,000 to 100,000 nucleotide residues in length. In particular embodiments, oligonucleotides may range from about 10 to 100 nucleotides in length. In various related embodiments, oligonucleotides, both single-stranded, double-stranded, and triple-stranded, may range in length from about 10 to about 50 nucleotides, from about 20 o about 50 nucleotides, from about 15 to about 30 nucleotides, from about 20 to about 30 nucleotides in length.

In particular embodiments, an oligonucleotide (or a strand thereof) of the present invention specifically hybridizes to or is complementary to a target polynucleotide. "Specifically hybridizable" and "complementary" are terms which are used to indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility or expression therefrom, and there is a sufficient degree of complementarity to avoid non-specific binding of the oligonucleotide to non-target sequences under conditions in which specific binding is desired, *i.e.,* under physiological conditions in the case of in vivo assays or therapeutic treatment, or, in the case of *in vitro* assays, under conditions in which the assays are conducted. Thus, in other embodiments, this oligonucleotide includes 1, 2, or 3 base substitutions as compared to the region of a gene or mRNA sequence that it is targeting or to which it specifically hybridizes.

RNA Interference Nucleic Acids

In particular embodiments, nucleic acid-lipid particles of the present invention are associated with RNA interference (RNAi) molecules. RNA interference methods using RNAi molecules may be used to disrupt the expression of a gene or polynucleotide of interest. In the last 5 years small interfering RNA (siRNA) has essentially replaced antisense ODN and ribozymes as the next generation of targeted oligonucleotide drugs under development. SiRNAs are RNA duplexes normally 21-30 nucleotides long that can associate with a cytoplasmic multi-protein complex known as RNAi-induced silencing complex (RISC). RISC loaded with siRNA mediates the degradation of homologous mRNA transcripts, therefore siRNA can be designed to knock down protein expression with high specificity. Unlike other antisense technologies, siRNA function through a natural mechanism evolved to control gene expression through non-coding RNA. This is generally considered to be the reason why their activity is more potent *in vitro* and *in vivo* than either antisense ODN or ribozymes. A variety of RNAi reagents, including siRNAs targeting clinically relevant targets, are currently under pharmaceutical development, as described, *e.g.,* in de Fougerolles, A. et al., Nature Reviews 6:443-453 (2007).

While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology 24:111-119). Thus, the invention includes the use of RNAi molecules comprising any of these different types of double-stranded molecules. In addition, it is understood that RNAi molecules may be used and introduced to cells in a variety of forms. Accordingly, as used herein, RNAi molecules encompasses any and all molecules capable of inducing an RNAi response in cells, including, but not limited to, double-stranded polynucleotides comprising two separate strands, *i.e.* a sense strand and an antisense strand, *e.g.,* small interfering RNA (siRNA); polynucleotides comprising a hairpin loop of complementary sequences, which forms a double-stranded region, *e.g.,* shRNAi molecules, and expression vectors that express one or more polynucleotides capable of forming a double-stranded polynucleotide alone or in combination with another polynucleotide.

RNA interference (RNAi) may be used to specifically inhibit expression of target polynucleotides. Double-stranded RNA-mediated suppression of gene and nucleic acid expression may be accomplished according to the invention by introducing dsRNA, siRNA or shRNA into cells or organisms. SiRNA may be double-stranded RNA, or a hybrid molecule comprising both RNA and DNA, *e.g.,* one RNA strand and one DNA strand. It has been demonstrated that the direct introduction of siRNAs to a cell can trigger RNAi in mammalian cells (Elshabir, S.M., et al. Nature

411:494-498 (2001)). Furthermore, suppression in mammalian cells occurred at the RNA level and was specific for the targeted genes, with a strong correlation between RNA and protein suppression (Caplen, N. et al., Proc. Natl. Acad. Sci. USA 98:9746-9747 (2001)). In addition, it was shown that a wide variety of cell lines, including HeLa S3, COS7, 293, NIH/3T3, A549, HT-29, CHO-KI and MCF-7 cells, are susceptible to some level of siRNA silencing (Brown, D. et al. TechNotes 9(1):1-7, available at http://www.dot.ambion.dot.com/techlib/tn/91/912.html (9/1/02)).

RNAi molecules targeting specific polynucleotides can be readily prepared according to procedures known in the art. Structural characteristics of effective siRNA molecules have been identified. Elshabir, S.M. et al. (2001) Nature 411:494-498 and Elshabir, S.M. et al. (2001), EMBO 20:6877-6888. Accordingly, one of skill in the art would understand that a wide variety of different siRNA molecules may be used to target a specific gene or transcript. In certain embodiments, siRNA molecules according to the invention are double-stranded and 16 - 30 or 18 - 25 nucleotides in length, including each integer in between. In one embodiment, an siRNA is 21 nucleotides in length. In certain embodiments, siRNAs have 0-7 nucleotide 3' overhangs or 0-4 nucleotide 5' overhangs. In one embodiment, an siRNA molecule has a two nucleotide 3' overhang. In one embodiment, an siRNA is 21 nucleotides in length with two nucleotide 3' overhangs (*i.e.* they contain a 19 nucleotide complementary region between the sense and antisense strands). In certain embodiments, the overhangs are UU or dTdT 3' overhangs.

Generally, siRNA molecules are completely complementary to one strand of a target DNA molecule, since even single base pair mismatches have been shown to reduce silencing. In other embodiments, siRNAs may have a modified backbone composition, such as, for example, 2'-deoxy- or 2'-O-methyl modifications. However, in preferred embodiments, the entire strand of the siRNA is not made with either 2' deoxy or 2'-O-modified bases.

In one embodiment, siRNA target sites are selected by scanning the target mRNA transcript sequence for the occurrence of AA dinucleotide sequences. Each AA dinucleotide sequence in combination with the 3' adjacent approximately 19 nucleotides are potential siRNA target sites. In one embodiment, siRNA target sites are preferentially not located within the 5' and 3' untranslated regions (UTRs) or regions near the start codon (within approximately 75 bases), since proteins that bind regulatory regions may interfere with the binding of the siRNP endonuclease complex (Elshabir, S. et al. Nature 411:494-498 (2001); Elshabir, S. et al. EMBO J. 20:6877-6888 (2001)). In addition, potential target sites may be compared to an appropriate genome database, such as BLASTN 2.0.5, available on the NCBI server at www.ncbi.nlm, and potential target sequences with significant homology to other coding sequences eliminated.

In particular embodiments, short hairpin RNAs constitute the nucleic acid component of nucleic acid-lipid particles of the present invention. Short Hairpin RNA (shRNA) is a form of hairpin RNA capable of sequence-specifically reducing expression of a target gene. Short hairpin RNAs may offer an advantage over siRNAs in suppressing gene expression, as they are generally more stable and less susceptible to degradation in the cellular environment. It has been established that such short hairpin RNA-mediated gene silencing works in a variety of normal and cancer cell lines, and in mammalian cells, including mouse and human cells. Paddison, P. et al., Genes Dev. 16(8):948-58 (2002). Furthermore, transgenic cell lines bearing chromosomal genes that code for engineered shRNAs have been generated. These cells are able to constitutively synthesize shRNAs, thereby facilitating long-lasting or constitutive gene silencing that may be passed on to progeny cells. Paddison, P. et al., Proc. Natl. Acad. Sci. USA 99(3):1443-1448 (2002).

ShRNAs contain a stem loop structure. In certain embodiments, they may contain variable stem lengths, typically from 19 to 29 nucleotides in length, or any number in between. In certain embodiments, hairpins contain 19 to 21 nucleotide stems, while in other embodiments, hairpins contain 27 to 29 nucleotide stems. In certain embodiments, loop size is between 4 to 23 nucleotides in length, although the loop size may be larger than 23 nucleotides without significantly affecting silencing activity. ShRNA molecules may contain mismatches, for example G-U mismatches between the two strands of the shRNA stem without decreasing potency. In fact, in certain embodiments, shRNAs are designed to include one or several G-U pairings in the hairpin stem to stabilize hairpins during propagation in bacteria, for example. However, complementarity between the portion of the stem that binds to the target mRNA (antisense strand) and the mRNA is typically required, and even a single base pair mismatch is this region may abolish silencing. 5' and 3' overhangs are not required, since they do not appear to be critical for shRNA function, although they may be present (Paddison et al. (2002) Genes & Dev. 16(8):948-58).

MicroRNAs

Micro RNAs (miRNAs) are a highly conserved class of small RNA molecules that are transcribed from DNA in the genomes of plants and animals, but are not translated into protein. Processed miRNAs are single stranded ~17-25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3'-untranslated region of specific mRNAs.RISC mediates down-regulation of gene expression through translational inhibition, transcript cleavage, or both. RISC is also implicated in transcriptional silencing in the nucleus of a wide range of eukaryotes.

The number of miRNA sequences identified to date is large and growing, illustrative examples of which can be found, for example, in: "miRBase: microRNA sequences, targets and gene nomenclature" Griffiths-Jones S, Grocock RJ, van Dongen S, Bateman A, Enright AJ. NAR, 2006, 34, Database Issue, D140-D144; "The microRNA Registry' Griffiths-Jones S. NAR, 2004, 32, Database Issue, D109-D111; and also at http://microrna.sanger.ac.uk/sequences/.

Antisense Oligonucleotides

In one embodiment, a nucleic acid is an antisense oligonucleotide directed to a target polynucleotide. The term "antisense oligonucleotide" or simply "antisense" is meant to include oligonucleotides that are complementary to a targeted polynucleotide sequence. Antisense oligonucleotides are single strands of DNA or RNA that are complementary to a chosen sequence. In the case of antisense RNA, they prevent translation of complementary RNA strands by binding to it. Antisense DNA can be used to target a specific, complementary (coding or non-coding) RNA. If binding takes places this DNA/RNA hybrid can be degraded by the enzyme RNase H. In particular embodiment, antisense oligonucleotides contain from about 10 to about 50 nucleotides, more preferably about 15 to about 30 nucleotides. The term also encompasses antisense oligonucleotides that may not be exactly complementary to the desired target gene. Thus, the invention can be utilized in instances where non-target specific-activities are found with antisense, or where an antisense sequence containing one or more mismatches with the target sequence is the most preferred for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal $GABA_A$ receptor and human EGF (Jaskulski et al., Science. 1988 Jun 10;240(4858):1544-6; Vasanthakumar and Ahmed, Cancer Commun. 1989;1 (4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, *e.g.* cancer (U. S. Patent 5,747,470; U. S. Patent 5,591,317 and U. S. Patent 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, $T_m$, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res. 1997, 25(17):3389-402).

Ribozymes

According to another embodiment of the invention, nucleic acid-lipid particles are associated with ribozymes. Ribozymes are RNA-protein complexes having specific catalytic domains that possess endonuclease activity (Kim and Cech, Proc Natl Acad Sci USA. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis δ virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis δ virus motif is described by Perrotta and Been, Biochemistry. 1992 Dec 1 ;31 (47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci USA. 1991 Oct 1;88(19):8826-30; Collins and Olive, Biochemistry. 1993 Mar 23;32(11):2795-9); and an example of the Group I intron is described in U. S. Patent 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to any polynucleotide sequence are known in the art. Ribozymes may be designed as described in Int. Pat. Appl. Publ. No. WO 93/23569 and Int. Pat. Appl. Publ. No. WO 94/02595, each specifically incorporated herein by reference, and synthesized to be tested *in vitro* and *in vivo,* as described therein.

Ribozyme activity can be optimized by altering the length of the ribozyme binding arms or chemically synthesizing ribozymes with modifications that prevent their degradation by serum ribonucleases (see *e.g.,* Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Immunostimulatory Oligonucleotides

Nucleic acids associated with lipid paticles of the present invention may be immunostimulatory, including immunostimulatory oligonucleotides (ISS; single-or double-stranded) capable of inducing an immune response when administered to a subject, which may be a mammal or other patient. ISS include, *e.g.,* certain palindromes leading to hairpin secondary structures (see Yamamoto S., et al. (1992) J. Immunol. 148: 4072-4076), or CpG motifs, as well as other known ISS features (such as multi-G domains, see WO 96/11266).

The immune response may be an innate or an adaptive immune response. The immune system is divided into a more innate immune system, and acquired adaptive immune system of vertebrates, the latter of which is further divided into humoral cellular components. In particular embodiments, the immune response may be mucosal.

In particular embodiments, an immunostimulatory nucleic acid is only immunostimulatory when administered in combination with a lipid particle, and is not immunostimulatory when administered in its "free form." According to the present invention, such an oligonucleotide is considered to be immunostimulatory.

Immunostimulatory nucleic acids are considered to be non-sequence specific when it is not required that they specifically bind to and reduce the expression of a target polynucleotide in order to provoke an immune response. Thus, certain immunostimulatory nucleic acids may comprise a seuqence correspondign to a region of a naturally occurring gene or mRNA, but they may still be considered non-sequence specific immunostimulatory nucleic acids.

In one embodiment, the immunostimulatory nucleic acid or oligonucleotide comprises at least one CpG dinucleotide. The oligonucleotide or CpG dinucleotide may be unmethylated or methylated. In another embodiment, the immunostimulatory nucleic acid comprises at least one CpG dinucleotide having a methylated cytosine. In one embodiment, the nucleic acid comprises a single CpG dinucleotide, wherein the cytosine in said CpG dinucleotide is methylated. In a specific embodiment, the nucleic acid comprises the sequence 5' TAACGTTGAGGGGCAT 3' (SEQ ID NO:2). In an alternative embodiment, the nucleic acid comprises at least two CpG dinucleotides, wherein at least one cytosine in the CpG dinucleotides is methylated. In a further embodiment, each cytosine in the CpG dinucleotides present in the sequence is methylated. In another embodiment, the nucleic acid comprises a plurality of CpG dinucleotides, wherein at least one of said CpG dinucleotides comprises a methylated cytosine.

In one specific embodiment, the nucleic acid comprises the sequence 5' TTCCATGACGTTCCTGACGT 3' (SEQ ID NO:33). In another specific embodiment, the nucleic acid sequence comprises the sequence 5' TCCATGACGTTCCTGACGT 3' (SEQ ID NO:31), wherein the two cytosines indicated in bold are methylated. In particular embodiments, the ODN is selected from a group of ODNs consisting of ODN #1, ODN #2, ODN #3, ODN #4, ODN #5, ODN #6, ODN #7, ODN #8, and ODN #9, as shown below.

Table 1. Exemplary Immunostimulatory Oligonucleotides (ODNs)

| ODN NAME | ODN SEQ ID NO | ODN SEQUENCE (5'-3'). |
|---|---|---|
| ODN 1 (INX-6295) human c-myc | SEQ ID NO: 2 | 5'-TAACGTTGAGGGGCAT-3 |
| * ODN 1m (INX-6303) | SEQ ID NO: 4 | 5'-TAAZGTTGAGGGGCAT-3 |
| ODN 2 (INX-1826) | SEQ ID NO: 1 | 5'-TCCATGACGTTCCTGACGTT-3 |
| * ODN 2m (INX-1826m) | SEQ ID NO: 31 | 5'-TCCATGAZGTTCCTGAZGTT-3 |
| ODN 3 (INX-6300) | SEQ ID NO: 3 | 5'-TAAGCATACGGGGTGT-3 |
| ODN 5 (INX-5001) | SEQ ID NO: 5 | 5'-AACGTT-3 |
| ODN 6 (INX-3002) | SEQ ID NO: 6 | 5'-GATGCTGTGTCGGGGTCTCCGGGC-3' |
| ODN 7 (INX-2006) | SEQ ID NO: 7 | 5'-TCGTCGTTTTGTCGTTTTGTCGTT-3' |
| ODN 7m (INX-2006m) | SEQ ID NO: 32 | 5'-TZGTZGTTTTGTZGTTTTGTZGTT-3' |
| ODN 8 (INX-1982) | SEQ ID NO: 8 | 5'-TCCAGGACTTCTCTCAGGTT-3' |
| ODN 9 (INX-G3139) | SEQ ID NO: 9 | 5'-TCTCCCAGCGTGCGCCAT-3' |
| ODN 10 (PS-3082) murine Intracellular Adhesion Molecule-1 | SEQ ID NO: 10 | 5'-TGCATCCCCCAGGCCACCAT-3 |
| ODN 11 (PS-2302) human Intracellular Adhesion Molecule-1 | SEQ ID NO: 11 | 5'-GCCCAAGCTGGCATCCGTCA-3' |
| ODN 12 (PS-8997) human Intracellular Adhesion Molecule-1 | SEQ ID NO: 12 | 5'-GCCCAAGCTGGCATCCGTCA-3' |
| ODN 13 (US3) human erb-B-2 | SEQ ID NO: 13 | 5'-GGT GCTCACTGC GGC-3' |
| ODN 14 (LR-3280) human c-myc | SEQ ID NO: 14 | 5'-AACC GTT GAG GGG CAT-3' |
| ODN 15 (LR-3001) human c-myc | SEQ ID NO: 15 | 5'-TAT GCT GTG CCG GGG TCT TCG GGC-3' |
| ODN 16 (Inx-6298) | SEQ ID NO: 16 | 5'-GTGCCG GGGTCTTCGGGC-3' |
| ODN 17 (hIGF-1R) human Insulin Growth Factor 1-Receptor | SEQ ID NO: 17 | 5'-GGACCCTCCTCCGGAGCC-3' |
| ODN 18 (LR-52) human Insulin Growth Factor 1-Receptor | SEQ ID NO: 18 | 5'-TCC TCC GGA GCC AGA CTT-3' |
| ODN 19 (hEGFR) human Epidermal Growth Factor-Receptor | SEQ ID NO: 19 | 5'-AAC GTT GAG GGG CAT-3' |
| ODN 20 (EGFR) Epidermal Growth Factor - Receptor | SEQ ID NO: 20 | 5'-CCGTGGTCA TGCTCC-3' |
| ODN 21 (hVEGF) human Vascular Endothelial Growth Factor | SEQ ID NO: 21 | 5'-CAG CCTGGCTCACCG CCTTGG-3' |
| ODN 22 (PS-4189) murine Phosphokinase C-alpha | SEQ ID NO: 22 | 5'-CAG CCA TGG TTC CCC CCA AC-3' |
| ODN 23 (PS-3521) | SEQ ID NO: 23 | 5'-GTT CTC GCT GGT GAG TTT CA-3' |

(continued)

| ODN NAME | ODN SEQ ID NO | ODN SEQUENCE (5'-3'). |
|---|---|---|
| ODN 24 (hBcl-2) human Bcl-2 | SEQ ID NO: 24 | 5'-TCT CCCAGCGTGCGCCAT-3' |
| ODN 25 (hC-Raf-1) human C-Raf-s | SEQ ID NO: 25 | 5'-GTG CTC CAT TGA TGC-3' |
| ODN #26 (hVEGF-R1) human Vascular Endothelial Growth Factor Receptor-1 | SEQ ID NO: 26 | 5'-GAGUUCUGAUGAGGCCGAAAGGCCGAA AGUCUG-3' |
| ODN #27 | SEQ ID NO: 27 | 5'-RRCGYY-3' |
| ODN # 28 (INX-3280). | SEQ ID NO: 28 | 5'-AACGTTGAGGGGCAT-3' |
| ODN #29 (INX-6302) | SEQ ID NO: 29 | 5'-CAACGTTATGGGGAGA-3' |
| ODN #30 (INX-6298) human c-myc | SEQ ID NO: 30 | 5'-TAACGTTGAGGGGCAT-3' |
| "Z" represents a methylated cytosine residue.<br>Note: ODN 14 is a 15-mer oligonucleotide and ODN 1 is the same oligonucleotide having a thymidine added onto the 5' end making ODN 1 into a 16-mer. No difference in biological activity between ODN 14 and ODN 1 has been detected and both exhibit similar immunostimulatory activity (Mui *et al.,* 2001) | | |

Additional specific nucleic acid sequences of oligonucleotides (ODNs) suitable for use in the compositions and methods of the invention are described in U.S. Patent Appln. 60/379,343, U.S. patent application Ser. No. 09/649,527, Int. Publ. WO 02/069369, Int. Publ. No. WO 01/15726, U.S. Pat. No. 6,406,705, and Raney et al., Journal of Pharmacology and Experimental Therapeutics, 298:1185-1192 (2001). In certain embodiments, ODNs used in the compositions and methods of the present invention have a phosphodiester ("PO") backbone or a phosphorothioate ("PS") backbone, and/or at least one methylated cytosine residue in a CpG motif.

Nucleic Acid Modifications

In the 1990's DNA-based antisense oligodeoxynucleotides (ODN) and ribozymes (RNA) represented an exciting new paradigm for drug design and development, but their application *in vivo* was prevented by endo- and exonuclease activity as well as a lack of successful intracellular delivery. The degradation issue was effectively overcome following extensive research into chemical modifications that prevented the oligonucleotide (oligo) drugs from being recognized by nuclease enzymes but did not inhibit their mechanism of action. This research was so successful that antisense ODN drugs in development today remain intact *in vivo* for days compared to minutes for unmodified molecules (Kurreck, J. 2003. Antisense technologies. Improvement through novel chemical modifications. Eur J Biochem 270:1628-44). However, intracellular delivery and mechanism of action issues have so far limited antisense ODN and ribozymes from becoming clinical products.

RNA duplexes are inherently more stable to nucleases than single stranded DNA or RNA, and unlike antisense ODN, unmodified siRNA show good activity once they access the cytoplasm. Even so, the chemical modifications developed to stabilize antisense ODN and ribozymes have also been systematically applied to siRNA to determine how much chemical modification can be tolerated and if pharmacokinetic and pharmacodynamic activity can be enhanced. RNA interference by siRNA duplexes requires an antisense and sense strand, which have different functions. Both are necessary to enable the siRNA to enter RISC, but once loaded the two strands separate and the sense strand is degraded whereas the antisense strand remains to guide RISC to the target mRNA. Entry into RISC is a process that is structurally less stringent than the recognition and cleavage of the target mRNA. Consequently, many different chemical modifications of the sense strand are possible, but only limited changes are tolerated by the antisense strand (Zhang *et al.*, 2006).

As is known in the art, a nucleoside is a base-sugar combination. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In

forming oligonucleotides, the phosphate groups covalently link adjacent nucleosides to one another to form a linear polymeric compound. In turn the respective ends of this linear polymeric structure can be further joined to form a circular structure. Within the oligonucleotide structure, the phosphate groups are commonly referred to as forming the internucleoside backbone of the oligonucleotide. The normal linkage or backbone of RNA and DNA is a 3' to 5' phosphodiester linkage.

The nucleic acid that is used in a lipid-nucleic acid particle according to this invention includes any form of nucleic acid that is known. Thus, the nucleic acid may be a modified nucleic acid of the type used previously to enhance nuclease resistance and serum stability. Surprisingly, however, acceptable therapeutic products can also be prepared using the method of the invention to formulate lipid-nucleic acid particles from nucleic acids that have no modification to the phosphodiester linkages of natural nucleic acid polymers, and the use of unmodified phosphodiester nucleic acids *(i.e.,* nucleic acids in which all of the linkages are phosphodiester linkages) is a preferred embodiment of the invention.

a. Backbone Modifications

Antisense, siRNA and other oligonucleotides useful in this invention include, but are not limited to, oligonucleotides containing modified backbones or non-natural internucleoside linkages. Oligonucleotides having modified backbones include those that retain a phosphorus atom in the backbone and those that do not have a phosphorus atom in the backbone. Modified oligonucleotides that do not have a phosphorus atom in their internucleoside backbone can also be considered to be oligonucleosides. Modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, phosphoroselenate, methylphosphonate, or O-alkyl phosphotriester linkages, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Particular nonlimiting examples of particular modifications that may be present in a nucleic acid according to the present invention are shown in Table 2.

Various salts, mixed salts and free acid forms are also included. Representative United States patents that teach the preparation of the above linkages include, but are not limited to, U.S. Patent Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

In certain embodiments, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include, e.g., those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts. Representative United States patents that describe the above oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

The phosphorothioate backbone modification (Table 2, #1), where a non-bridging oxygen in the phosphodiester bond is replaced by sulfur, is one of the earliest and most common means deployed to stabilize nucleic acid drugs against nuclease degradation. In general, it appears that PS modifications can be made extensively to both siRNA strands without much impact on activity (Kurreck, J., Eur. J. Biochem. 270:1628-44, 2003). However, PS oligos are known to avidly associate non-specifically with proteins resulting in toxicity, especially upon i.v. administration. Therefore, the PS modification is usually restricted to one or two bases at the 3' and 5' ends. The boranophosphate linker (Table 2, #2) is a recent modification that is apparently more stable than PS, enhances siRNA activity and has low toxicity (Hall et al., Nucleic Acids Res. 32:5991-6000, 2004).

Table 2. Chemical Modifications Applied to siRNA and Other Nucleic Acids

| # | Abbreviation | Name | Modification Site | Structure |
|---|---|---|---|---|
| **1** | PS | Phosphorothioate | Backbone | 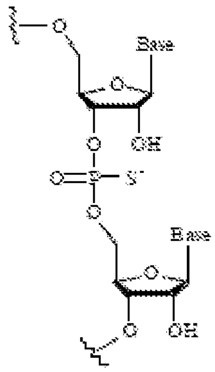 |
| **2** | PB | Boranophosphate | Backbone | 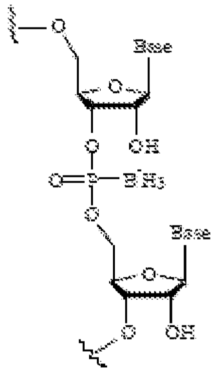 |
| **3** | N3-MU | N3-methyl-uridine | Base | 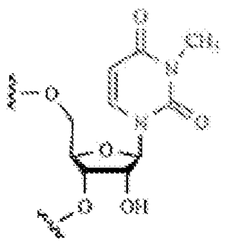 |
| **4** | 5'-BU | 5'-bromo-uracil | Base | 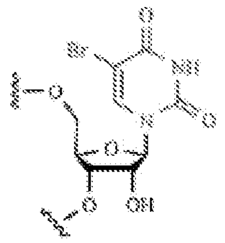 |
| **5** | 5'-IU | 5'-iodo-uracil | Base | 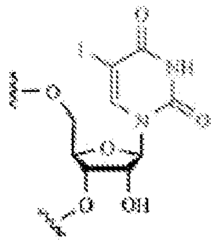 |

(continued)

| # | Abbreviation | Name | Modification Site | Structure |
|---|---|---|---|---|
| **6** | 2,6-DP | 2,6-diaminopurine | Base | 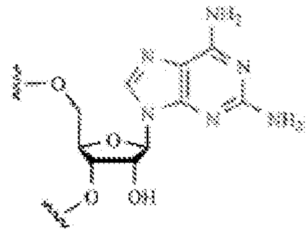 |
| **7** | 2’-F | 2’-Fluoro | Sugar | 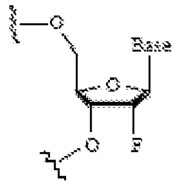 |
| **8** | 2’-OME | 2"-O-methyl | Sugar | 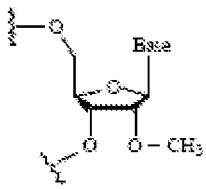 |
| **9** | 2’-O-MOE | 2’-O-(2-methoxylethyl) | Sugar | 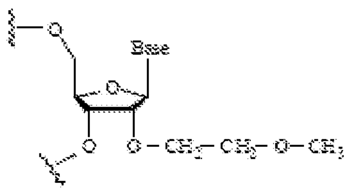 |
| **10** | 2’-DNP | 2’-O-(2,4-dinitrophenyl) | Sugar | 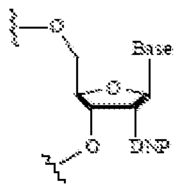 |
| **11** | LNA | Locked Nucleic Acid (methylene bridge connecting the 2’-oxygen with the 4’-carbon of the ribose ring) | Sugar | 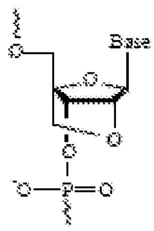 |
| **12** | 2’-Amino | 2’-Amino | Sugar | 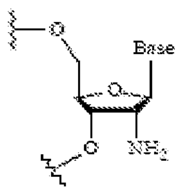 |

(continued)

| # | Abbreviation | Name | Modification Site | Structure |
|---|---|---|---|---|
| **13** | 2'-Deoxy | 2'-Deoxy | Sugar | 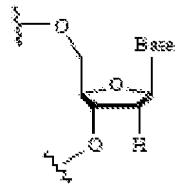 |
| **14** | 4'-th io | 4'-thio-ribonucleotide | Sugar | 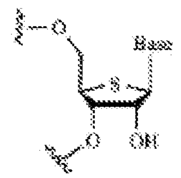 |

Other useful nucleic acids derivatives include those nucleic acids molecules in which the bridging oxygen atoms (those forming the phosphoester linkages) have been replaced with -S-, -NH-, -CH2- and the like. In certain embodiments, the alterations to the antisense, siRNA, or other nucleic acids used will not completely affect the negative charges associated with the nucleic acids. Thus, the present invention contemplates the use of antisense, siRNA, and other nucleic acids in which a portion of the linkages are replaced with, for example, the neutral methyl phosphonate or phosphoramidate linkages. When neutral linkages are used, in certain embodiments, less than 80% of the nucleic acid linkages are so substituted, or less than 50% of the linkages are so substituted.

b. Base Modifications

Base modifications are less common than those to the backbone and sugar. The modifications shown in 0.3-6 all appear to stabilize siRNA against nucleases and have little effect on activity ( Zhang, H.Y., Du, Q., Wahlestedt, C., Liang, Z. 2006. RNA Interference with chemically modified siRNA. Curr Top Med Chem 6:893-900).

Accordingly, oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C or m5c), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine.

Certain nucleobases are particularly useful for increasing the binding affinity of the oligomeric compounds of the invention, including 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C. (Sanghvi, Y. S., Crooke, S. T. and Lebleu, B., eds., Antisense Research and Applications 1993, CRC Press, Boca Raton, pages 276-278). These may be combined, in particular embodiments, with 2'-O-methoxyethyl sugar modifications. United States patents that teach the preparation of certain of these modified nucleobases as well as other modified nucleobases include, but are not limited to, the above noted U.S. Pat. No. 3,687,808, as well as U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941.

c. Sugar Modifications

Most modifications on the sugar group occur at the 2'-OH of the RNA sugar ring, which provides a convenient chemically reactive site Manoharan, M. 2004. RNA interference and chemically modified small interfering RNAs. Curr Opin Chem Biol 8:570-9; Zhang, H.Y., Du, Q., Wahlestedt, C., Liang, Z. 2006. RNA Interference with chemically modified

siRNA. Curr Top Med Chem 6:893-900). The 2'-F and 2'-OME (0.7 and 8) are common and both increase stability, the 2'-OME modification does not reduce activity as long as it is restricted to less than 4 nucleotides per strand ( Holen, T., Amarzguioui, M., Babaie, E., Prydz, H. 2003. Similar behaviour of single-strand and double-strand siRNAs suggests they act through a common RNAi pathway. Nucleic Acids Res 31:2401-7). The 2'-O-MOE (0.9) is most effective in siRNA when modified bases are restricted to the middle region of the molecule ( Prakash, T.P., Allerson, C.R., Dande, P., Vickers, T.A., Sioufi, N., Jarres, R., Baker, B.F., Swayze, E.E., Griffey, R.H., Bhat, B. 2005. Positional effect of chemical modifications on short interference RNA activity in mammalian cells. J Med Chem 48:4247-53). Other modifications found to stabilize siRNA without loss of activity are shown in 0.10-14.

Modified oligonucleotides may also contain one or more substituted sugar moieties. For example, the invention includes oligonucleotides that comprise one of the following at the 2' position: OH; F; O-, S-, or N-alkyl, O-alkyl-O-alkyl, O-, S-, or N-alkenyl, or O-, S- or N-alkynyl, wherein the alkyl, alkenyl and alkynyl may be substituted or unsubstituted $C_1$ to $C_{10}$ alkyl or C2 to C10 alkenyl and alkynyl. Particularly preferred are $O[(CH_2)_nO]_mCH_3$, $O(CH_2)_nOCH_3$, $O(CH_2)_2ON(CH_3)_2$, $O(CH_2)_nNH_2$, $O0(CH_2)_nCH_3$, $O(CH_2)_nONH_2$, and $O(CH_2)_nON[(CH_2)_nCH_3)]_2$, where n and m are from 1 to about 10. Other preferred oligonucleotides comprise one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. One modification includes 2'-methoxyethoxy (2'-O--$CH_2CH_2OCH_3$, also known as 2'-O-(2-methoxyethyl) or 2'-MOE) (Martin et al., Helv. Chim. Acta 1995, 78, 486-504), *i.e.,* an alkoxyalkoxy group. Other modifications include 2'-dimethylaminooxyethoxy, *i.e.,* a $O(CH_2)_2ON(CH_3)_2$ group, also known as 2'-DMAOE, and 2'-dimethylaminoethoxyethoxy (2'-DMAEOE).

Additional modifications include 2'-methoxy (2'-O--$CH_3$), 2'-aminopropoxy (2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugars structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

In other oligonucleotide mimetics, both the sugar and the internucleoside linkage, *i.e.,* the backbone, of the nucleotide units are replaced with novel groups, although the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos. 5,539,082; 5,714,331; and 5,719,262. Further teaching of PNA compounds can be found in Nielsen etal. (Science, 1991, 254, 1497-1500).

Particular embodiments of the invention are oligonucleotides with phosphorothioate backbones and oligonucleosides with heteroatom backbones, and in particular --$CH_2$--NH--O--$CH_2$--, --$CH_2$--N($CH_3$) --O--$CH_2$- (referred to as a methylene (methylimino) or MMI backbone) --$CH_2$--O--N($CH_3$) --$CH_2$--, --$CH_2$-N($CH_3$)--N($CH_3$) --$CH_2$-- and --O--N($CH_3$) --$CH_2$--$CH_2$-(wherein the native phosphodiester backbone is represented as --O--P--O--$CH_2$ --) of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

d. Chimeric Oligonucleotides

It is not necessary for all positions in a given compound to be uniformly modified, and in fact more than one of the aforementioned modifications may be incorporated in a single compound or even at a single nucleoside within an oligonucleotide. Certain preferred oligonucleotides of this invention are chimeric oligonucleotides. "Chimeric oligonucleotides" or "chimeras," in the context of this invention, are oligonucleotides that contain two or more chemically distinct regions, each made up of at least one nucleotide. These oligonucleotides typically contain at least one region of modified nucleotides that confers one or more beneficial properties (such as, *e,g.,* increased nuclease resistance, increased uptake into cells, increased binding affinity for the RNA target) and a region that is a substrate for RNase H cleavage.

In one embodiment, a chimeric oligonucleotide comprises at least one region modified to increase target binding affinity. Affinity of an oligonucleotide for its target is routinely determined by measuring the Tm of an oligonucleotide/target pair, which is the temperature at which the oligonucleotide and target dissociate; dissociation is detected spectrophotometrically. The higher the Tm, the greater the affinity of the oligonucleotide for the target. In one embodiment, the region of the oligonucleotide which is modified to increase target mRNA binding affinity comprises at least one nucleotide modified at the 2' position of the sugar, most preferably a 2'-O-alkyl, 2'-O-alkyl-O-alkyl or 2'-fluoro-modified nucleotide. Such modifications are routinely incorporated into oligonucleotides and these oligonucleotides have been shown to have a higher Tm *(i.e.,* higher target binding affinity) than 2'-deoxyoligonucleotides against a given target. The effect of such increased affinity is to greatly enhance oligonucleotide inhibition of target gene expression.

In another embodiment, a chimeric oligonucletoide comprises a region that acts as a substrate for RNAse H. Of course, it is understood that oligonucleotides may include any combination of the various modifications described herein

Another modification of the oligonucleotides of the invention involves chemically linking to the oligonucleotide one or more moieties or conjugates which enhance the activity, cellular distribution or cellular uptake of the oligonucleotide. Such conjugates and methods of preparing the same are known in the art.

Those skilled in the art will realize that for *in vivo* utility, such as therapeutic efficacy, a reasonable rule of thumb is that if a thioated version of the sequence works in the free form, that encapsulated particles of the same sequence, of any chemistry, will also be efficacious. Encapsulated particles may also have a broader range of *in vivo* utilities, showing efficacy in conditions and models not known to be otherwise responsive to antisense therapy. Those skilled in the art know that applying this invention they may find old models which now respond to antisense therapy. Further, they may revisit discarded antisense sequences or chemistries and find efficacy by employing the invention.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of the routineer. It is also well known to use similar techniques to prepare other oligonucleotides such as the phosphorothioates and alkylated derivatives.

## Characteristic of Nucleic Acid-Lipid Particles

In certain embodiments, the present invention relates to methods and compositions for producing lipid-encapsulated nucleic acid particles in which nucleic acids are encapsulated within a lipid layer. Such nucleic acid-lipid particles, incorporating siRNA oligonucleotides, are characterized using a variety of biophysical parameters including: (1)drug to lipid ratio; (2) encapsulation efficiency; and (3) particle size. High drug to lipid rations, high encapsulation efficiency, good nuclease resistance and serum stability and controllable particle size, generally less than 200 nm in diameter are desirable. In addition, the nature of the nucleic acid polymer is of significance, since the modification of nucleic acids in an effort to impart nuclease resistance adds to the cost of therapeutics while in many cases providing only limited resistance. Unless stated otherwise, these criteria are calculated in this specification as follows:

Nucleic acid to lipid ratio is the amount of nucleic acid in a defined volume of preparation divided by the amount of lipid in the same volume. This may be on a mole per mole basis or on a weight per weight basis, or on a weight per mole basis. For final, administration-ready formulations, the nucleic acid:lipid ratio is calculated after dialysis, chromatography and/or enzyme (*e.g.,* nuclease) digestion has been employed to remove as much of the external nucleic acid as possible;

Encapsulation efficiency refers to the drug to lipid ratio of the starting mixture divided by the drug to lipid ratio of the final, administration competent formulation. This is a measure of relative efficiency. For a measure of absolute efficiency, the total amount of nucleic acid added to the starting mixture that ends up in the administration competent formulation, can also be calculated. The amount of lipid lost during the formulation process may also be calculated. Efficiency is a measure of the wastage and expense of the formulation; and

Size indicates the size (diameter) of the particles formed. Size distribution may be determined using quasi-elastic light scattering (QELS) on a Nicomp Model 370 sub-micron particle sizer. Particles under 200 nm are preferred for distribution to neo-vascularized (leaky) tissues, such as neoplasms and sites of inflammation.

## Pharmaceutical Compositions

The lipid particles of present invention, particularly when associated with a therapeutic agent, may b formulated as a pharmaceutical composition, e.g., which further comprises a pharmaceutically acceptable diluent, excipient, or carrier, such as physiological saline or phosphate buffer, selected in accordance with the route of administration and standard pharmaceutical practice.

In particular embodiments, pharmaceutical compositions comprising the lipid-nucleic acid particles of the invention are prepared according to standard techniques and further comprise a pharmaceutically acceptable carrier. Generally, normal saline will be employed as the pharmaceutically acceptable carrier. Other suitable carriers include,

*e.g.,* water, buffered water, 0.9% saline, 0.3% glycine, and the like, including glycoproteins for enhanced stability, such as albumin, lipoprotein, globulin, *etc.* In compositions comprising saline or other salt containing carriers, the carrier is preferably added following lipid particle formation. Thus, after the lipid-nucleic acid compositions are formed, the compositions can be diluted into pharmaceutically acceptable carriers such as normal saline.

The resulting pharmaceutical preparations may be sterilized by conventional, well known sterilization techniques. The aqueous solutions can then be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, *etc.* Additionally, the lipidic suspension may include lipid-protective agents which protect lipids against free-radical and lipid-peroxidative damages on storage. Lipophilic free-radical quenchers, such as $\alpha$-tocopherol and water-soluble iron-specific chelators, such as ferrioxamine, are suitable.

The concentration of lipid particle or lipid-nucleic acid particle in the pharmaceutical formulations can vary widely, *i.e.,* from less than about 0.01%, usually at or at least about 0.05-5% to as much as 10 to 30% by weight and will be selected primarily by fluid volumes, viscosities, *etc.,* in accordance with the particular mode of administration selected. For example, the concentration may be increased to lower the fluid load associated with treatment. This may be particularly desirable in patients having atherosclerosis-associated congestive heart failure or severe hypertension. Alternatively, complexes composed of irritating lipids may be diluted to low concentrations to lessen inflammation at the site of administration. In one group of embodiments, the nucleic acid will have an attached label and will be used for diagnosis (by indicating the presence of complementary nucleic acid). In this instance, the amount of complexes administered will depend upon the particular label used, the disease state being diagnosed and the judgement of the clinician but will generally be between about 0.01 and about 50 mg per kilogram of body weight, preferably between about 0.1 and about 5 mg/kg of body weight.

As noted above, the lipid-therapeutic agent (*e.g.,* nucleic acid) particels of the invention may include polyethylene glycol (PEG)-modified phospholipids, PEG-ceramide, or ganglioside $G_{M1}$-modified lipids or other lipids effective to prevent or limit aggregation. Addition of such components does not merely prevent complex aggregation. Rather, it may also provide a means for increasing circulation lifetime and increasing the delivery of the lipid-nucleic acid composition to the target tissues.

The present invention also provides lipid-therapeutic agent compositions in kit form. The kit will typically be comprised of a container that is compartmentalized for holding the various elements of the kit. The kit will contain the particles or pharmaceutical compositions of the present invention, preferably in dehydrated or concentrated form, with instructions for their rehydration or dilution and administration. In certain embodiments, the particles comprise the active agent, while in other embodiments, they do not.

D. Methods of Manufacture

The methods and compositions of the invention make use of certain cationic lipids, the synthesis, preparation and characterization of which is described below and in the accompanying Examples. In addition, the present invention provides methods of preparing lipid particles, including those associated with a therapeutic agent, *e.g.,* a nucleic acid. In the methods described herein, a mixture of lipids is combined with a buffered aqueous solution of nucleic acid to produce an intermediate mixture containing nucleic acid encapsulated in lipid particles wherein the encapsulated nucleic acids are present in a nucleic acid/lipid ratio of about 3 wt% to about 25 wt%, preferably 5 to 15 wt%. The intermediate mixture may optionally be sized to obtain lipid-encapsulated nucleic acid particles wherein the lipid portions are unilamellar vesicles, preferably having a diameter of 30 to 150 nm, more preferably about 40 to 90 nm. The pH is then raised to neutralize at least a portion of the surface charges on the lipid-nucleic acid particles, thus providing an at least partially surface-neutralized lipid-encapsulated nucleic acid composition.

As described above, several of these cationic lipids are amino lipids that are charged at a pH below the $pK_a$ of the amino group and substantially neutral at a pH above the $pK_a$. These cationic lipids are termed titratable cationic lipids and can be used in the formulations of the invention using a two-step process. First, lipid vesicles can be formed at the lower pH with titratable cationic lipids and other vesicle components in the presence of nucleic acids. In this manner, the vesicles will encapsulate and entrap the nucleic acids. Second, the surface charge of the newly formed vesicles can be neutralized by increasing the pH of the medium to a level above the $pK_a$ of the titratable cationic lipids present, *i.e.,* to physiological pH or higher. Particularly advantageous aspects of this process include both the facile removal of any surface adsorbed nucleic acid and a resultant nucleic acid delivery vehicle which has a neutral surface. Liposomes or lipid particles having a neutral surface are expected to avoid rapid clearance from circulation and to avoid certain toxicities which are associated with cationic liposome preparations. Additional details concerning these uses of such titratable cationic lipids in the formulation of nucleic acid-lipid particles are provided in US Patent 6,287,591 and US Patent 6,858,225, incorporated herein by reference.

It is further noted that the vesicles formed in this manner provide formulations of uniform vesicle size with high content of nucleic acids. Additionally, the vesicles have a size range of from about 30 to about 150 nm, more preferably about 30 to about 90 nm.

Without intending to be bound by any particular theory, it is believed that the very high efficiency of nucleic acid encapsulation is a result of electrostatic interaction at low pH. At acidic pH (e.g. pH 4.0) the vesicle surface is charged and binds a portion of the nucleic acids through electrostatic interactions. When the external acidic buffer is exchanged for a more neutral buffer *(e.g..* pH 7.5) the surface of the lipid particle or liposome is neutralized, allowing any external nucleic acid to be removed. More detailed information on the formulation process is provided in various publications (*e.g.,* US Patent 6,287,591 and US Patent 6,858,225).

In view of the above, the present invention provides methods of preparing lipid/nucleic acid formulations. In the methods described herein, a mixture of lipids is combined with a buffered aqueous solution of nucleic acid to produce an intermediate mixture containing nucleic acid encapsulated in lipid particles, *e.g.,* wherein the encapsulated nucleic acids are present in a nucleic acid/lipid ratio of about 10 wt% to about 20 wt%. The intermediate mixture may optionally be sized to obtain lipid-encapsulated nucleic acid particles wherein the lipid portions are unilamellar vesicles, preferably having a diameter of 30 to 150 nm, more preferably about 40 to 90 nm. The pH is then raised to neutralize at least a portion of the surface charges on the lipid-nucleic acid particles, thus providing an at least partially surface-neutralized lipid-encapsulated nucleic acid composition.

In certain embodiments, the mixture of lipids includes at least two lipid components: a first amino lipid component of the present invention that is selected from among lipids which have a pKa such that the lipid is cationic at pH below the pKa and neutral at pH above the pKa, and a second lipid component that is selected from among lipids that prevent particle aggregation during lipid-nucleic acid particle formation. In particular embodiments, the amino lipid is a novel cationic lipid of the present invention.

In preparing the nucleic acid-lipid particles of the invention, the mixture of lipids is typically a solution of lipids in an organic solvent. This mixture of lipids can then be dried to form a thin film or lyophilized to form a powder before being hydrated with an aqueous buffer to form liposomes. Alternatively, in a preferred method, the lipid mixture can be solubilized in a water miscible alcohol, such as ethanol, and this ethanolic solution added to an aqueous buffer resulting in spontaneous liposome formation. In most embodiments, the alcohol is used in the form in which it is commercially available. For example, ethanol can be used as absolute ethanol (100%), or as 95% ethanol, the remainder being water. This method is described in more detail in US Patent 5,976,567).

In one exemplary embodiment, the mixture of lipids is a mixture of cationic amino lipids, neutral lipids (other than an amino lipid), a sterol (*e.g.,* cholesterol) and a PEG-modified lipid (*e.g.,* a PEG-S-DMG, PEG-C-DOMG or PEG-DMA) in an alcohol solvent. In preferred embodiments, the lipid mixture consists essentially of a cationic amino lipid, a neutral lipid, cholesterol and a PEG-modified lipid in alcohol, more preferably ethanol. In further preferred embodiments, the first solution consists of the above lipid mixture in molar ratios of about 20-70% amino lipid: 5-45% neutral lipid:20-55% cholesterol:0.5-15% PEG-modified lipid. In still further preferred embodiments, the first solution consists essentially of DLin-K-DMA, DSPC, Chol and PEG-S-DMG, PEG-C-DOMG or PEG-DMA, more preferably in a molar ratio of about 20-60% DLin-K-DMA: 5-25% DSPC:25-55% Chol:0.5-15% PEG-S-DMG, PEG-C-DOMG or PEG-DMA. In another group of preferred embodiments, the neutral lipid in these compositions is replaced with POPC, DOPE or SM.

In accordance with the invention, the lipid mixture is combined with a buffered aqueous solution that may contain the nucleic acids. The buffered aqueous solution of is typically a solution in which the buffer has a pH of less than the $pK_a$ of the protonatable lipid in the lipid mixture. Examples of suitable buffers include citrate, phosphate, acetate, and MES. A particularly preferred buffer is citrate buffer. Preferred buffers will be in the range of 1-1000 mM of the anion, depending on the chemistry of the nucleic acid being encapsulated, and optimization of buffer concentration may be significant to achieving high loading levels (*see, e.g.,* US Patent 6,287,591 and US Patent 6,858,225). Alternatively, pure water acidified to pH 5-6 with chloride, sulfate or the like may be useful. In this case, it may be suitable to add 5% glucose, or another non-ionic solute which will balance the osmotic potential across the particle membrane when the particles are dialyzed to remove ethanol, increase the pH, or mixed with a pharmaceutically acceptable carrier such as normal saline. The amount of nucleic acid in buffer can vary, but will typically be from about 0.01 mg/mL to about 200 mg/mL, more preferably from about 0.5 mg/mL to about 50 mg/mL.

The mixture of lipids and the buffered aqueous solution of therapeutic nucleic acids is combined to provide an intermediate mixture. The intermediate mixture is typically a mixture of lipid particles having encapsulated nucleic acids. Additionally, the intermediate mixture may also contain some portion of nucleic acids which are attached to the surface of the lipid particles (liposomes or lipid vesicles) due to the ionic attraction of the negatively-charged nucleic acids and positively-charged lipids on the lipid particle surface (the amino lipids or other lipid making up the protonatable first lipid component are positively charged in a buffer having a pH of less than the $pK_a$ of the protonatable group on the lipid). In one group of preferred embodiments, the mixture of lipids is an alcohol solution of lipids and the volumes of each of the solutions is adjusted so that upon combination, the resulting alcohol content is from about 20% by volume to about 45% by volume. The method of combining the mixtures can include any of a variety of processes, often depending upon the

scale of formulation produced. For example, when the total volume is about 10-20 mL or less, the solutions can be combined in a test tube and stirred together using a vortex mixer. Large-scale processes can be carried out in suitable production scale glassware.

Optionally, the lipid-encapsulated therapeutic agent (*e.g.,* nucleic acid) complexes which are produced by combining the lipid mixture and the buffered aqueous solution of therapeutic agents (nucleic acids) can be sized to achieve a desired size range and relatively narrow distribution of lipid particle sizes. Preferably, the compositions provided herein will be sized to a mean diameter of from about 70 to about 200 nm, more preferably about 90 to about 130 nm. Several techniques are available for sizing liposomes to a desired size. One sizing method is described in U.S. Pat. No. 4,737,323, incorporated herein by reference. Sonicating a liposome suspension either by bath or probe sonication produces a progressive size reduction down to small unilamellar vesicles (SUVs) less than about 0.05 microns in size. Homogenization is another method which relies on shearing energy to fragment large liposomes into smaller ones. In a typical homogenization procedure, multilamellar vesicles are recirculated through a standard emulsion homogenizer until selected liposome sizes, typically between about 0.1 and 0.5 microns, are observed. In both methods, the particle size distribution can be monitored by conventional laser-beam particle size determination. For certain methods herein, extrusion is used to obtain a uniform vesicle size.

Extrusion of liposome compositions through a small-pore polycarbonate membrane or an asymmetric ceramic membrane results in a relatively well-defined size distribution. Typically, the suspension is cycled through the membrane one or more times until the desired liposome complex size distribution is achieved. The liposomes may be extruded through successively smaller-pore membranes, to achieve a gradual reduction in liposome size. In some instances, the lipid-nucleic acid compositions which are formed can be used without any sizing.

In particular embodiments, methods of the present invention further comprise a step of neutralizing at least some of the surface charges on the lipid portions of the lipid-nucleic acid compositions. By at least partially neutralizing the surface charges, unencapsulated nucleic acid is freed from the lipid particle surface and can be removed from the composition using conventional techniques. Preferably, unencapsulated and surface adsorbed nucleic acids are removed from the resulting compositions through exchange of buffer solutions. For example, replacement of a citrate buffer (pH about 4.0, used for forming the compositions) with a HEPES-buffered saline (HBS pH about 7.5) solution, results in the neutralization of liposome surface and nucleic acid release from the surface. The released nucleic acid can then be removed via chromatography using standard methods, and then switched into a buffer with a pH above the pKa of the lipid used.

Optionally the lipid vesicles (*i.e.,* lipid particles) can be formed by hydration in an aqueous buffer and sized using any of the methods described above prior to addition of the nucleic acid. As described above, the aqueous buffer should be of a pH below the pKa of the amino lipid. A solution of the nucleic acids can then be added to these sized, preformed vesicles. To allow encapsulation of nucleic acids into such "pre-formed" vesicles the mixture should contain an alcohol, such as ethanol. In the case of ethanol, it should be present at a concentration of about 20% (w/w) to about 45% (w/w). In addition, it may be necessary to warm the mixture of pre-formed vesicles and nucleic acid in the aqueous buffer-ethanol mixture to a temperature of about 25° C to about 50° C depending on the composition of the lipid vesicles and the nature of the nucleic acid. It will be apparent to one of ordinary skill in the art that optimization of the encapsulation process to achieve a desired level of nucleic acid in the lipid vesicles will require manipulation of variable such as ethanol concentration and temperature. Examples of suitable conditions for nucleic acid encapsulation are provided in the Examples. Once the nucleic acids are encapsulated within the prefromed vesicles, the external pH can be increased to at least partially neutralize the surface charge. Unencapsulated and surface adsorbed nucleic acids can then be removed as described above.

E. Method of Use

The lipid particles of the present invention may be used to deliver a therapeutic agent to a cell, *in vitro* or *in vivo.* In particular embodiments, the therapeutic agent is a nucleic acid, which is delivered to a cell using a nucleic acid-lipid particles of the present invention. While the following description o various methodsof using the lipid particles and related pharmaceutical compositions of the present invention are exemplified by description related to nucleic acid-lipid particles, it is understood that these methods and compositions may be readily adapted for the delivery of any therapeutic agent for the treatment of any disease or disorder that would benefit from such treatment.

In certain embodiments, the present invention provides methods for introducing a nucleic acid into a cell. Preferred nucleic acids for introduction into cells are siRNA, immune-stimulating oligonucleotides, plasmids, antisense and ribozymes. These methods may be carried out by contacting the particles or compositions of the present invention with the cells for a period of time sufficient for intracellular delivery to occur.

The compositions of the present invention can be adsorbed to almost any cell type. Once adsorbed, the nucleic acid-lipid particles can either be endocytosed by a portion of the cells, exchange lipids with cell membranes, or fuse with the cells. Transfer or incorporation of the nucleic acid portion of the complex can take place via any one of these pathways.

Without intending to be limited with respect to the scope of the invention, it is believed that in the case of particles taken up into the cell by endocytosis the particles then interact with the endosomal membrane, resulting in destabilization of the endosomal membrane, possibly by the formation of non-bilayer phases, resulting in introduction of the encapsulated nucleic acid into the cell cytoplasm. Similarly in the case of direct fusion of the particles with the cell plasma membrane, when fusion takes place, the liposome membrane is integrated into the cell membrane and the contents of the liposome combine with the intracellular fluid. Contact between the cells and the lipid-nucleic acid compositions, when carried out *in vitro,* will take place in a biologically compatible medium. The concentration of compositions can vary widely depending on the particular application, but is generally between about 1 μmol and about 10 mmol. In certain embodiments, treatment of the cells with the lipid-nucleic acid compositions will generally be carried out at physiological temperatures (about 37°C) for periods of time from about 1 to 24 hours, preferably from about 2 to 8 hours. For *in vitro* applications, the delivery of nucleic acids can be to any cell grown in culture, whether of plant or animal origin, vertebrate or invertebrate, and of any tissue or type. In preferred embodiments, the cells will be animal cells, more preferably mammalian cells, and most preferably human cells.

In one group of embodiments, a lipid-nucleic acid particle suspension is added to 60-80% confluent plated cells having a cell density of from about $10^3$ to about $10^5$ cells/mL, more preferably about 2 x $10^4$ cells/mL. The concentration of the suspension added to the cells is preferably of from about 0.01 to 20 μg/mL, more preferably about 1 μg/mL.

Typical applications include using well known procedures to provide intracellular delivery of siRNA to knock down or silence specific cellular targets. Alternatively applications include delivery of DNA or mRNA sequences that code for therapeutically useful polypeptides. In this manner, therapy is provided for genetic diseases by supplying deficient or absent gene products (*i.e.,* for Duchenne's dystrophy, see Kunkel, et al., Brit. Med. Bull. 45(3):630-643 (1989), and for cystic fibrosis, see Goodfellow, Nature 341:102-103 (1989)). Other uses for the compositions of the present invention include introduction of antisense oligonucleotides in cells (see, Bennett, et al., Mol. Pharm. 41:1023-1033 (1992)).

Methods of the present invention may be practiced *in vitro, ex vivo,* or *in vivo.* For example, the compositions of the present invention can also be used for deliver of nucleic acids to cells *in vivo,* using methods which are known to those of skill in the art. With respect to application of the invention for delivery of DNA or mRNA sequences, Zhu, et al., Science 261:209-211 (1993), incorporated herein by reference, describes the intravenous delivery of cytomegalovirus (CMV)-chloramphenicol acetyltransferase (CAT) expression plasmid using DOTMA-DOPE complexes. Hyde, et al., Nature 362:250-256 (1993), incorporated herein by reference, describes the delivery of the cystic fibrosis transmembrane conductance regulator (CFTR) gene to epithelia of the airway and to alveoli in the lung of mice, using liposomes. Brigham, et al., Am. J. Med. Sci. 298:278-281 (1989), incorporated herein by reference, describes the *in vivo* transfection of lungs of mice with a functioning prokaryotic gene encoding the intracellular enzyme, chloramphenicol acetyltransferase (CAT). Thus, the compositions of the invention can be used in the treatment of infectious diseases.

For *in vivo* administration, the pharmaceutical compositions are preferably administered parenterally, *i.e.,* intraarticularly, intravenously, intraperitoneally, subcutaneously, or intramuscularly. In particular embodiments, the pharmaceutical compositions are administered intravenously or intraperitoneally by a bolus injection. For one example, see Stadler, et al., U.S. Patent No. 5,286,634, which is incorporated herein by reference. Intracellular nucleic acid delivery has also been discussed in Straubringer, *et al.,* METHODS IN ENZYMOLOGY, Academic Press, New York. 101:512-527 (1983); Mannino, et al., Biotechniques 6:682-690 (1988); Nicolau, et al., Crit. Rev. Ther. Drug Carrier Syst. 6:239-271 (1989), and Behr, Acc. Chem. Res. 26:274-278 (1993). Still other methods of administering lipid-based therapeutics are described in, for example, Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179; Lenk et al., U.S. Patent No. 4,522,803; and Fountain et al., U.S. Patent No. 4,588,578.

In other methods, the pharmaceutical preparations may be contacted with the target tissue by direct application of the preparation to the tissue. The application may be made by topical, "open" or "closed" procedures. By "topical," it is meant the direct application of the pharmaceutical preparation to a tissue exposed to the environment, such as the skin, oropharynx, external auditory canal, and the like. "Open" procedures are those procedures which include incising the skin of a patient and directly visualizing the underlying tissue to which the pharmaceutical preparations are applied. This is generally accomplished by a surgical procedure, such as a thoracotomy to access the lungs, abdominal laparotomy to access abdominal viscera, or other direct surgical approach to the target tissue. "Closed" procedures are invasive procedures in which the internal target tissues are not directly visualized, but accessed via inserting instruments through small wounds in the skin. For example, the preparations may be administered to the peritoneum by needle lavage. Likewise, the pharmaceutical preparations may be administered to the meninges or spinal cord by infusion during a lumbar puncture followed by appropriate positioning of the patient as commonly practiced for spinal anesthesia or metrazamide imaging of the spinal cord. Alternatively, the preparations may be administered through endoscopic devices.

The lipid-nucleic acid compositions can also be administered in an aerosol inhaled into the lungs (see, Brigham, et al., Am. J. Sci. 298(4):278-281 (1989)) or by direct injection at the site of disease (Culver, Human Gene Therapy, MaryAnn Liebert, Inc., Publishers, New York. pp.70-71 (1994)).

The methods of the present invention may be practiced in a variety of subjects or hosts. Preferred subjects or hosts include mammalian species, such as humans, non-human primates, dogs, cats, cattle, horses, sheep, and the like. In particular embodiments, the subject is a mammal, such as a human, in need of treatment or prevention of a disease or disorder, *e.g.,* a subject diagnosed with or considered at risk for a disease or disorder.

Dosages for the lipid-therapeutic agent particles of the present invention will depend on the ratio of therapeutic agent to lipid and the administrating physician's opinion based on age, weight, and condition of the patient.

In one embodiment, the present invention provides a method of modulating the expression of a target polynucleotide or polypeptide. These methods generally comprise contacting a cell with a lipid particle of the present invention that is associated with a nucleic acid capable of modulating the expression of a target polynucleotide or polypeptide. As used herein, the term "modulating" refers to altering the expression of a target polynucleotide or polypeptide. In different embodiments, modulating can mean increasing or enhancing, or it can mean decreasing or reducing. Methods of measuring the level of expression of a target polynucleotide or polypeptide are known and available in the arts and include, e.g., methods employing reverse transcription-polymerase chain reaction (RT-PCR) and immunohistochemical techniques. In particular embodiments, the level of expression of a target polynucleotide or polypeptide is increased or reduced by at least 10%, 20%, 30%, 40%, 50%, or greater than 50% as compared to an appropriate control value.

For example, if increased expression of a polypeptide desired, the nucleic acid may be an expression vector that includes a polynucleotide that encodes the desired polypeptide. On the other hand, if reduced expression of a polynucleotide or polypeptide is desired, then the nucleic acid may be, *e.g.,* an antisense oligonucleotide, siRNA, or microRNA that comprises a polynucleotide sequence that specifically hybridizes to a polnucleotide that encodes the target polypeptide, thereby disrupting expression of the target polynucleotide or polypeptide. Alternatively, the nucleic acid may be a plasmid that expresses such an antisense oligonucletoide, siRNA, or microRNA.

In one particular embodiment, the present invention provides a method of modulating the expression of a polypeptide by a cell, comprising providing to a cell a lipid particle that consists of or consists essentially of DLin-K-DMA, DSPC, Chol and PEG-S-DMG, PEG-C-DOMG or PEG-DMA, *e.g.,* in a molar ratio of about 20-60% DLin-K-DMA: 5-25% DSPC:25-55% Chol:0.5-15% PEG-S-DMG, PEG-C-DOMG or PEG-DMA, wherein the lipid particle is assocated with a nucleic acid capable of modulating the expression of the polypeptide. In particular embodiments, the molar lipid ratio is approximately 40/10/40/10 (mol% DLin-K-DMA/DSPC/Chol/PEG-S-DMG, PEG-C-DOMG or PEG-DMA). In another group of embodiments, the neutral lipid in these compositions is replaced with POPC, DOPE or SM.

In particular embodiments, the nucleic acid active agent or therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof, such that the expression of the polypeptide is reduced.

In other embodiments, the nucleic acid is a plasmid that encodes the polypeptide or a functional variant or fragment thereof, such that expression of the polypeptide or the functional variant or fragment thereof is increased.

In related embodiments, the present invention provides a method of treating a disease or disorder characterized by overexpression of a polypeptide in a subject, comprising providing to the subject a pharmaceutical composition of the present invention, wherein the therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof.

In one embodiment, the pharmaceutical composition comprises a lipid particle that consists of or consists essentially of DLin-K-DMA, DSPC, Chol and PEG-S-DMG, PEG-C-DOMG or PEG-DMA, *e.g.,* in a molar ratio of about 20-60% DLin-K-DMA: 5-25% DSPC:25-55% Chol:0.5-15% PEG-S-DMG, PEG-C-DOMG or PEG-DMA, wherein the lipid particle is assocated with the therapeutic nucleic acid. In particular embodiments, the molar lipid ratio is approximately 40/10/40/10 (mol% DLin-K-DMA/DSPC/Chol/PEG-S-DMG, PEG-C-DOMG or PEG-DMA). In another group of embodiments, the neutral lipid in these compositions is replaced with POPC, DOPE or SM.

In another related embodiment, the present invention includes a method of treating a disease or disorder characterized by underexpression of a polypeptide in a subject, comprising providing to the subject a pharmaceutical composition of the present invention, wherein the therapeutic agent is a plasmid that encodes the polypeptide or a functional variant or fragment thereof.

In one embodiment, the pharmaceutical composition comprises a lipid particle that consists of or consists essentially of DLin-K-DMA, DSPC, Chol and PEG-S-DMG, PEG-C-DOMG or PEG-DMA, *e.g.,* in a molar ratio of about 20-60% DLin-K-DMA: 5-25% DSPC:25-55% Chol:0.5-15% PEG-S-DMG, PEG-C-DOMG or PEG-DMA, wherein the lipid particle is assocated with the therapeutic nucleic acid. In particular embodiments, the molar lipid ratio is approximately 40/10/40/10 (mol% DLin-K-DMA/DSPC/Chol/PEG-S-DMG, PEG-C-DOMG or PEG-DMA). In another group of embodiments, the neutral lipid in these compositions is replaced with POPC, DOPE or SM.

The present invention further provides a method of inducing an immune response in a subject, comprising

providing to the subject the pharmaceutical composition of the present invention, wherein the therapeutic agent is an immunostimulatory oligonucleotide. In certain embodiments, the immune response is a humoral or mucosal immune response. In one embodiment, the pharmaceutical composition comprises a lipid particle that consists of or consists essentially of DLin-K-DMA, DSPC, Chol and PEG-S-DMG, PEG-C-DOMG or PEG-DMA, *e.g.,* in a molar ratio of about 20-60% DLin-K-DMA: 5-25% DSPC:25-55% Chol:0.5-15% PEG-S-DMG, PEG-C-DOMG or PEG-DMA, wherein the lipid particle is assocated with the therapeutic nucleic acid. In particular embodiments, the molar lipid ratio is approximately 40/10/40/10 (mol% DLin-K-DMA/DSPC/Chol/PEG-S-DMG, PEG-C-DOMG or PEG-DMA). In another group of embodiments, the neutral lipid in these compositions is replaced with POPC, DOPE or SM.

In further embodiments, the pharmaceutical composition is provided to the subject in combination with a vaccine or antigen. Thus, the present invention itself provides vaccines comprising a lipid particle of the present invention, which comprises an immunostimulatory oligonucleotide, and is also associated with an antigen to which an immune response is desired. In particular embodiments, the antigen is a tumor antigen or is associated with an infective agent, such as, *e.g.,* a virus, bacteria, or parasiste.

A variety of tumor antigens, infections agent antigens, and antigens associated with other disease are well known in the art and examples of these are described in references cited herein. Examples of antigens suitable for use in the present invention include, but are not limited to, polypeptide antigens and DNA antigens. Specific examples of antigens are Hepatitis A, Hepatitis B, small pox, polio, anthrax, influenza, typhus, tetanus, measles, rotavirus, diphtheria, pertussis, tuberculosis, and rubella antigens. In a preferred embodiment, the antigen is a Hepatitis B recombinant antigen. In other aspects, the antigen is a Hepatitis A recombinant antigen. In another aspect, the antigen is a tumor antigen. Examples of such tumor-associated antigens are MUC-1, EBV antigen and antigens associated with Burkitt's lymphoma. In a further aspect, the antigen is a tyrosinase-related protein tumor antigen recombinant antigen. Those of skill in the art will know of other antigens suitable for use in the present invention.

Tumor-associated antigens suitable for use in the subject invention include both mutated and non-mutated molecules that may be indicative of single tumor type, shared among several types of tumors, and/or exclusively expressed or overexpressed in tumor cells in comparison with normal cells. In addition to proteins and glycoproteins, tumor-specific patterns of expression of carbohydrates, gangliosides, glycolipids and mucins have also been documented. Exemplary tumor-associated antigens for use in the subject cancer vaccines include protein products of oncogenes, tumor suppressor genes and other genes with mutations or rearrangements unique to tumor cells, reactivated embryonic gene products, oncofetal antigens, tissue-specific (but not tumor-specific) differentiation antigens, growth factor receptors, cell surface carbohydrate residues, foreign viral proteins and a number of other self proteins.

Specific embodiments of tumor-associated antigens include, *e.g.,* mutated antigens such as the protein products of the Ras p21 protooncogenes, tumor suppressor p53 and BCR-abl oncogenes, as well as CDK4, MUM1, Caspase 8, and Beta catenin; overexpressed antigens such as galectin 4, galectin 9, carbonic anhydrase, Aldolase A, PRAME, Her2/neu, ErbB-2 and KSA, oncofetal antigens such as alpha fetoprotein (AFP), human chorionic gonadotropin (hCG); self antigens such as carcinoembryonic antigen (CEA) and melanocyte differentiation antigens such as Mart 1/Melan A, gp100, gp75, Tyrosinase, TRP1 and TRP2; prostate associated antigens such as PSA, PAP, PSMA, PSM-P1 and PSM-P2; reactivated embryonic gene products such as MAGE 1, MAGE 3, MAGE 4, GAGE 1, GAGE 2, BAGE, RAGE, and other cancer testis antigens such as NY-ESO1, SSX2 and SCP1; mucins such as Muc-1 and Muc-2; gangliosides such as GM2, GD2 and GD3, neutral glycolipids and glycoproteins such as Lewis (y) and globo-H; and glycoproteins such as Tn, Thompson-Freidenreich antigen (TF) and sTn. Also included as tumor-associated antigens herein are whole cell and tumor cell lysates as well as immunogenic portions thereof, as well as immunoglobulin idiotypes expressed on monoclonal proliferations of B lymphocytes for use against B cell lymphomas.

Pathogens include, but are not limited to, infectious agents, *e.g.,* viruses, that infect mammals, and more particularly humans. Examples of infectious virus include, but are not limited to: Retroviridae (*e.g.,* human immunodeficiency viruses, such as HIV-1 (also referred to as HTLV-III, LAV or HTLV-III/LAV, or HIV-III; and other isolates, such as HIV-LP; Picornaviridae (*e.g.,* polio viruses, hepatitis A virus; enteroviruses, human Coxsackie viruses, rhinoviruses, echoviruses); Calciviridae (*e.g.,* strains that cause gastroenteritis); Togaviridae (*e.g.,* equine encephalitis viruses, rubella viruses); Flaviridae (*e.g.,* dengue viruses, encephalitis viruses, yellow fever viruses); Coronoviridae (*e.g.,* coronaviruses); Rhabdoviradae (*e.g.,* vesicular stomatitis viruses, rabies viruses); Coronaviridae (*e.g.,* coronaviruses); Rhabdoviridae (*e.g.,* vesicular stomatitis viruses, rabies viruses); Filoviridae (*e.g.,* ebola viruses); Paramyxoviridae (*e.g.,* parainfluenza viruses, mumps virus, measles virus, respiratory syncytial virus); Orthomyxoviridae (*e.g.,* influenza viruses); Bungaviridae (*e.g.,* Hantaan viruses, bunga viruses, phleboviruses and Nairo viruses); Arena viridae (hemorrhagic fever viruses); Reoviridae (*e.g.,* reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; Poxviridae (variola viruses, vaccinia viruses, pox viruses); and Iridoviridae (*e.g.,* African swine fever virus); and unclassified viruses (*e.g.,* the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted

(*i.e.,* Hepatitis C); Norwalk and related viruses, and astroviruses).

Also, gram negative and gram positive bacteria serve as antigens in vertebrate animals. Such gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to: Helicobacterpyloris, Borelia burgdorferi, Legionella pneumophilia, Mycobacteria sps (*e.g.,* M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcusfaecalis, Streptococcus bovis, Streptococcus (anaerobic sps.), Streptococcus pneumoniae, pathogenic Campylobacter sp., Enterococcus sp., Haemophilus infuenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelli.

Additional examples of pathogens include, but are not limited to, infectious fungi that infect mammals, and more particularly humans. Examples of infectious fingi include, but are not limited to: Cryptococcus neoformans, Histoplasma capsulatum, Coccidioides immitis, Blastomyces dermatitidis, Chlamydia trachomatis, Candida albicans. Examples of infectious parasites include Plasmodium such as Plasmodium falciparum, Plasmodium malariae, Plasmodium ovale, and Plasmodium vivax. Other infectious organisms *(i.e.,* protists) include Toxoplasma gondii.

## EXAMPLES

### EXAMPLE 1

### SYNTHESIS OF 2,2-DILINOLEYL-4-DIMETHYLAMINOMETHYL-[1,3]-DIOXOLANE (DLIN-K-DMA)

DLin-K-DMA was synthesized as shown in the following schematic and described below.

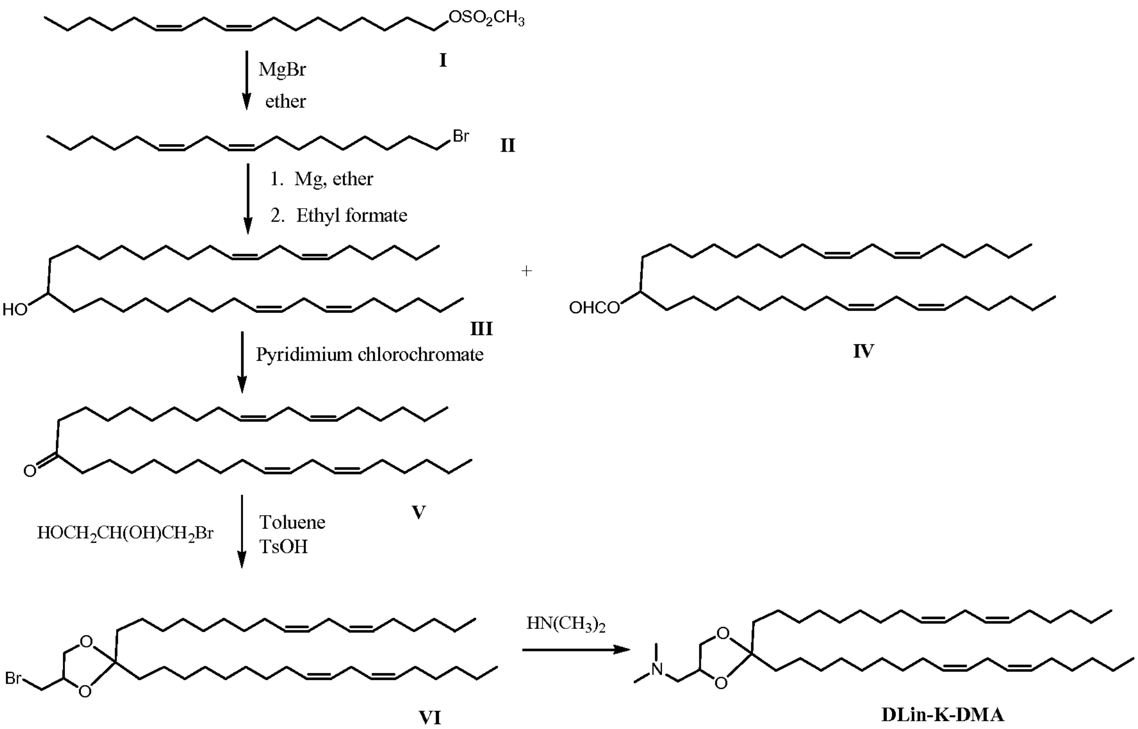


### Synthesis of Linoleyl Bromide (II)

A mixture of linoleyl methane sulfonate (6.2g, 18 mmol) and magnesium bromide etherate (17g, 55 mmol) in anhydrous ether (300 mL) was stirred under argon overnight (21 hours). The resulting suspension was poured into 300 mL of chilled water. Upon shaking, the organic phase was separated. The aqueous phase was extracted with ether (2 x 150 mL). The combined ether phase was washed with water (2 x 150 mL), brine (150 mL), and dried over anhydrous

$Na_2SO_4$. The solvent was evaporated to afford 6.5g of colourless oil. The crude product was purified by column chromatography on silica gel (230-400 mesh, 300 mL) eluted with hexanes. This gave 6.2 g (approximately 100%) of linoleyl bromide (II). 1 H NMR (400 MHz, $CDCl_3$) δ: 5.27-5.45 (4H, m, 2 x CH=CH), 3.42 (2H, t, $CH_2Br$), 2.79 (2H, t, C=C-$CH_2$-C=C), 2.06 (4H, q, 2 x allylic $CH_2$), 1.87 (2H, quintet, $CH_2$), 1.2-1.5 (16H, m), 0.90 (3H, t, $CH_3$) ppm.

Synthesis of Dilinoleyl Methanol (III)

To a suspension of Mg turnings (0.45g, 18.7 mmol) with one crystal of iodine in 200 mL of anhydrous ether under nitrogen was added a solution of linoleyl bromide (II) in 50 mL of anhydrous ether at room temperature. The resulting mixture was refluxed under nitrogen overnight. The mixture was cooled to room temperature. To the cloudy mixture under nitrogen was added dropwise at room temperature a solution of ethyl formate (0.65g, 18.7 mmol) in 30 mL of anhydrous ether. Upon addition, the mixture was stirred at room temperature overnight (20 hours). The ether layer was washed with 10% $H_2SO_4$ aqueous solution (100 mL), water (2 x 100 mL), brine (150 mL), and then dried over anhydrous $Na_2SO_4$. Evaporation of the solvent gave 5.0g of pale oil. Column chromatography on silica gel (230-400 mesh, 300 mL) with 0-7% ether gradient in hexanes as eluent afforded two products, dilinoleyl methanol (2.0g, III) and dilinoleylmethyl formate (1.4g, IV). 1 H NMR (400 MHz, $CDCl_3$) for dilinoleylmethyl formate (IV) δ: 8.10 (1 H, s, CHO), 5.27-5.45 (8H, m, 4 x CH=CH), 4.99 (1 H, quintet, OCH), 2.78 (4H, t, 2 x C=C-$CH_2$-C=C), 2.06 (8H, q, 4 x allylic $CH_2$), 1.5-1.6 (4H, m, 2 x $CH_2$), 1.2-1.5 (32H, m), 0.90 (6H, t, 2 x $CH_3$) ppm.

Dilinoleylmethyl formate (IV, 1.4g) and KOH (0.2g) were stirred in 85% EtOH at room temperature under nitrogen overnight. Upon completion of the reaction, half of the solvent was evaporated. The resulting mixture was poured into 150 mL of 5% HCL solution. The aqueous phase was extracted with ether (3 x 100 mL). The combined ether extract was washed with water (2 x 100 mL), brine (100 mL), and dried over anhydrous Na2SO4. Evaporation of the solvent gave 1.0 g of dilinoleyl methanol (III) as colourless oil. Overall, 3.0 g (60%) of dilinoleyl methanol (III) were afforded. 1 H NMR (400 MHz, $CDCl_3$) for dilinoleyl methanol (III) δ: ppm.

Synthesis of Dilinoleyl Ketone (V)

To a mixture of dilinoleyl methanol (2.0g, 3.8 mmol) and anhydrous sodium carbonate (0.2g) in 100 mL of $CH_2Cl_2$ was added pydimium chlorochromate (PCC, 2.0g, 9.5 mmol). The resulting suspension was stirred at room temperature for 60 min. Ether (300 mL) was then added into the mixture, and the resulting brown suspension was filtered through a pad of silica gel (300 mL). The silica gel pad was further washed with ether (3 x 200 mL). The ether filtrate and washes were combined. Evaporation of the solvent gave 3.0 g of an oily residual as a crude product. The crude product was purified by column chromatography on silica gel (230-400 mesh, 250 mL) eluted with 0-3% ether in hexanes. This gave 1.8 g (90%) of dilinoleyl ketone (V). 1 H NMR (400 MHz, $CDCl_3$) δ: 5.25-5.45 (8H, m, 4 x CH=CH), 2.78 (4H, t, 2 x C=C-$CH_2$-C=C), 2.39 (4H, t, 2 x $COCH_2$), 2.05 (8H, q, 4 x allylic $CH_2$), 1.45-1.7 (4H, m), 1.2-1.45 (32H, m), 0.90 (6H, t, 2 x $CH_3$) ppm.

Synthesis of 2,2-Dilinoleyl-4-bromomethyl-[1,3]-dioxolane (VI)

A mixture of dilinoleyl methanol (V, 1.3g, 2.5 mmol), 3-bromo-1,2-propanediol (1.5g, 9.7 mmol) and p-toluene sulonic acid hydrate (0.16g, 0.84 mmol) in 200 mL of toluene was refluxed under nitrogen for 3 days with a Dean-Stark tube to remove water. The resulting mixture was cooled to room temperature. The organic phase was washed with water (2 x 50 mL), brine (50 mL), and dried over anhydrous $Na_2SO_4$. Evaporation of the solvent resulted in a yellowish oily residue. Column chromatography on silica gel (230-400 mesh, 100 mL) with 0-6% ether gradient in hexanes as eluent afforded 0.1 g of pure VI and 1.3 g of a mixture of VI and the starting material. 1 H NMR (400 MHz, $CDCl_3$) δ: 5.27-5.45 (8H, m, 4 x CH=CH), 4.28-4.38 (1 H, m, OCH), 4.15 (1 H, dd, OCH), 3.80 (1 H, dd, OCH), 3.47 (1 H, dd, CHBr), 3.30 (1 H, dd, CHBr), 2.78 (4H, t, 2 x C=C-$CH_2$-C=C), 2.06 (8H, q, 4 x allylic $CH_2$), 1.52-1.68 (4H, m, 2 x $CH_2$), 1.22-1.45 (32H, m), 0.86-0.94 (6H, m, 2 x $CH_3$) ppm.

Synthesis of 2,2-Dilinoleyl-4-dimethylaminomethyl-[1,3]-dioxolane (DLin-K-DMA)

Anhydrous dimethyl amine was bubbled into an anhydrous THF solution (100 mL) containing 1.3 g of a mixture of 2,2-dilinoleyl-4-bromomethyl-[1,3]-dioxolane (VI) and dilinoleyl ketone (V) at 0°C for 10 min. The reaction flask was then sealed and the mixture stirred at room temperature for 6 days. Evaporation of the solvent left 1.5 g of a residual. The crude product was purified by column chromatography on silica gel (230-400 mesh, 100 mL) and eluted with 0-5% methanol gradient in dichloromethane. This gave 0.8 g of the desired product DLin-K-DMA. 1 H NMR (400 MHz, $CDCl_3$) δ: 5.25-5.45 (8, m, 4x CH=CH), 4.28-4.4 (1 H, m, OCH), 4.1 (1 H, dd, OCH), 3.53 (1 H, t OCH), 2.78 (4H, t, 2 x C=C-$CH_2$-C=C), 2.5-2.65 (2H, m, $NCH_2$), 2.41 (6H, s, 2 x $NCH_3$), 2.06 (8H, q, 4 x allylic $CH_2$), 1.56-1.68 (4H, m, 2 x $CH_2$),

1.22-1.45 (32H, m), 0.90 (6H, t, 2 x $CH_3$) ppm.

EXAMPLE 2

Synthesis OF 1,2-DILINOLEYLOXY-N,N-DIMETHYL-3-AMINOPROPANE (DLINDMA)

DLinDMA was synthesized as described below.

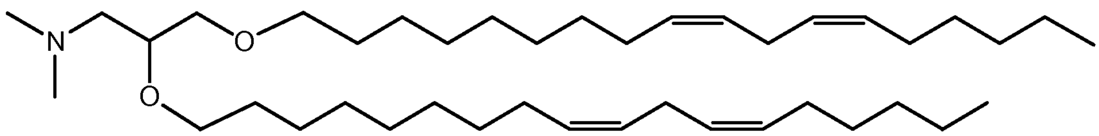

1,2-Dilinoleyloxy-3-dimethylaminopropane (DLinDMA)

To a suspension of NaH (95%, 5.2 g, 0.206 mol) in 120 mL of anhydrous benzene was added dropwise N,N-dimethyl-3-aminopropane-1,2-diol (2.8 g, 0.0235 mol) in 40 mL of anhydrous benzene under argon. Upon addition, the resulting mixture was stirred at room temperature for 15 min. Linoleyl methane sulfonate (99%, 20 g, 0.058 mol) in 75 mL of anhydrous benzene was added dropwise at room temperature under argon to the above mixture. After stirred at room temperature for 30 min., the mixture was refluxed overnight under argon. Upon cooling, the resulting suspension was treated dropwise with 250 mL of 1:1 (V:V) ethanol-benzene solution. The organic phase was washed with water (150 mL), brine (2 x 200 mL), and dried over anhydrous sodium sulfate. Solvent was evaporated in vacuo to afford 17.9 g of light oil as a crude product. 10.4 g of pure DLinDMA were obtained upon purification of the crude product by column chromatography twice on silica gel using 0-5% methanol gradient in methylene chloride. 1 H NMR (400 MHz, $CDCl_3$) δ: 5.35 (8H, m, CH=CH), 3.5 (7H, m, OCH), 2.75 (4H, t, 2 x $CH_2$), 2.42 (2H, m, $NCH_2$), 2.28 (6H, s, 2 x $NCH_3$), 2.05 (8H, q, vinyl $CH_2$), 1.56 (4H, m, 2 x $CH_2$), 1.28 (32H, m, 16 x $CH_2$), 0.88 (6H, t, 2 x $CH_3$) ppm.

EXAMPLE 3

SYNTHESIS OF 1 ,2-DILINOLEYLOXY-3-TRIMETHYLAMINOPROPANE CHLORIDE (DLIN-TMA.CL)

DLin-TMA.Cl was synthesized as shown in the schematic and described below.

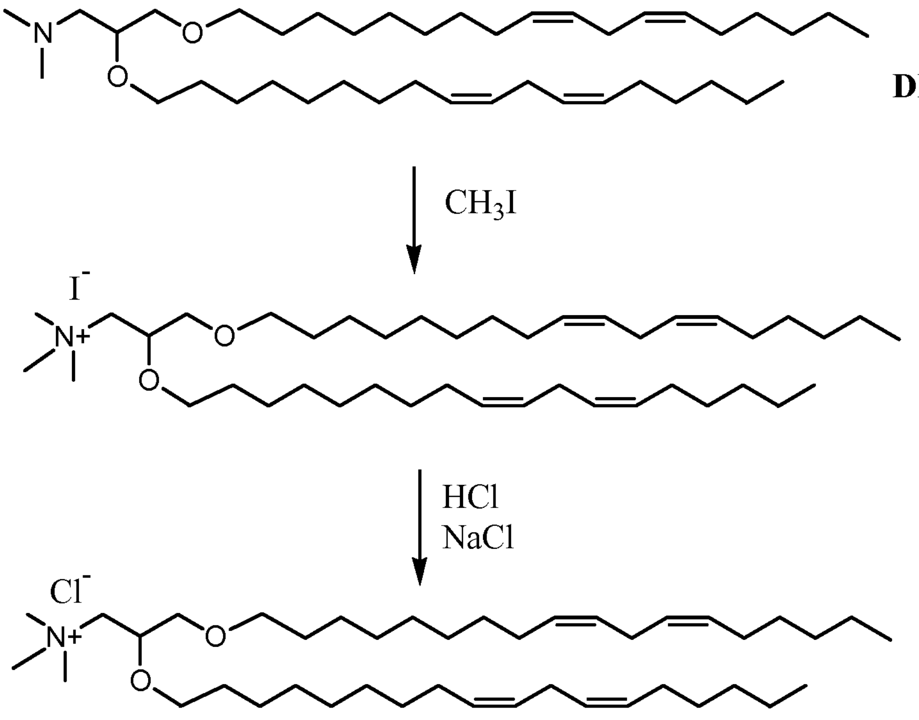


**DLin-DMA**

**DLin-TMA.I**

**DLin-TMA.Cl**

Synthesis of 1,2-Dilinoleyloxy-3-dimethylaminopropane (DLin-DMA)

DLin-DMA was prepared as described in Example 2, based on etherification of 3-dimethylamino-1,2-propanediol by linoleyl methane sulfonate.

Synthesis of 1,2-Dilinoleyloxy-3-trimethylaminopropane Iodide (DLin-TMA.I)

A mixture of 1,2-dilinoleyloxy-3-dimethylaminopropane (DLin-DMA, 5.5g, 8.9 mmol) and $CH_3I$ (7.5 mL, 120 mmol) in 20 mL of anhydrous $CH_2Cl_2$ was stirred under nitrogen at room temperature for 7 days. Evaporation of the solvent and excess of iodomethane afforded 7.0 g of yellow syrup as a crude DLin-TMA.I which was used in the following step without further purification.

Preparation of 1,2-Dilinoleyloxy-3-trimethylaminopropane Chloride (DLin-TMA.Cl)

The above crude 1,2-dilinoleyloxy-3-trimethylaminopropane iodide (DLin-TMA.I, 7.0 g) was dissolved in 150 mL of $CH_2Cl_2$ in a separatory funnel. 40 mL of 1 N HCl methanol solution was added, and the resulting solution was shaken well. To the solution was added 50 mL of brine, and the mixture was shaken well. The organic phase was separated. The aqueous phase was extracted with 15 mL of CH2Cl2. The organic phase and extract were then combined. This completed the first step of ion exchange. The ion exchange step was repeated four more times. The final organic phase was washed with brine (100 mL) and dried over anhydrous $Na_2SO_4$. Evaporation of the solvent gave 6.0 g of yellow oil. The crude product was purified by column chromatography on silica gel (230-400 mesh, 250 mL) eluted with 0-15% methanol gradient in chloroform. This afforded 2.3 g of 1,2-dilinoleyloxy-3-trimethylaminopropane chloride (DLin-DMA.Cl) as a colourless syrup. 1 H NMR (400 MHz, $CDCl_3$) $\delta$: 5.26-5.46 (8H, m, 4 x CH=CH), 3.95-4.15 (2H, m, $NCH_2$), 3.71 (1 H, dd, OCH), 3.35-3.65 (6H, m, 3 x $OCH_2$), 3.51 (9H, s, 3 x $NCH_3$), 2.77 (4H, t, 2 x C=C-$CH_2$-C=C), 2.05 (8H, q, 4 x allylic $CH_2$), 1.75-2.0 (2H, br.), 1.49-1.75 (4H, m, 2 x $CH_2$), 1.2-1.45 (30H, m), 0.89 (6H, t, 2 x $CH_3$) ppm.

EXAMPLE 4

SYNTHESIS OF 1,2-DIOLEYLOXY-N,N-DIMETHYL-3-AMINOPROPANE (DODMA)

DODMA was synthesized as indicated below.

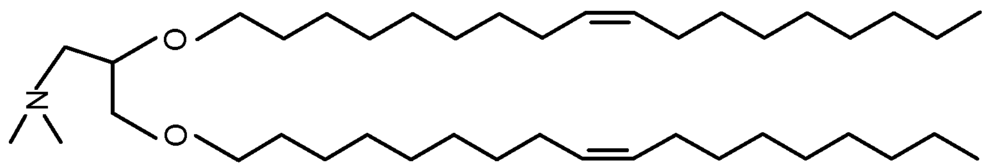

1,2-Dioleyloxy-3-dimethylaminopropane (DODMA) DLinDMA was synthesized in the same manner, except that oleyl mesylate was replaced with linoley mesylate.

Benzene (800 mL) was added to sodium hydride (52g, 95%, 2.06 mol) in a 3L pear-shaped round bottom flask with a stir bar under argon. A solution of N,N-dimethylaminopropane-1,2-diol (28.1g, 234.8 mmol) in benzene (200mL) was slowly added to the reaction flask under argon, rinsing with a further 50mL of benzene and allowed to stir for 10 minutes.

Oleyl mesylate (200.3g, 578.9 mmol) in benzene (200mL) was added to the reaction mixture under argon and rinsed with a further 1200mL of benzene. The reaction mixture was allowed to reflux under argon overnight.

The reaction mixture was transferred to 4L erlenmeyer flask and ethanol (100 mL) was added slowly under argon to quench unreacted sodium hydride. Additional ethanol (1300 mL) was added to give a total ethanol content of 1400mL such that benzene:ethanol is 1:1. The reaction mixture (800mL) was aliquoted to a 2L separatory funnel and 240mL water was added (benzene:ethanol:water 1:1:0.6 v/v). The organic phase was collected and the aqueous layer was re-extracted with benzene (100mL).

Oleyl mesylate (200.3g, 578.9 mmol) in benzene (200mL) was added to the reaction mixture under argon and rinsed with a further 1200mL of benzene. The reaction mixture was allowed to reflux under argon overnight. This step was repeated again.

The combined organic fractions were dried with anhydrous magnesium sulphate (30g) and filtered under vacuum using a sintered glass funnel. Solvent was removed on a rotovap (water bath 50 - 60□C). The viscous oily product was redissolved in dichloromethane (300 mL) and vacuum filtered through a sintered glass funnel with a filter paper and

silica gel 60 (80g, 230 - 400 mesh). Dichloromethane was removed on a rotovap at 50 - 60□C.

The product was purified by column chromatography. A total of 151 g product was divided into two ~75g aliquots and loaded onto two 600g silica gel 60 columns. The product was dissolved in 2% MeOH in dichloromethane (~1:1 w/v) prior to loading onto the column. 2% MeOH in dichloromethane (~1 L) was used until product came out. Approximately 1 L of 5%, 7.5% and then 10% MeOH in dichloromethane were used to elute the columns collecting ~200 mL fractions.

Fractions with a top or bottom spot on TLC (impurity) and product were rotovaped separately from the pure fractions. Impure DODMA was collected from other batches, added together, and put down a column a second time to purify. The yield of DODMA was 95g.

EXAMPLE 5

SYNTHESIS OF 1,2-DILINOLEYLOXY-3-(N-METHYLPIPERAZINO) PROPANE (DLIN-MPZ)

DLin-MPZ was synthesized as shown in the schematic diagram and described below.

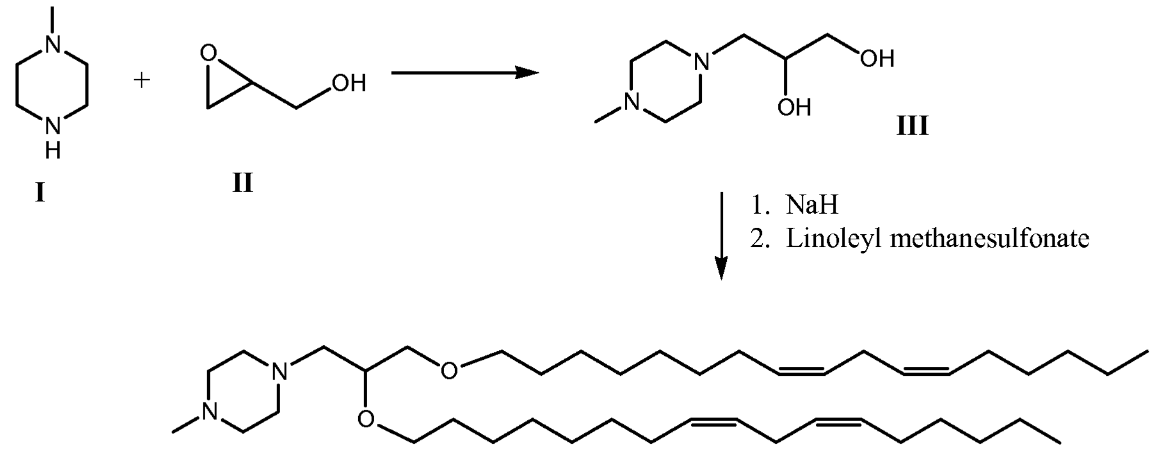


I II III

DLin-MPZ

Synthesis of 3-(N-methylpiperazino)-1,2-propanediol (III)

To a solution of 1-methylpiperazine (1.02g, 10.2 mmol) in anhydrous $CH_2Cl_2$ (100 mL) was added dropwise glycidol (0.75g, 9.7 mmol). The resulting mixture was stirred at room temperature for 2 days. Evaporation of the solvent gave an oily residual. The residual was re-dissolved in 100 mL of benzene. 1.8 g of viscous oil was obtained as a crude product after the solvent was evaporated. The crude product was used in the following step without further purification.

Synthesis of 1,2-Dilinoleyloxy-3-N-methylpiperazinopropane (DLin-MPZ)

To a suspension of NaH (2.0g, 60%, 50 mmol) in 100 mL of anhydrous benzene under nitrogen was added dropwise a solution of 3-(N-methylpiperazino)-1,2-propanediol (III, 0.74g, 4.2 mmol) in 5 mL of anhydrous benzene. The resulting mixture was stirred at room temperature for 20 min. A solution of linoleyl methane sulfonate (3.2g, 9.3 mmol) in 20 mL of anhydrous benzene was then added dropwise. After stirred at room temperature for 20 min, the mixture was refluxed under nitrogen overnight. Upon cooling, 40 mL of 1:1 (V:V) ethanol-benzene was added slowly to the mixture followed by additional 60 mL of benzene and100 mL of EtOH. The organic phase was washed with water (200 mL) and dried over anhydrous $Na_2SO_4$. Evaporation of the solvent gave 3.0 g of yellow oil as a crude product. The crude product was purified by repeated column chromatography on silica gel (230-400 mesh, 250 mL) eluted with 0-8% methanol gradient in chloroform. This afforded 1.1 g (39%) 1,2-dilinoleyloxy-3-N-methylpiperazinopropane (DLin-MPZ) as yellowish oil. 1 H NMR (400 MHz, $CDCl_3$) $\delta$: 5.27-5.45 (8H, m, 4 x CH=CH), 3.37-3.65 (7H, m, OCH and 3 x $OCH_2$), 2.77 (4H, t, 2 x C=C-$CH_2$-C=C), 2.33-2.74 (10H, br. and m, 5 x $NCH_2$), 2.31 (3H, s, $NCH_3$), 2.06 (8H, q, 4 x allylic $CH_2$), 1.49-1.63 (4H, m, 2 x $CH_2$), 1.2-1.45 (32H, m), 0.89 (6H, t, 2 x $CH_3$) ppm.

EXAMPLE 6

SYNTHESIS OF 3-(N,N-DILINOLEYLAMINO)-1,2-PROPANEDIOL (DLINAP)

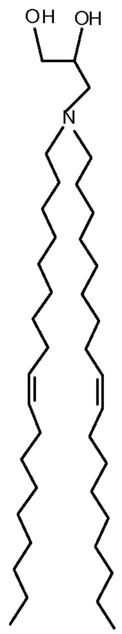

DOAP
3-(Dioleylamino)-1,2-propanediol

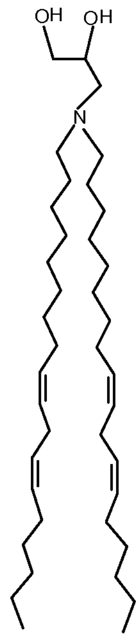

DLinAP
3-(Dilinoleylamino)-1,2-propanediol

The procedure described below was followed to synthesize DOAP, and DLinAP was synthesized in the same manner except that linoleyl methane sulfonate was used instead of oleyl Br.

Step 1:

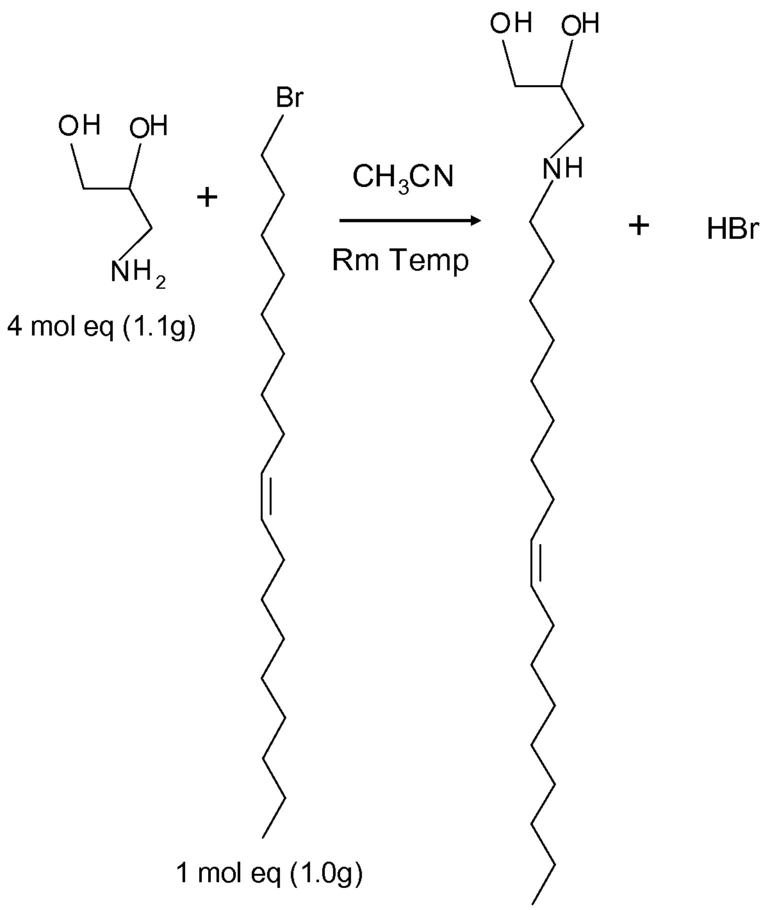


(±)-3-Amino-1,2-propanediol was alkylated with oleyl bromide in acetonitrile at room temperature using 3.0 mol eq excess of the primary amine under nitrogen. The reaction was monitored by TLC. Loss of oleyl bromide was an indication of reaction completion. The product was precipitated as the hydrobromide salt.

Step 2:

The secondary amine from step 1 (0.9g, 1 mol eq), N,N-diisopropylethylamine (Hunig's base) (0.5g, 1.5 mol eq), oleyl bromide (1.0g, 1.1 mol eq) and 20 mL of acetonitrile were placed in a round bottom flask and stirred at room temperature. The completion of the reaction was followed by TLC. The reaction mixture was taken to dryness in the rotovap. Residue was dissolved in $CH_2Cl_2$ (10 - 20 mL) and washed with distilled water (10 - 20 mL). The aqueous layer was washed with 3 x $CH_2Cl_2$ (10 -20 mL). The combined organic fractions were dryed over MgSO4 and solvent was removed with a rotovap and purified by column chromatography.

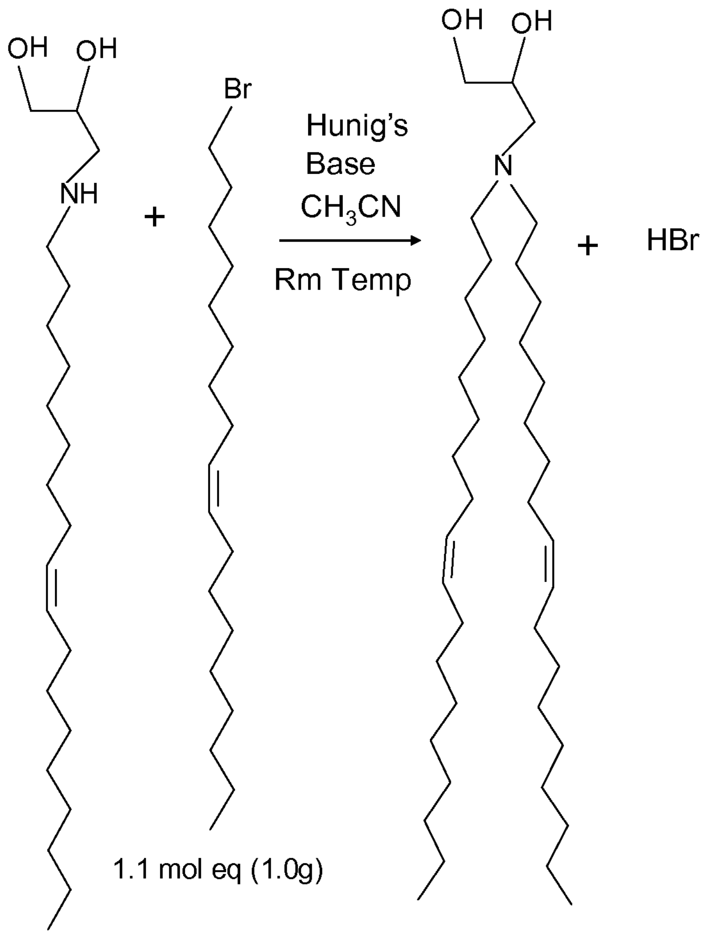


1.1 mol eq (1.0g)

1 mol eq (0.9g)

EXAMPLE 7

SYNTHESIS OF 2-LINOLEYOLOXYL-3-LINOLEYLOXYL-1-N,N-DIMETHYLAMINOPROPANE (DLIN-2-DMAP)

DLin-2-DMAP was synthesized as shown in the schematic diagram and described below.

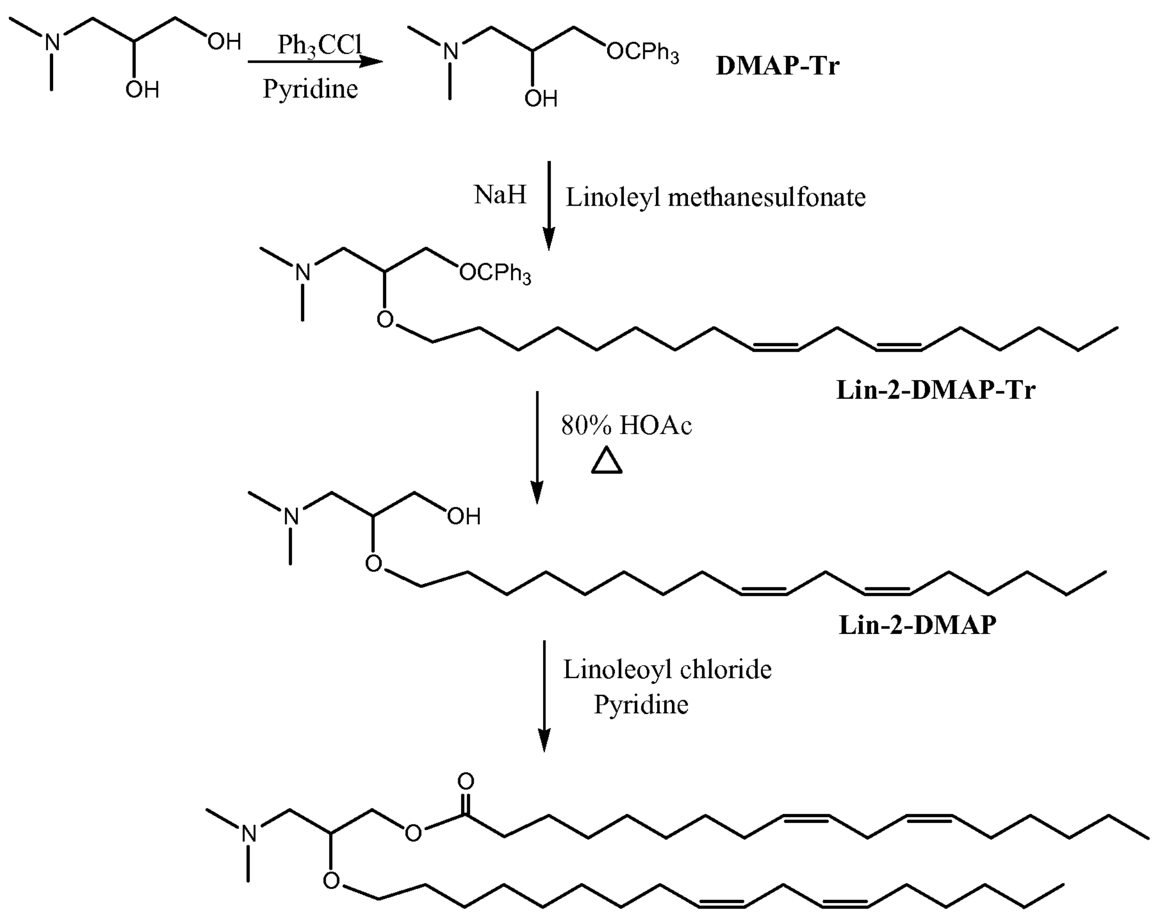


DLin-2-DMAP

Synthesis of 1-Triphennylmethyloxy-3-(N,N-dimethylamino)-2-propanol (DMAP-Tr)

A mixture of 3-(dimethylamino)-1,2-propanediol (3.0 g, 25 mmol) and triphenylmethyl chloride (7.75 g, 27.8 mmol) in dry pyridine (100 mL) was refluxed for 30 min. Upon cooling, most of the solvent was evaporated in vacuo, and the resulting residual was re-dissolved in 400 mL of dichloromethane. The organic phase was washed with water (3 x 200 mL), brine (150 mL), and dried over anhydrous $Na_2SO_4$. Evaporation of the solvent gave 6.3 g of yellow oil as a crude product. The crude product was purified by column chromatography on silica gel (230-400 mesh, 500 mL) eluted with 0-10% methanol gradient in dichloromethane. This afforded 4.0 g of the product (DMAP-Tr) as yellow oil.

Synthesis of 1-Triphenylmethyloxy-2-linoleyloxy-3-N,N-dimethylanninopropane (Lin-2-DMAP-Tr)

NaH (60%, 2.17 g, 54 mmol) was washed with hexanes (3 x 40 mL) under nitrogen and then suspended in anhydrous benzene (60 mL). To the suspension was added dropwise DMAP-Tr (4.0 g, 11 mmol) in 20 mL of anhydrous benzene. Upon stirring of the resulting mixture at room temperature for 20 min, a solution of linoleyl methanesulfonate (4.5 g, 13 mmol) in 40 mL of anhydrous benzene was added dropwise under nitrogen. The mixture was stirred at room temperature for 30 min and then refluxed overnight. Upon cooling to room temperature, 30 mL of 1:1 (V:V) ethanol-benzene solution were added dropwise under nitrogen followed by 100 mL of benzene and 100 mL of water. Upon shaking, the aqueous phase was separated. The organic phase was washed with brine (2 x 100 mL) and dried over anhydrous sodium sulfate. Evaporation of the solvent afforded 6.8 g of yellowish oil. The crude product was chromatographed on a silica gel column (230-400 mesh, 400 mL) eluted with 0-3% methanol gradient in chloroform. 5.8 g (84%) of the desired product (Lin-2-DMAP-Tr) were obtained as yellowish oil.

Synthesis of 2-Linoleyloxy-3-(N,N-dimethylamino)-1-propanol (Lin-2-DMAP)

Lin-2-DMAP-Tr (5.8 g, 9.2 mmoL) was refluxed in 80% HOAc (25 mL) under nitrogen for 10 min. Upon cooling

to room temperature, the mixture was diluted with water (100 mL). The resulting aqueous solution was neutralized to about pH 6 with 0.5% NaOH solution. The aqueous phase was then extracted with dichloromethane (4 x 100 mL). The combined organic phase was washed with 0.1% NaOH solution (100 mL), water (100 mL), brine (100 mL), and dried over anhydrous sodium sulfate. Evaporation of the solvent gave 5.6 g of a mixture of product and starting material as yellowish oil. The mixture was chromatographed on a silica gel column (230-400 mesh, 400 mL) eluted with 0-10% methanol gradient in chloroform. 2.2 g (62%) of the desired product (Lin-2-DMAP) were afforded as yellowish oil. 1 H NMR (400 MHz, $CDCl_3$) $\delta$: 5.28-5.43 (4H, m, CH=CH), 4.25 (1 H, br, OH), 3.78 (1 H, dd, J = 11 and 4.8 Hz, OCH), 3.68 (1 H, dd, J = 11 and 6.8 Hz, OCH), 3.49 (3H, m, OCH and $OCH_2$), 2.77 (2H, t, $=CH-CH_2-CH=$), 2.50-2.65 (2H, m, $NCH_2$), 2.32 (6H, s, 2 x $NCH_3$), 2.05 (4H, q, allylic 2 x $CH_2$), 1.55 (2H, m, $CH_2$), 1.30 (16H, m, 8 x $CH_2$), 0.89 (3H, t, $CH_3$) ppm.

Synthesis of 2-Linoleyoloxyl-3-linoleyloxyl-1-N,N-dimethylaminoproprane (DLin-2-DMAP)

To a solution of linoleic acid (2.36 g, 8.4 mmol) in anhydrous benzene (50 mL) was added dropwise oxalyl chloride (1.45 g, 11.4 mmol) under nitrogen. The resulting mixture was stirred at room temperature for 4 hours. Solvent and excess of oxalyl chloride was removed in vacuo to give linoleyol chloride as light yellowish oil.

The above linoleyol chloride was re-dissolved in anhydrous benzene (85 mL). To the resulting solution was added dropwise a solution of Lin-2-DMAP (2.9 g, 7.5 mmol) and dry pyridine (1 mL) in 15 mL of anhydrous benzene. The mixture was then stirred at room temperature under nitrogen for 2 days and a suspension was resulted. The mixture was diluted with benzene (100 mL). The organic phase was washed with a solution of 3:5 (V:V) ethanol-water (320 mL), brine (2 x 75 mL), and dried over anhydrous Na2SO4. The solvent was removed in vacuo affording 5.2 g of oil. The crude product was purified by column chromatography on silica gel (230-400 mesh, 450 mL) eluted with 0-4% methanol gradient in chloroform. This afforded 3.9 g (80%) of DLin-2-DMAP as yellowish oil. 1 H NMR (400 MHz, $CDCl_3$) $\delta$: 5.25 (8H, m, 4 x CH=CH), 4.17 (1 H, dd, J = 11.6 and 4 Hz, OCH), 3.96 (1 H, dd, J = 11.6 and 5.2 Hz, OCH), 3.53-3.64 (1 H, m, OCH), 3.35-3.53 (2H, m, $OCH_2$), 2.68 (4H, t, $=CH-CH_2-CH=$), 2.41 (2H, m, $CH_2$), 2.25 (6H, s, 2 x $NCH_3$), 2.21 (2H, m, $CH_2$), 1.96 (8H, q, allylic 4 x $CH_2$), 1.4-1.6 (4H, m, 2 x $CH_2$), 1.21 (30H, s, 15 x $CH_2$), 0.80 (6H, t, 2 x $CH_3$) ppm.

EXAMPLE 8

SYNTHESIS OF 1,2-DILINOLEYLOXY-3-(2-N,N-DIMETHYLAMINO) ETHOXYPROPANE (DLIN-EG-DMA)

DLin-EG-DMA was synthesized as shown in the schematic diagram and described below.

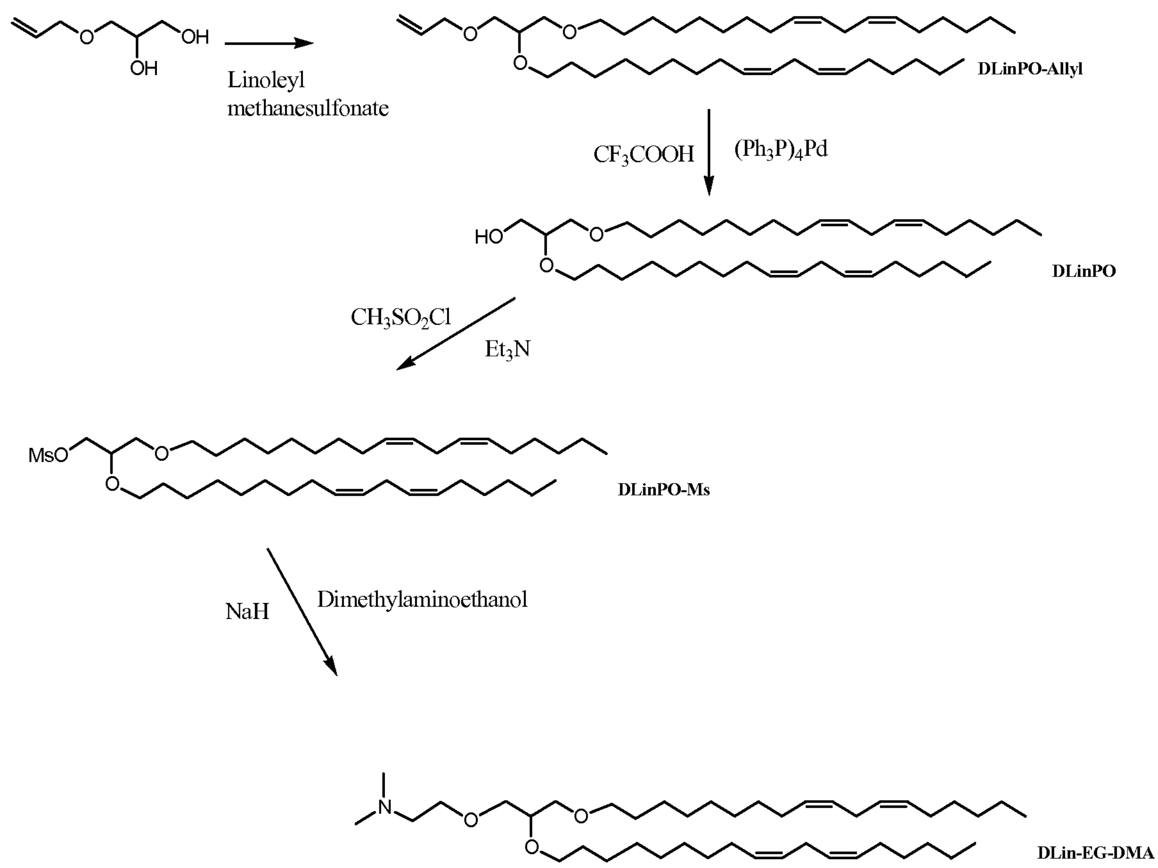


Synthesis of 1,2-Dilinoleyloxy-3-allyloxypropane (DLinPO-Allyl)

NaH (10g, 60%, 250 mmol) was washed three times with hexanes (3 x 75 mL) under nitrogen and then suspended in 200 mL of anhydrous benzene. To the NaH suspension was added dropwise a solution of 3-allyloxy-1,2-propanediol (4.2g, 32 mmol) in 10 mL of anhydrous benzene. The resulting mixture was stirred at room temperature for 15 min. A solution of linoleyl methane sulfonate (25.8g, 74.9 mmol) in 90 mL of anhydrous benzene was then added dropwise. The resulting mixture was stirred under nitrogen at room temperature for 30 min and the refluxed overnight. Upon cooling, 100 mL of 1:1 (V:V) ethanol-benzene was added slowly to the mixture followed by additional 300 mL of benzene. The organic phase was washed with water (300 mL), brine (2 x 300 mL), and dried over anhydrous Na2SO4. Evaporation of the solvent gave 22.2 g of yellow oil as a crude product. The crude product was purified by column chromatography on silica gel (230-400 mesh, 1200 mL) eluted with 0-8% ether gradient in hexanes. This afforded 12.4 g (62%) 1,2-dilinoleyloxy-3-allyloxypropane (DLinPO-Allyl) as colourless oil.

Synthesis of 1,2-Dilinoleyloxy-3-hydroxypropane (DLinPO)

A mixture of 1,2-dilinoleyloxy-3-allyloxypropane (DLinPO-Allyl, 4.8g, 7.6 mmol), tetrakis(triphenylphosphine) palladium (1.2 g, catalyst) and trifluoroacetic acid (5 mL) in ethanol (80 mL) was refluxed in dark under nitrogen overnight (25 hours). A brownish solution was resulted. Volume of the mixture was reduced by half by evaporation of the solvent, and the resulting residual was dissolved in 200 mL of ethyl acetate. The organic phase was washed with water (2 x 100 mL), brine (100 mL), and dried over anhydrous $Na_2SO_4$. Evaporation of the solvent gave 5.5 g of yellowish oil as a crude product. The crude product was purified by repeated column chromatography on silica gel (230-400 mesh, 100 mL) eluted with 0-2% methanol gradient in dichoromethane. This afforded 2.8 g (63%) 1,2-dilinoleyloxy-3-hydroxypropane (DLinPO) as yellowish oil. 1 H NMR (400 MHz, $CDCl_3$) $\delta$: 5.27-5.45 (8H, m, 4 x CH=CH), 3.67-3.78 (1 H, dd, OCH), 3.58-3.67 (2H, m, $OCH_2$), 3.4-3.58 (6H, m, 3 x $OCH_2$), 2.78 (4H, t, 2 x C=C-$CH_2$-C=C), 2.06 (8H, q, 4 x allylic $CH_2$), 1.49-1.67 (4H, m, 2 x $CH_2$), 1.23-1.45 (32H, m), 0.90 (6H, t, 2 x $CH_3$) ppm.

Synthesis of 1,2-Dilinoleyloxy-3-methysulfonyoxypropane (DLinPO-Ms)

To a solution of 1,2-dilinoleyloxy-3-hydroxypropane (DLinPO, 3.6g, 6.1 mmol) and anhydrous triethylamine

(1.6 mL, 11.5 mmol) in 100 mL of anhydrous dichloromethane under nitrogen was added dropwise methylsulfonyl chloride (0.8 mL, 9.1 mmol). The resulting mixture was stirred at room temperature overnight (23 hours). The reaction mixture was diluted with 100 mL of dichloromethane. The organic phase was washed water (2 x 100 mL), brine (100 mL), and dried over anhydrous Na2SO4, Evaporation of the solvent resulted in 4.1 g of brownish oil as a crude product, DLinPO-Ms. The crude product was used in the following step without further purification.

Synthesis of 1,2-Dilinoleyloxy-3-(2-N,N-dimethylamino)ethoxypropane (DLin-EG-DMA)

NaH (1.37g, 60%, 34.2 mmol) was washed twice with hexanes (2 x 15 mL) under nitrogen and then suspended in 120 mL of anhydrous benzene. To the NaH suspension was added dropwise a solution of dimethylaminoethanol (0.44g, 4.9 mmol) in 10 mL of anhydrous benzene. The resulting mixture was stirred at room temperature for 20 min. A solution of 1,2-dilinoleyloxy-3-methylsulfonyoxypropane (DLinPO-Ms, 3.4g, 5.1 mmol) in 20 mL of anhydrous benzene was then added dropwise. The resulting mixture was stirred under nitrogen at room temperature for 20 min and the refluxed overnight. Upon cooling, 100 mL of 1:1 (V:V) ethanol-benzene was added slowly to the mixture followed by additional 50 mL of benzene and 70 mL of ethanol. The organic phase was washed with water (200 mL), and dried over anhydrous $Na_2SO_4$. Evaporation of the solvent gave 3.2 g of yellowish oil as a crude product. The crude product was purified by column chromatography on silica gel (230-400 mesh, 300 mL) eluted with 0-6% methanol gradient in chloroform. This afforded 0.34 g (11%) 1,2-dilinoleyloxy-3-(2-N,N-dimethylamino)ethoxypropane (DLin-EG-DMA) as pale oil. 1 H NMR (400 MHz, $CDCl_3$) $\delta$: 5.27-5.46 (8H, m, 4 x CH=CH), 3.62 (2H, t, $OCH_2$), 3.35-3.60 (9H, m, OCH and 4 x $OCH_2$), 2.78 (4H, t, 2 x C=C-$CH_2$-C=C), 2.61 (2H, t, $NCH_2$), 2.35 (6H, s, 2 x $NCH_3$), 2.05 (8H, q, 4 x allylic $CH_2$), 1.49-1.65 (4H, m, 2 x $CH_2$), 1.23-1.45 (32H, m), 0.90 (6H, t, 2 x $CH_3$) ppm.

EXAMPLE 9

SYNTHESIS OF 1,2-DILINOLEYLOXY-3-(DIMETHYLAMINO)ACETOXYPROPANE (DLIN-DAC)

DLin-DAC was synthesized as indicated in the schematic diagram and described below.

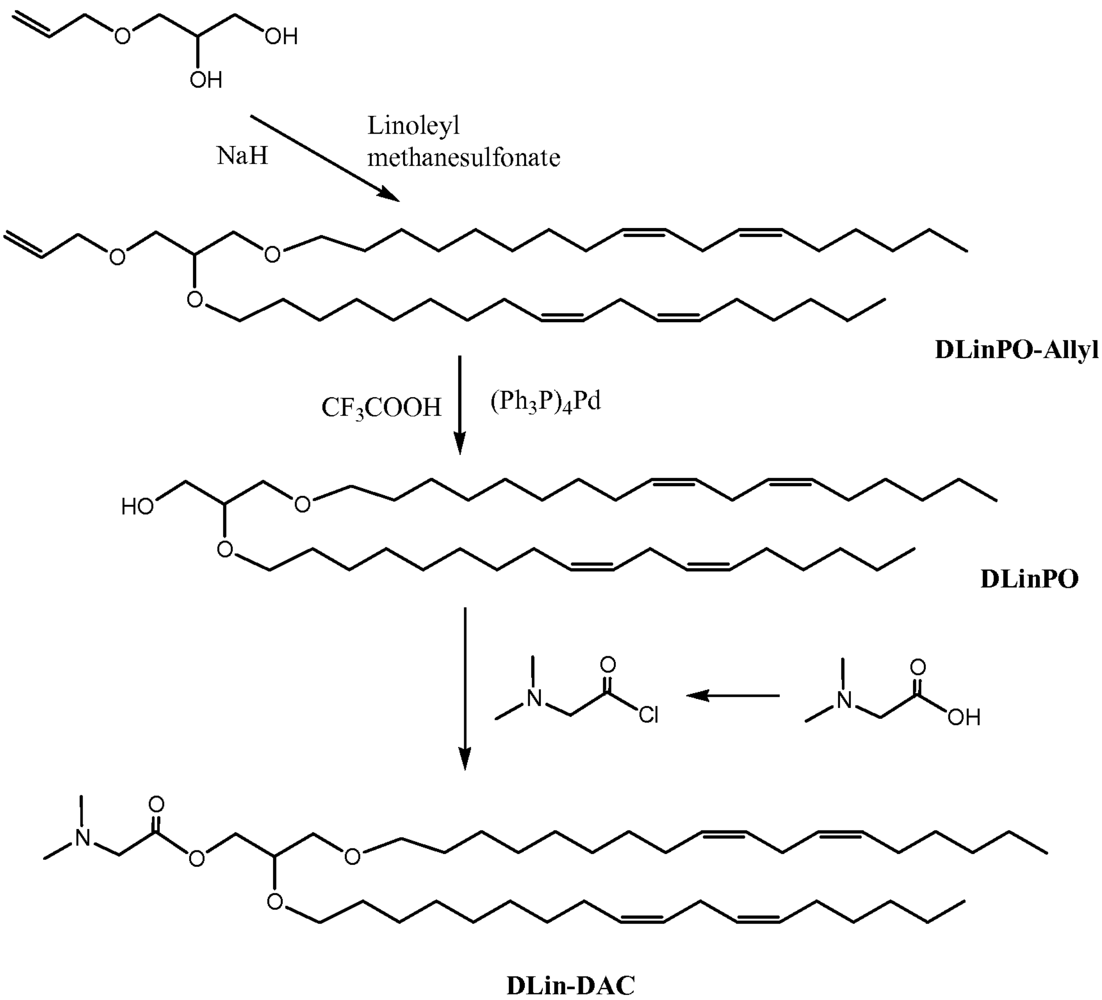


Synthesis of 1,2-Dilinoleyloxy-3-allyloxypropane (DLinPO-Allyl)

NaH (60%, 10 g, 250 mmol) was washed with hexanes (3 x 75 mL) under nitrogen and then suspended in anhydrous benzene (200 mL). To the suspension was added dropwise 3-allyloxy-1,2-propanediol (4.2 g, 32 mmol) in 10 mL of anhydrous benzene. Upon stirring of the resulting mixture at room temperature for 10 min, a solution of linoleyl methanesulfonate (25.8 g, 74.9 mmol) in 90 mL of anhydrous benzene was added dropwise under nitrogen. The mixture was stirred at room temperature for 30 min and then refluxed overnight. Upon cooling to room temperature, 100 mL of 1:1 (V:V) ethanol-benzene solution were added dropwise under nitrogen followed by 300 mL of benzene. The organic phase was washed with water (300 mL), brine (2 x 300 mL) and dried over anhydrous sodium sulfate. Evaporation of the solvent afforded 22.2 g of yellowish oil as a crude product. Column purification of the crude product (1200 mL silica gel, 230-400 mesh, eluted with 0-8% diethyl ether gradient in hexanes) afforded 12.4 g (62%) of colourless oil DLinPO-Allyl.

Synthesis of 2,3-Dilinoleyloxy-1-propanol (DLinPO)

To a solution of DLinPO-Allyl (12.4 g, 19.7 mmol) in 180 mL of ethanol was added trifluoroacetic acid (13 mL) followed by tetrakis(triphenylphosphine) palladium (3.1 g, 2.7 mmol). The resulting suspension was refluxed under nitrogen in dark overnight. After evaporation of the solvent, ethyl acetate (400 mL) was added to the residual. The organic phase was washed with water (2 x 100 mL), brine (100 mL), and dried over anhydrous Na2SO4. 12 g of yellowish oil were resulted upon removal of the solvent. The oily material was purified by column chromatography on silica gel (230-400 mesh, 500 mL) eluted with 0-1.5% methanol gradient in dichloromethane. This afforded 5.8 g (50%) of the product DLinPO.

Synthesis of 1,2-Dilinoleyloxy-3-(dimethylamino)acetoxypropane (DLin-DAC)

N,N-Dimethylglycine hydrochloride (1.0 g, 6.7 mmol) was refluxed in 5 mL of oxalyl chloride for 60 min. The excess of oxalyl chloride was removed in vacuo. To the residual was added 50 mL of anhydrous benzene, and the solvent was evaporated to give a slightly brownish solid. The crude N,N-dimethylglycine acylchloride salt was used in the following step directly.

The above crude acylchloride was suspended in 50 mL of anhydrous dichloromethane under nitrogen. To the suspension was added dropwise a solution of DLinPO (1.0 g, 1.7 mmol) and dry triethylamine (1.4 mL, 11 mmol) in 20 mL of anhydrous dichloromethane. The resulting mixture was stirred at room temperature under nitrogen overnight. 100 mL of dichloromethane were then added. The organic phase was washed with water (2 x 75 mL), brine (75 mL), and dried over anhydrous Na2SO4. Evaporation of the solvent gave 1.1 g of light brownish oil as a mixture of the starting material and product. The desired product DLin-DAC, 0.24 g (20%), was isolated by column chromatography on silica gel (230-400 mesh, 200 mL) eluted with 0-40% ethyl acetate gradient in hexanes. 1 H NMR (400 MHz, $CDCl_3$) $\delta$: 5.36 (8H, m, 4 x CH=CH), 4.34 (1 H, dd, J = 11.2 and 3.6 Hz, OCH), 4.18 (1 H, dd, J = 11.6 and 5.6 Hz, OCH), 3.64 (1 H, m, OCH), 3.4-3.6 (6H, m, 3 x $OCH_2$), 3.34 (2H, s, $NCH_2$), 2.78 (4H, t, =CH-CH2-CH=), 2.50 (6H, s, 2 x $NCH_3$), 2.05 (8H, q, allylic 4 x $CH_2$), 1.5-1.63 (4H, m, 2 x $CH_2$), 1.3 (32H, m, 16 x $CH_2$), 0.90 (6H, t, 2 x $CH_3$) ppm.

EXAMPLE 10

SYNTHESIS OF 1,2-DILINOLEOYL-3-DIMETHYLAMINOPROPANE

1,2-Dilinoleoyl-3-N,N-dimethylaminopropane (DLin-DAP) was synthesized as described below.

To a solution of linoleic acid (99%, 49.7 g, 0.177 mol) in 800 mL of anhydrous benzene was added dropwise oxalyl chloride (99%, 29.8 g, 0.235 mol) under argon. Upon addition, the resulting mixture was stirred at room temperature for 2 hours until no bubble was released. The solvent and excess of oxalyl chloride was removed in vacuo. To the residual was added anhydrous benzene (1 L) followed by a solution of 3-N,N-dimethylamino-1,2-propanediol and dry pyridine in anhydrous benzene (100 mL) dropwise. The resulting mixture was stirred at room temperature for 2 days. Upon evaporation of the solvent, 64 g of yellowish syrup were afforded. 19 g of pure DLinDAP were obtained upon purification of the crude product by column chromatography three times on silica gel using 0-5% methanol gradient in chloroform. $^1$H NMR (400 MHz, $CDCl_3$) $\delta$: 5.49 (1 H, m), 5.43-5.26 (8H, m), 4.41 (1 H, dd), 4.13 (1 H, dd), 3.15-3.35 (2H, m), 2.82 (6H, s, 2 x $NCH_3$), 2.76 (4H, t), 2.35-2.6 (2H, m), 2.31 (2H, t), 2.03 (8H, q, vinyl $CH_2$), 1.53-1.68 (4H, m, 2 x $CH_2$), 1.2-1.4 (28H, m, 14 x $CH_2$), 0.88 (6H, t, 2 x $CH_3$) ppm.

EXAMPLE 11

SYNTHESIS OF DLIN-C-DAP

DLin-C-DAP was synthesized as shown in the schematic diagram and described below.

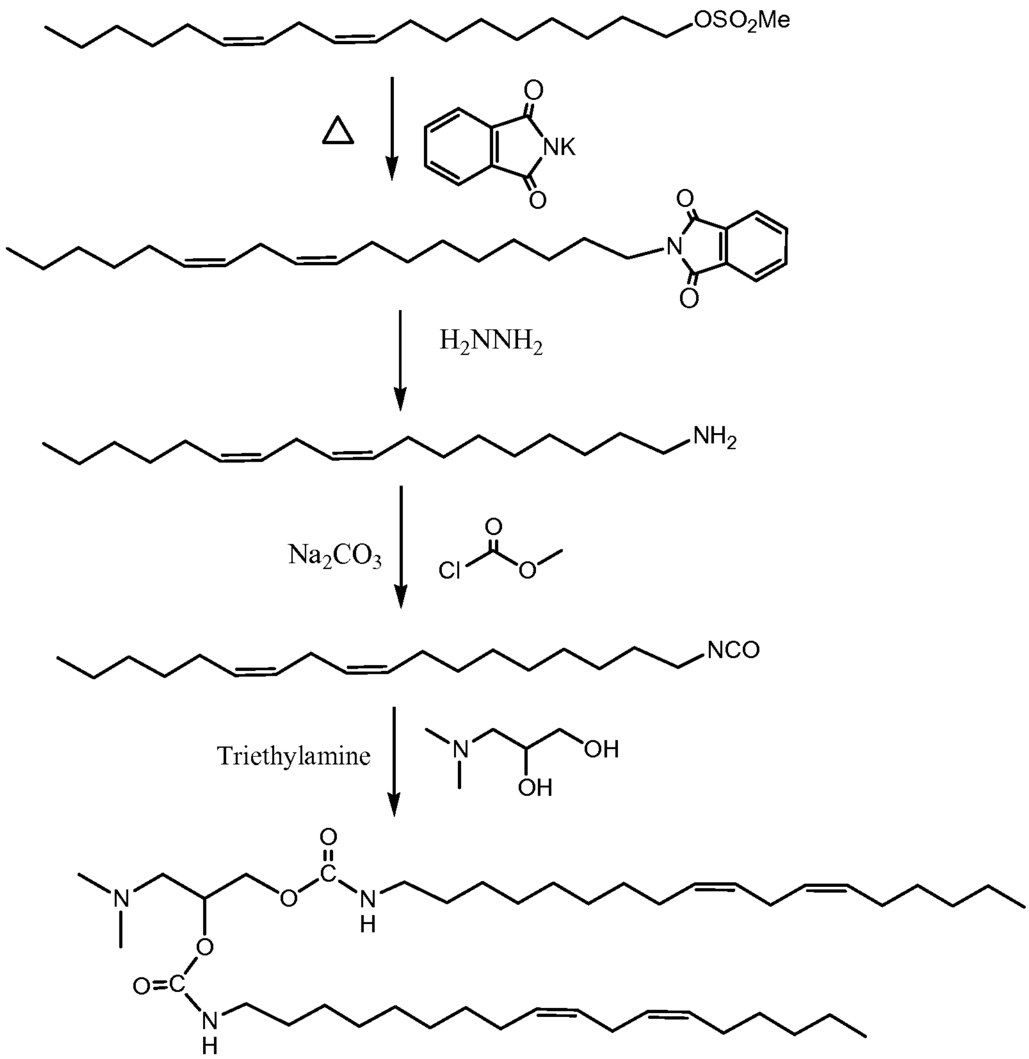


**DLin-C-DAP**

Preparation of Linoleyl Phthalimide

A mixture of potassium phthalimide (11.2 g, 59.5 mmol) and linoleyl methanesulfonate (9.3 g, 27 mmol) in 250 mL of anhydrous DMF was stirred at 70°C under nitrogen overnight. The resulting suspension was poured into 500 mL of cold water. The aqueous phase was extracted with EtOAc (3 x 200 mL). The combined extract was washed with water (200 mL), brine (200 mL), and dried over anhydrous $Na_2SO_4$. Solvent was evaporated to give a mixture of solid and oily materials. To the mixture was added 300 mL of hexanes. The solid was filtered and washed with hexanes (2 x 25 mL). The filtrate and washes were combined, and the solvent was evaporated to result in 11 g of yellow oil as a crude product. The crude product was used in the next step with further purification.

Preparation of Linoleylamine

The above crude linoleyl phthalimide (11 g, ca. 27 mmol) and hydrazine (10 mL) were refluxed in 350 mL of ethanol under nitrogen overnight. The resulting white solid was filtered upon cooling the mixture to about 40-50°C and the solid was washed with warm EtOH (2 x 30 mL). The filtrate and washes were combined and solvent evaporated. To the residual was added 400 mL of chloroform which resulted in precipitation of white solid. The solid was filtered again. The organic phase of the resulting filtrate was washed with water (2 x 100 mL), brine (100 mL), and dried over anhydrous Na2SO4. Solvent was removed in vacuo to afford 7.3 g of yellow oil as a crude product. This crude product was used in the next step without further purification. Pure linoleylamine was obtained by column chromatography on silica gel eluted with 0-20% methanol gradient in chloroform. 1 H NMR (400 MHz, $CDCl_3$) δ: 5.35 (4H, m, 2 x CH=CH), 2.76 (2H, t, J = 6.8 Hz, =CH-$CH_2$-CH=),), 2.68 (2H, t, J = 6.8 Hz, $NCH_2$), 2.04 (4H, q, allylic 2 x $CH_2$), 1.61 (2H, br., $NH_2$), 1.44

(2H, m, $CH_2$), 1.29 (18H, m, 9 x $CH_2$), 0.88 (6H, t, 2 x $CH_3$) ppm.

Preparation of Linoleyl Isocyanate

Anhydrous sodium carbonate (11 g g) was suspended in a solution of linoleylamine (7.3 g, ca. 27 mmol) in anhydrous $CH_2Cl_2$ (200 mL) under good stirring and nitrogen. The suspension was cooled to 0-5°C with an ice bath. To the suspension was added diphosgene (8.2 g, 41 mmol) in 10 mL of anhydrous $CH_2Cl_2$ under vigorous stirring. Upon addition, the resulting suspension was stirred at 0-5°C under nitrogen for 60 min and then at room temperature for 2 hours. Upon completion of the reaction, 100 mL of water was added to the mixture and the mixture was stirred at room temperature for 30 min. The organic layer was separated, and washed with water (100 mL) and brine (100 mL). After drying with anhydrous $Na_2SO_4$, the solvent was evaporated to give 7.6 g of yellow oil as a crude product. The crude product was used in the following step without further purification.

Condensation of Linoleyl Isocyanate with 3-(Dimethylamino)-1,2-propanediol

To a solution of the above crude linoleyl isocyanate (7.6 g, ca. 25 mmol) in 150 mL of anhydrous benzene under nitrogen was added dropwise a solution of 3-(dimethylamino)-1,2-propanediol (0.99 g, 8.3 mmol) in 20 mL of anhydrous benzene. The resulting mixture was stirred at room temperature for 60 min and then refluxed for 4 hours followed by stirring at room temperature overnight. Upon dilution of the mixture with 150 mL benzene, the organic phase was washed with water (3 x100 mL), brine (100 mL), and dried over anhydrous $Na_2SO_4$. Evaporation of the solvent gave 8.4 g of yellow oil. Column purification of the oily material (500 mL silica gel, 230-400 mesh, eluted with 0-3% methanol gradient in chloroform) afforded 2.2 g (38%) of yellowish oil as the product DLin-C-DAP. 1 H NMR (400 MHz, $CDCl_3$) δ: 5.37 (8H, m, 4 x CH=CH), 5.06 (1 H, br. CONH), 4.91 (1 H, br. CONH), 4.79 (1 H, m, OCH), 4.28 (1 H, br. d, J = 11 Hz, OCH), 4.16 (1 H, dd, J = 12 and 6 Hz, OCH), 3.16 (4H, m, 2 x $NCH_2$), 2.77 (4H, t, J = 6.4 Hz, =CH-$CH_2$-CH=), 2.4-2.7 (2H, m, $NCH_2$), 2.33 (6H, s, 2 x $NCH_3$), 2.05 (8H, m, allylic 4 x $CH_2$), 1.4-1.55 (4H, m, 2 x $CH_2$), 1.29 (40H, s, 20 x $CH_2$), 0.89 (6H, t, 2 x $CH_3$) ppm.

EXAMPLE 12

SYNTHESIS OF 1,2-DILINOLEYLOXY-3-MORPHOLINOPROPANE (DLIN-MA)

DLin-MA was synthesized as shown in the schematic diagram and described below.

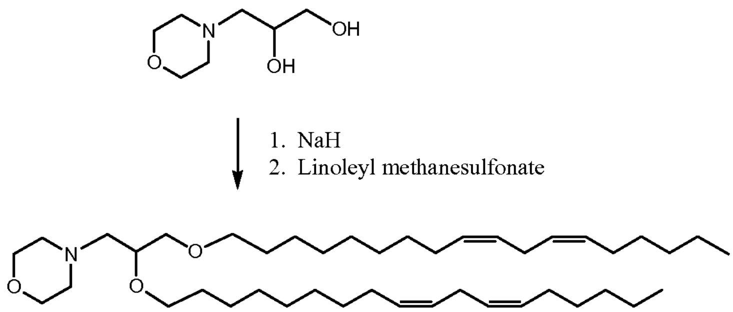


DLin-MA

To a suspension of NaH (7.6g, 95%, 0.30 mol) in 150 mL of anhydrous benzene under nitrogen was added dropwise a solution of 3-(N-morpholino)-1,2-propanediol (1.02g, 6.3 mmol) in 10 mL of anhydrous benzene. The resulting mixture was stirred at room temperature for 20 min. A solution of linoleyl methane sulfonate (5g, 14.5 mmol) in 20 mL of anhydrous benzene was then added dropwise. After stirred at room temperature for 20 min, the mixture was refluxed under nitrogen overnight. Upon cooling, 100 mL of 1:1 (V:V) ethanol-benzene was added slowly to the mixture followed by additional 90 mL of EtOH. The organic phase was washed with water (240 mL) and dried over anhydrous $Na_2SO_4$. Evaporation of the solvent gave yellow oil as a crude product. The crude product was purified by column chromatography on silica gel (230-400 mesh) eluted with 0-8% methanol gradient in dichloromethan. This afforded 2g of 1,2-dilinoleyloxy-3-N-morpholinopropane (DLin-MA) as yellowish oil. $^1$H NMR (400 MHz, $CDCl_3$) δ: 5.27-5.45 (8H, m, 4 x CH=CH), 3.3-3.8 (11 H, m, OCH and 5 x $OCH_2$), 2.78 (4H, t, 2 x C=C-$CH_2$-C=C), 2.4-2.6 (6H, br. and m, 3 x $NCH_2$), 2.07 (8H, q, 4 x allylic $CH_2$), 1.49-1.63 (4H, m, 2 x $CH_2$), 1.2-1.5 (32H, m), 0.89 (6H, t, 2 x $CH_3$) ppm.

EXAMPLE 13

SYNTHESIS OF 1,2-DILINOLEYLTHIO-3-DIMETHYLAMINOPROPANE (DLIN-S-DMA)

DLin-S-DMA was synthesized as shown in the schematics and described below.

A.

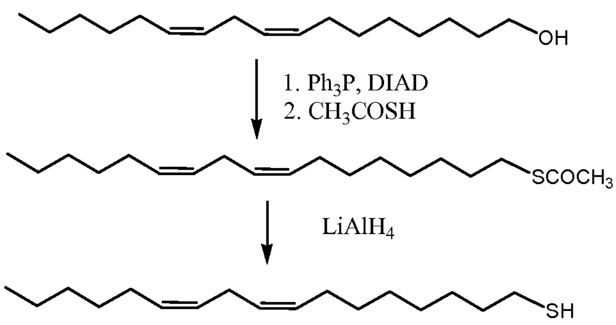


I

II

III

Synthesis of Linoleylthio Acetate (II)

To a solution of triphenylphosphine (18.0g, 68.2 mmol) in 250 mL of anhydrous THF under nitrogen at 0-5°C was added dropwise diisopropyl azodicarboxylate (DIAD, 14.7 mL, 68 mmol). Upon addition, the resulting mixture was stirred at 0-5°C for 45 min. A yellow suspension was resulted. A solution of linoleyl alcohol (I, 9.1g, 34 mmol) and thiolacetic acid (5.1 mL, 68 mmol) was then added at 0-5°C dropwise over 30 min to the yellow suspension under nitrogen. The resulting mixture was stirred at 0-5°C for one hour and then let warm up to room temperature. After stirring at room temperature for 60 min, a brown solution was resulted. Evaporation of the solvent led to a brownish oily residual. The residual was re-dissolved in 600 mL of ether. The ether phase was washed with water (2 x 250 mL), brine (250 mL), and dried over anhydrous $Na_2SO_4$. The solvent was evaporated to afford 31 g of brown oil which partially solidified overnight. This crude mixture was treated with 100 mL of hexanes. The solid was filtered off and washed with hexanes (2 x 30 mL). The filtrate and washes were combined and solvent evaporated to give 13 g of brown oil as a crude product. The crude product was purified by column chromatography twice on silica gel (230-400 mesh, 600 mL) eluted with 0-3% ether gradient in hexanes. This gave 10.0 g (91%) of linoleylthio acetate (II) as yellowish oil. 1 H NMR (400 MHz, $CDCl_3$) $\delta$: 5.27-5.45 (4H, m, 2 x CH=CH), 2.87 (2H, t, $SCH_2$), 2.78 (2H, t, C=C-$CH_2$-C=C), 2.33 (3H, s, $COCH_3$), 2.06 (4H, q, 2 x allylic $CH_2$), 1.5-1.62 (2H, m, $CH_2$), 1.24-1.55 (16H, m), 0.90 (3H, t, $CH_3$) ppm.

Synthesis of Linoleyl Mercaptane (III)

To a suspension of $LiAlH_4$ (4.7g, 124 mmol) in 150 mL of anhydrous ether under nitrogen at 0-5°C was added dropwise a solution of with one crystal of iodine in 200 mL of anhydrous ether under nitrogen was added a solution of linoleylthio acetate (II, 10.0g, 30.8 mmol) in 100 mL of anhydrous ether. Upon addition, the suspension was allowed to warm up to room temperature and then stirred at room temperature for 4 hours. The resulting mixture was cooled to 0-5°C and 10 mL of NaCl saturated aqueous solution was added very slowly. After stirred at room temperature for 60 min, the suspension was filtered through a pad of diatomaceous earth. The solids were washed with ether (3 x 100 mL). The filtrate and washes were combined and solvent evaporated resulting in 7.2 g (83%) of linoleyl mercaptane (III) as colourless oil. 1 H NMR (400 MHz, $CDCl_3$) $\delta$: 5.27-5.5 (4H, m, 2 x CH=CH), 2.78 (2H, t, C=C-$CH_2$-C=C), 2.53 (2H, q, $SCH_2$), 2.06 (4H, q, 2 x allylic $CH_2$), 1.5-1.62 (2H, m, $CH_2$), 1.23-1.45 (16H, m), 0.90 (3H, t, $CH_3$) ppm.

B.

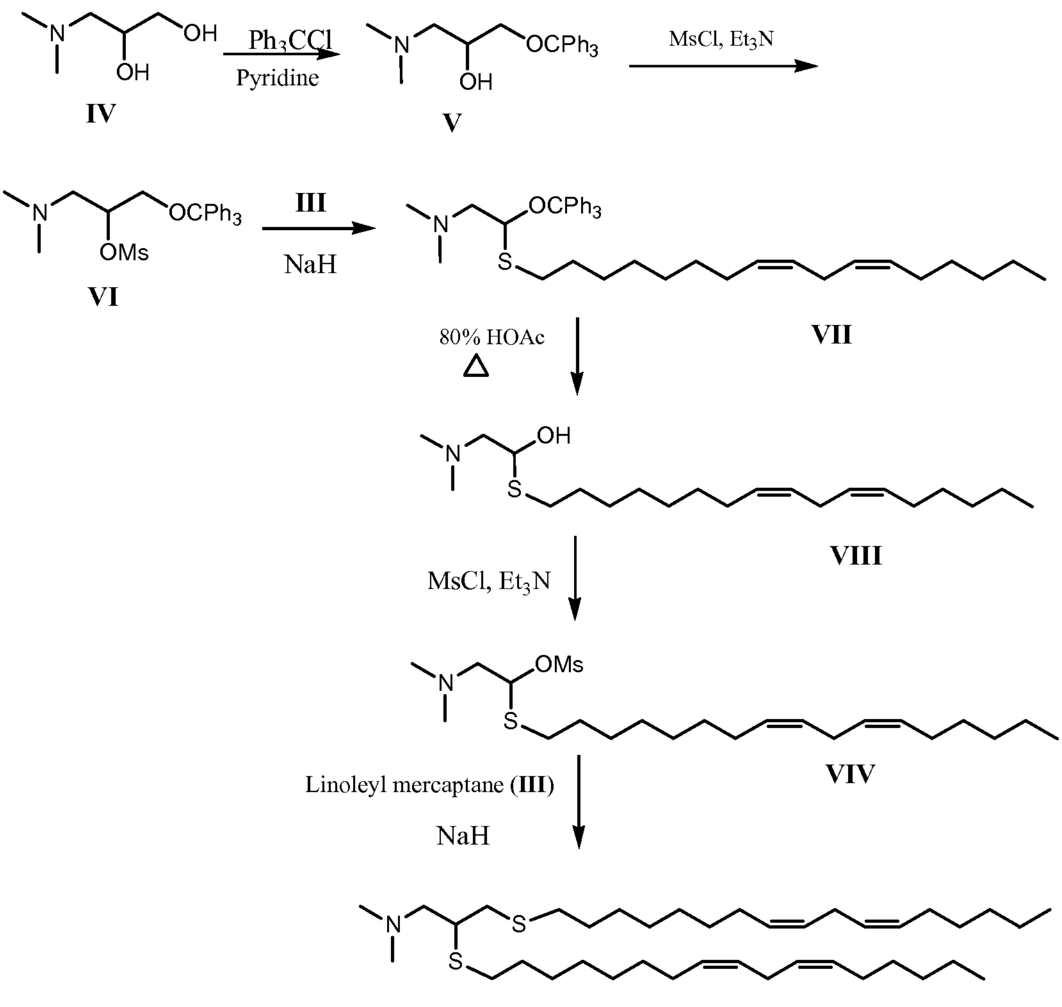


IV V VI VII VIII VIV

DLin-S-DMA

Synthesis of 1-Triphenylmethyloxy-2-hydroxy-3-dimethylaminopropane (V)

A mixture of 3-(dimethylamino)-1,2-propanediol (IV, 6.3g, 53 mmol) and triphenylmethyl chloride (15.5g, 55.6 mmol) in anhydrous pyridine (200 mL) was refluxed for 40 min. Upon cooling to room temperature, most of the solvent was removed in vacuo. To the resulting oily residual was added 400 mL of ethyl acetate. A large amount of solid was formed. The solid was filtered off and dried in air. The filtrate phase was washed with water (2 x 150 mL), brine (150 mL) and dried over anhydrous $Na_2SO_4$. Evaporation of the solvent afforded 8.5 g of brown oil as a crude product. The crude product was purified by column chromatography on silica gel (230-400 mesh, 500 mL) eluted with 0-10% methanol gradient in chloroform. This gave 4.1 g (21%) of 1-triphenylmethyloxy-2-hydroxy-3-dimethylaminopropane (V) as yellowish solid.

Synthesis of 1-Triphenylmethyloxy-2-methylsulfonyloxy-3-dimethylaminopropane (VI)

To a solution of 1-triphenylmethyloxy-2-hydroxy-3-dimethylaminopropane (V, 4.2g, 11.7 mmol) and anhydrous triethylamine (2.5 mL, 17.9 mmol) in 150 mL of anhydrous dichloromethane under nitrogen was added dropwise with an ice-water cooling bath methylsulfonyl chloride (1.0 mL, 13 mmol). Upon addition, the cooling bath was removed and the mixture stirred at room temperature under nitrogen overnight (20 hours). The resulting mixture was diluted with 100 mL of dichloromethane. The organic phase was washed with water (2 x 100 mL), brine (100 mL), and dried over anhydrous $Na_2SO_4$. Evaporation of the solvent gave 4.3 g of yellowish oil as a crude product (VI). The crude product was used in the next step without further purification.

Synthesis of 1-Triphenylmethyloxy-2-linoleylthio-3-dimethylaminopropane (VII)

To a suspension of NaH (2.0 g, 95%, 79 mmol) in 100 mL of anhydrous benzene under nitrogen was added

dropwise a solution of linoleyl mercaptane (III, 3.1 g, 11 mmol) in 30 mL of anhydrous benzene. The resulting mixture was stirred at room temperature for 20 min. A solution of 1-triphenylmethyloxy-2-methylsulfonyloxy-3-dimethylaminopropane (VI, 4.5g, 10 mmol) in 30 mL of anhydrous benzene was then added dropwise. After stirred at room temperature for 15 min, the mixture was refluxed gently under nitrogen for 3 days. Upon cooling, 30 mL of 1:1 (V:V) ethanol-benzene was added slowly to the mixture. The organic phase was washed once with 1:2 ethanol-water (360 mL) and dried over anhydrous Na2SO4. Evaporation of the solvent gave 7.1 g of yellowish oil as a crude product (VII). The crude product was purified by column chromatography on silica gel (230-400 mesh, 250 mL) eluted with 0-5% methanol gradient in chloroform. This gave 5.5 g (88%) of 1-triphenylmethyloxy-2-linoleylthio-3-dimethylaminopropane (VII) as yellowish oil.

Synthesis of 1-Hydroxy-2-linoleylthio-3-dimethylaminopropane (VIII)

1-Triphenylmethyloxy-2-linoleylthio-3-dimethylaminopropane (VII, 5.5g, 8.8 mmol) was refluxed in 150 mL of 80% HOAc under nitrogen for 7 hours. Upon cooling, the solvent was removed to give a pale semi-solid. The material was re-dissolved in 200 mL of ethyl acetate. The organic phase was washed subsequently with 0.5% NaOH aqueous solution (100 mL), water (100 mL), and brine (100 mL). After drying over anhydrous $Na_2SO_4$, the solvent was evaporated. 5.1 g of a pale solid was resulted. Column chromatography of the crude product on silica gel (230-400 mesh, 250 mL) eluted with 0-7% methanol gradient in chloroform afforded 1.3 g (39%) of 1-hydroxy-2-linoleylthio-3-dimethylaminopropane (VIII). 1 H NMR (400 MHz, $CDCl_3$) $\delta$: 5.27-5.53 (4H, m, 2 x CH=CH), 3.81 (1 H, dd, OCH), 3.43 (1 H, dd, OCH), 3.0-3.38 (1 H, br.), 2.88 (1 H, m, NCH), 2.7-2.82 (3H, m, C=C-$CH_2$-C=C and NCH), 2.52 (2H, t, $SCH_2$), 2.41 (6H, s, 2 x $NCH_3$), 2.06 (4H, q, 2 x allylic $CH_2$), 1.52-1.65 (2H, m, $CH_2$), 1.23-1.45 (16H, m), 0.90 (3H, t, $CH_3$) ppm.

Synthesis of 1-Methylulfonyloxyxy-2-linoleylthio-3-dimethylaminopropane (VIV)

To a solution of 1-hydroxy-2-linoleylthio-3-dimethylaminopropane (VIII, 1.3g, 3.2 mmol) and anhydrous triethylamine (0.7 mL, 5 mmol) in 50 mL of anhydrous dichloromethane under nitrogen was added dropwise methylsulfonyl chloride (0.5g, 4.3 mmol). The resulting mixture was stirred at room temperature overnight (19 hours). The reaction mixture was diluted with 50 mL of dichloromethane. The organic phase was washed water (2 x 50 mL), brine (50 mL), and dried over anhydrous $Na_2SO_4$. Evaporation of the solvent resulted in 1.4 g of yellowish oil as a crude product. The crude product was used in the following step without further purification.

Synthesis of 1,2-Dilinoleylthio-3-dimethylaminopropane (DLin-S-DMA)

NaH (0.89g, 60%, 22 mmol) was washed twice with hexanes (2 x 15 mL) under nitrogen and then suspended in 70 mL of anhydrous benzene. To the suspension was added dropwise a solution of linoleyl mercaptane (III, 1.1 g, 3.9 mmol) in 15 mL of anhydrous benzene. The resulting mixture was stirred at room temperature for 20 min. A solution of 1- methylsulfonyloxy-2-linoleylthio-3-dimethylaminopropane (VIV, 1.4g, 3.0 mmol) in 15 mL of anhydrous benzene was then added dropwise. After stirred at room temperature for 20 min, the mixture was refluxed gently under nitrogen for 2 days. Upon cooling, 200 mL of 1:1 (V:V) ethanol-benzene was added slowly to the mixture. The organic phase was washed with water (200 mL) and dried over anhydrous Na2SO4. Evaporation of the solvent gave 2.5 g of yellowish oil as a crude product. The crude product was purified by repeated column chromatography on silica gel (230-400 mesh, 250 mL) eluted with 0-3% methanol gradient in chloroform. This afforded 0.4 g (20%) 1,2-dilinoleylthio-3-dimethylaminopropane (DLin-S-DMA) as yellowish oil. 1 H NMR (400 MHz, $CDCl_3$) $\delta$: 5.27-5.48 (8H, m, 4 x CH=CH), 2.88-3.0 (1 H, m), 2.83 (2H, d, $CH_2$), 2.7 (4H, t, 2 x C=C-$CH_2$-C=C), 2.63-2.73 (1 H, m), 2.58 (4H, double triplet, 2 x $SCH_2$), 2.39-2.49 (1 H, m), 2.31 (6H, s, 2 x $NCH_3$), 2.06 (8H, q, 4 x allylic $CH_2$), 1.52-1.65 (4H, m, 2 x $CH_2$), 1.23-1.45 (32H, m), 0.90 (6H, t, 2 x $CH_3$) ppm.

EXAMPLE 14

SYNTHESIS OF 1,2-DILINOLEOYL-3-TRIMETHYLAMINOPROPANE CHLORIDE (DLIN-TAP.CL)

DLin-TAP.Cl was synthesized as shown in the schematic diagram and described below.

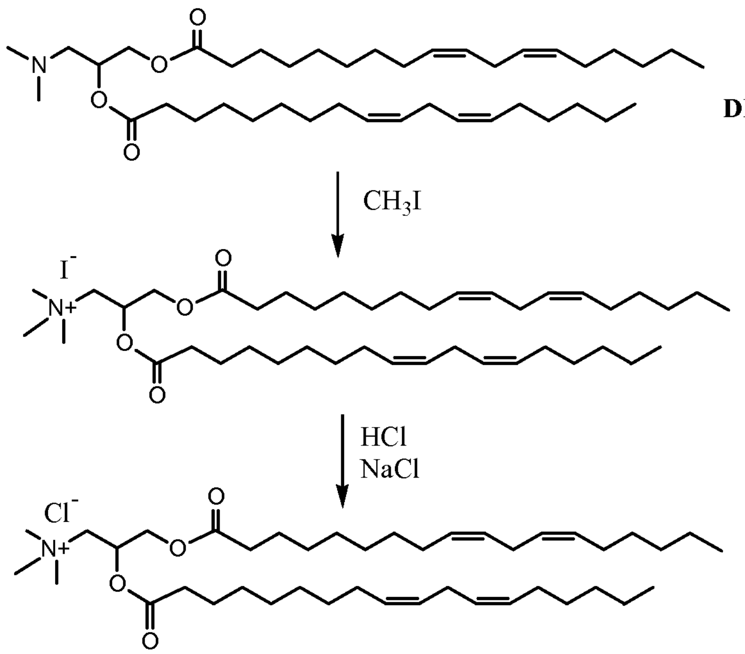


DLin-DAP

DLin-TAP.I

DLin-TAP.Cl

Synthesis of 1,2-Dilinoleoyl-3-dimethylaminopropane (DLin-DAP)

DLin-DAP was prepared according to procedures described in Example 10, based on estherification of 3-dimethylamino-1,2-propanediol by linoleoyl chloride.

Synthesis of 1,2-Dilinoleoyl-3-trimethylaminopropane Iodide (DLin-TAP.I)

A mixture of 1,2-dilinoleoyl-3-dimethylaminopropane (DLin-DAP, 5.5g, 8.8 mmol) and CH3I (7.5 mL, 120 mmol) in 20 mL of anhydrous $CH_2Cl_2$ was stirred under nitrogen at room temperature for 10 days. Evaporation of the solvent and excess of iodomethane afforded 6.4 g of yellow syrup as a crude DLin-TAP.I which was used in the following step without further purification.

Preparation of 1,2-Dilinoleoyl-3-trimethylaminopropane Chloride (DLin-TAP.Cl)

The above 1,2-dilinoleoyl-3-trimethylaminopropane iodide (DLin-TAP.I, 6.4 g) was dissolved in 150 mL of $CH_2Cl_2$ in a separatory funnel. 35 mL of 1 N HCl methanol solution was added, and the resulting solution was shaken well. To the solution was added 50 mL of brine and the mixture was shaken well. The organic phase was separated. The aqueous phase was extracted with 15 mL of $CH_2Cl_2$. The organic phase and extract were then combined. This completed the first step of ion exchange. The ion exchange step was repeated four more times. The final organic phase was washed with brine (75 mL) and dried over anhydrous Na2SO4. Evaporation of the solvent gave brownish oil. The crude product was purified by column chromatography on silica gel (230-400 mesh, 250 mL) eluted with 0-25% methanol gradient in chloroform. This afforded 2.2 g of 1,2-dilinoleoyl-3-trimethylaminopropane chloride (DLin-TAP.Cl) as white wax. 1 H NMR (400 MHz, $CDCl_3$) $\delta$: 5.61 (1 H, br. OCH), 5.25-5.45 (8H, m, 4 x CH=CH), 4.4-4.7 (2H, m, NCH2), 4.11 (1 H, dd, OCH), 3.80 (1 H, dd, OCH), 3.51 (9H, s, 3 x $NCH_3$), 2.77 (4H, t, 2 x C=C-$CH_2$-C=C), 2.2-2.5 (4H, m, 2 x $COCH_2$), 2.04 (8H, q, 4 x allylic $CH_2$), 1.75-2.0 (2H, br.), 1.49-1.75 (4H, m, 2 x $CH_2$), 1.2-1.45 (28H, m), 0.89 (6H, t, 2 x $CH_3$) ppm.

EXAMPLE 15

Synthesis OF 2,3-DIMYRISTOLEOLOXYL-1-N,N-DIMETHYLAMINOPROPANE (DMDAP)

DMDAP was synthesized as shown in the schematic and described below.

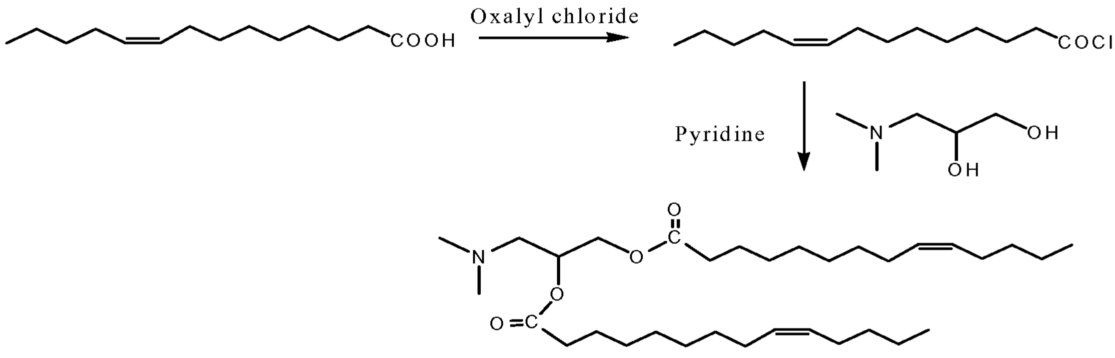


Synthesis of 2,3-Dimyristoleoloxyl-1-N,N-dimethylaminopropane (DMDAP)

To a solution of myristoleic acid (5.1 g, 22.5 mmol) in anhydrous benzene (60 mL) was added dropwise oxalyl chloride (3.93 g, 30.9 mmol) under argon. The resulting mixture was stirred at room temperature for 2 hours. Solvent and excess of oxalyl chloride was removed in vacuo and the residual was dissolved in anhydrous benzene (75 mL). To the resulting solution was added dropwise a solution of 3-(dimethylamino)-1,2-propanediol (1.28 g, 10.7 mmol) and dry pyridine (1.3 mL) in 10 mL of anhydrous benzene. The mixture was then stirred at room temperature under argon for 3 days and a suspension was resulted. The solid was filtered and washed with benzene. The wash was combined with the filtrate. The combine organic phase was diluted with benzene to about 250 mL and then washed with water (100 mL), dilute NaOH aqueous solution (ca. 0.01 %) and brine (2 x 100 mL). The aqueous phase in each of the washes was back-extracted with benzene. Finally, the organic phase was dried over anhydrous $Na_2SO_4$. The solvent was removed in vacuo affording 6.5 g of colourless oil. The crude product was purified by column chromatography on silica gel (230-400 mesh, 300 mL) eluted with 0-30% ethyl acetate gradient in hexanes. This gave 3.4g (59% yield) of DMDAP. 1 H NMR (400 MHz, $CDCl_3$) δ: 5.29-5. (4H, m, CH=CH), 5.18-5.26 (1 H, m, OCH), 4.37 (1 H, dd, J = 11.6 and 3.2 Hz, OCH), 4.09 (1 H, dd, J = 11.6 and 6.0 Hz, OCH), 2.52 (2H, m, $NCH_2$), 2.35-2.27 (4H, m, 2 x $COCH_2$), 2.30 (6H, s, 2 x $NCH_3$), 2.02 (8H, m, allylic 4 x $CH_2$), 1.62 (4H, m, 2 x $CH_2$), 1.30 (24H, m, 12 x $CH_2$), 0.90 (6H, t, 2 x $CH_3$) ppm.

EXAMPLE 16

SYNTHESIS OF 1,2-DIOLEYLCARBAMOYLOXY-3-DIMETHYLAMINOPROPANE (DO-C-DAP)

DO-C-DAP was synthesized as shown in the schematic and described below.

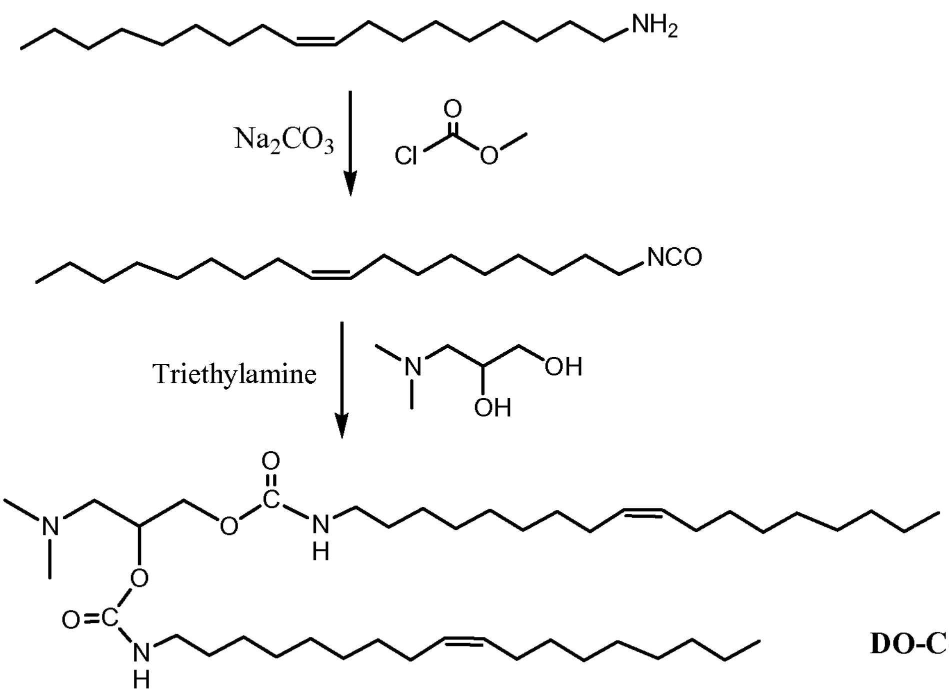


DO-C-DAP

Preparation of Oleyl Isocyanate

Anhydrous sodium carbonate (5 g, 47 mmol) was suspended in a solution of oleylamine (3.83 g, 14.3 mmol) in anhydrous $CH_2Cl_2$ (100 mL) under good stirring and nitrogen. The suspension was cooled to 0-5°C with an ice bath. To the suspension was added diphosgene (3.86 g, 19.5 mmol) in 5 mL of anhydrous $CH_2Cl_2$ under vigorous stirring. Upon addition, the resulting suspension was stirred at 0-5°C under nitrogen for 60 min and then at room temperature for 2 hours. Upon completion of the reaction, the organic phase was washed first with water (6 x 100 mL) until pH of the aqueous phase was about 6 and then with brine (100 mL). After drying with anhydrous $Na_2SO_4$, the solvent was evaporated to give 4.4 g of slightly brownish oil as a crude product. The crude product was used in the following step without further purification.

Condensation of Oleyl Isocyanate with 3-(Dimethylamino)-1,2-propanediol

To a solution of the above crude oleyl isocyanate (4.4 g, ca. 15 mmol) in 60 mL of anhydrous benzene under nitrogen was added dropwise a solution of 3-(dimethylamino)-1,2-propanediol (0.59 g, 5 mmol) in 10 mL of anhydrous benzene. The resulting mixture was stirred at room temperature for 90 min and then refluxed for 4 hours followed by stirring at room temperature overnight. Upon dilution of the mixture with 100 mL benzene, the organic phase was washed with water (4 x 75 mL), brine (75 mL), and dried over anhydrous $Na_2SO_4$. Evaporation of the solvent gave 5.0 g of yellow oil. Column purification of the oily material (400 mL silica gel, 230-400 mesh, eluted with 0-4% methanol gradient in chloroform) afforded 1.4 g (39%) of yellowish oil as the product DO-C-DAP. 1 H NMR (400 MHz, $CDCl_3$) $\delta$: 5.35 (4H, m, 2 x CH=CH), 5.04 (1 H, br. CONH), 4.90 (1 H, br. CONH), 4.80 (1 H, m, OCH), 4.28 (1 H, br. d, J = 12 Hz, OCH), 4.16 (1 H, dd, J = 12 and 6 Hz, OCH), 3.17 (4H, m, 2 x $NCH_2$), 2.38-2.65 (2H, m, $NCH_2$), 2.31 (6H, s, 2 x $NCH_3$), 2.02 (8H, m, allylic 4 x $CH_2$), 1.4-1.55 (4H, m, 2 x $CH_2$), 1.28 (44H, s, 22 x $CH_2$), 0.88 (6H, t, 2 x $CH_3$) ppm.

EXAMPLE 17

SYNTHESIS OF 1-DILINOLEYLMETHYLOXY-3-DIMETHYLAMINOPROPANE (DLIN-M-DMA)

DLin-M-DMA was synthesized as shown in the schematic and described below.

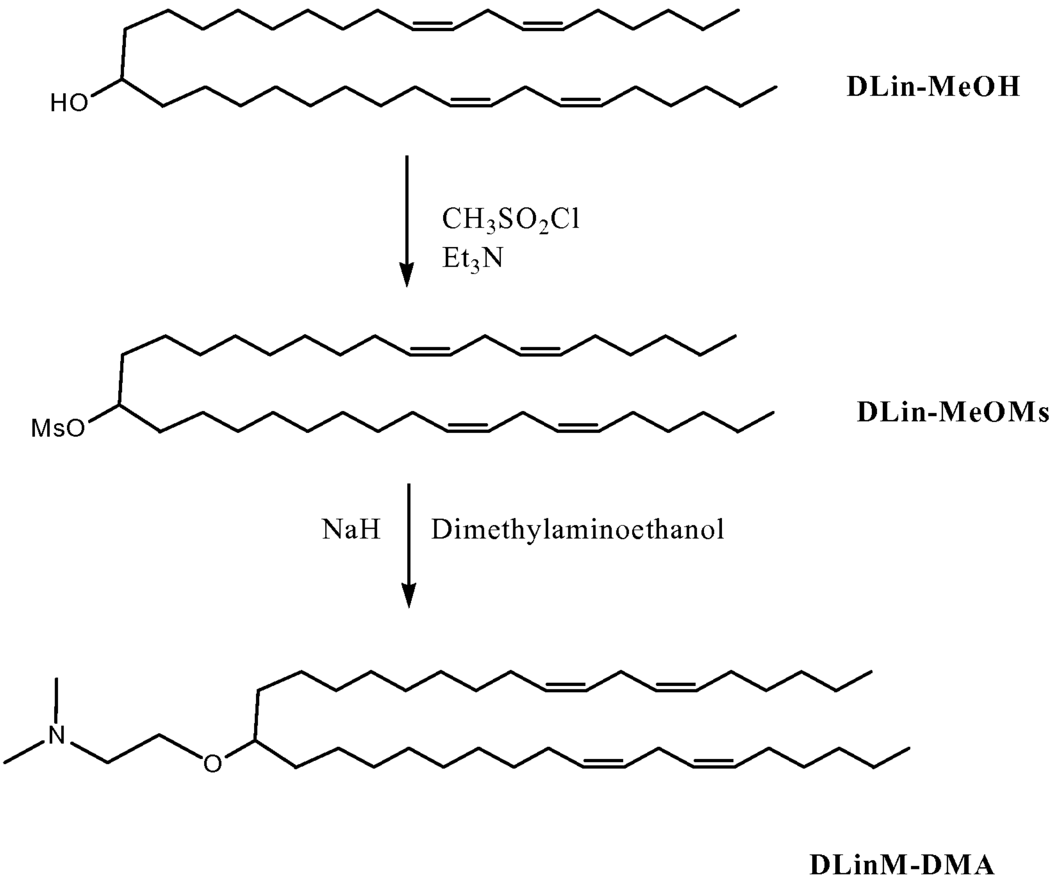


DLin-MeOH

DLin-MeOMs

DLinM-DMA

Synthesis of Dilinoleylmethanol (DLin-MeOH)

Dilinoleylmethanol (DLin-MeOH) was prepared as described in the above.

Synthesis of Dilinoleylmethyl Methane Sulfonate (DLin-MeOMs)

To a solution of dilinoleylmethanol (DLin-MeOH, 1.0g, 1.9 mmol) and anhydrous triethylamine (0.4 mL, 2.9 mmol) in 100 mL of anhydrous dichloromethane under nitrogen was added dropwise methylsulfonyl chloride (0.20 mL, 2.6 mmol). The resulting mixture was stirred at room temperature overnight (21 hours). The reaction mixture was diluted with 50 mL of dichloromethane. The organic phase was washed water (50 mL), brine (75 mL), and dried over anhydrous Na2SO4, Evaporation of the solvent resulted in 1.26 g of yellowish oil as a crude product, DLin-MeOMs. The crude product was purified by column chromatography on silica gel (230-400 mesh, 100 mL) eluted with 0-7% ether gradient in hexanes. This afforded 1.18 g of dilinoleylmethyl methane sulfonate as pale oil. 1 H NMR (400 MHz, $CDCl_3$) $\delta$: 5.28-5.46 (8H, m, 4 x CH=CH), 4.71 (1 H, quintet, OCH), 3.00 (3H, s, $OSO_2CH_3$), 2.78 (4H, t, 2 x C=C-$CH_2$-C=C), 2.06 (8H, q, 4 x allylic $CH_2$), 1.6-1.78 (4H, m, 2 x $CH_2$), 1.23-1.45 (36H, m), 0.90 (6H, t, 2 x $CH_3$) ppm.

Synthesis of Dilinoleylmethyloxy-3-dimethylaminopropane (DLin-M-DMA)

NaH (0.50g, 60%, 12.5 mmol) was washed twice with hexanes (2 x 15 mL) under nitrogen and then suspended in 75 mL of anhydrous benzene. To the NaH suspension was added dropwise a solution of dimethylaminoethanol (0.17g, 1.9 mmol) in 5 mL of anhydrous benzene. The resulting mixture was stirred at room temperature for 30 min. A solution of dilinoleylmethyl methane sulfonate (DLin-MeOMs, 1.15g, 1.9 mmol) in 20 mL of anhydrous benzene was then added dropwise. The resulting mixture was stirred under nitrogen at room temperature for 20 min and then refluxed overnight. Upon cooling, 50 mL of ethanol was added slowly to the mixture. The organic phase was washed with water (100 mL), and dried over anhydrous $Na_2SO_4$. Evaporation of the solvent gave 1.06 g of yellowish oil as a crude product. The crude product was purified by column chromatography on silica gel (230-400 mesh, 100 mL) eluted with 0-5% methanol gradient in dichloromethane. This afforded 60 mg (5%) dilinoleylmethyloxy-3-dimethylaminopropane (DLin-M-DMA) as pale oil. 1 H NMR (400 MHz, $CDCl_3$) $\delta$: 5.27-5.46 (8H, m, 4 x CH=CH), 3.73 (2H, t, $OCH_2$), 3.26 (1 H, quintet, OCH), 2.90 (2H, s, br., $NCH_2$), 2.78 (4H, t, 2 x C=C-$CH_2$-C=C), 2.60 (6H, s, 2 x $NCH_3$), 2.06 (8H, q, 4 x allylic $CH_2$), 1.1-1.6 (36H, m), 0.90 (6H, t, 2 x $CH_3$) ppm.

EXAMPLE 18

SYNHESIS OF CHIRAL FORMS OF 2,2-DILINOLEYL-4-DIMETHYLAMINOMETHYL-[1,3]-DIOXOLANE (DLIN-K-DMA)

(R)- and (S)-DLin-K-DMA was synthesized as describd below and depicted in the following diagram.

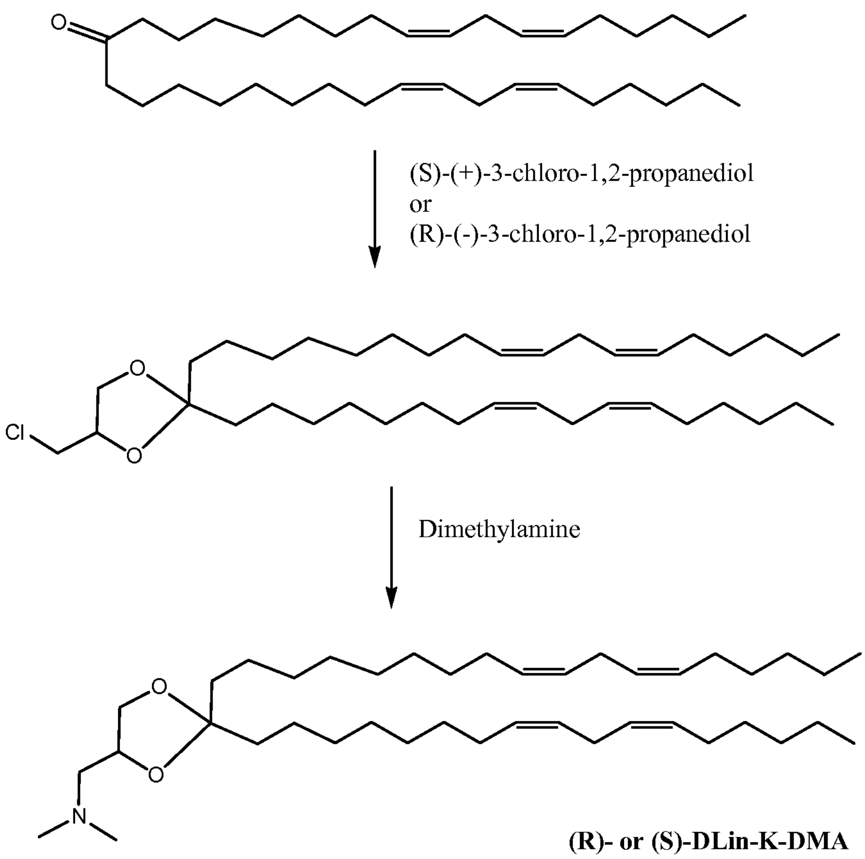


**(R)- or (S)-DLin-K-DMA**

Synthesis of Linoleyl bromide

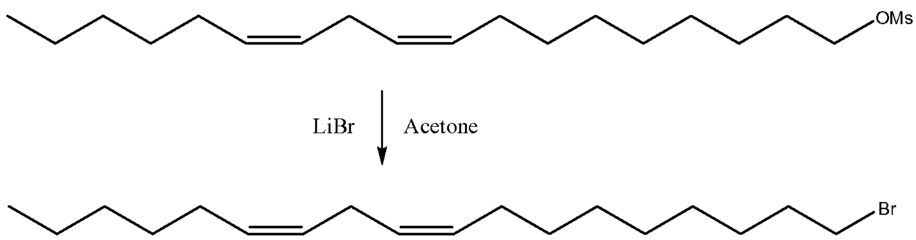


Linoleyl mesylate (100g, 0.29 mol) was added portion-wise to a stirred mixture of lithium bromide (113.4g, 1.306 mol) in acetone (1250mL) at room temperature. The reaction mixture was continued at room temperature for 16 hours. The solids were filtered under reduced pressure and washed with acetone. The filtrate was evaporated *in vacuo* and the resulting yellow liquid was purified by flash chromatography eluting with hexanes to give linoleyl bromide (90g, 95%) as colorless liquid.

Synthesis of Dilinoleyl methanol

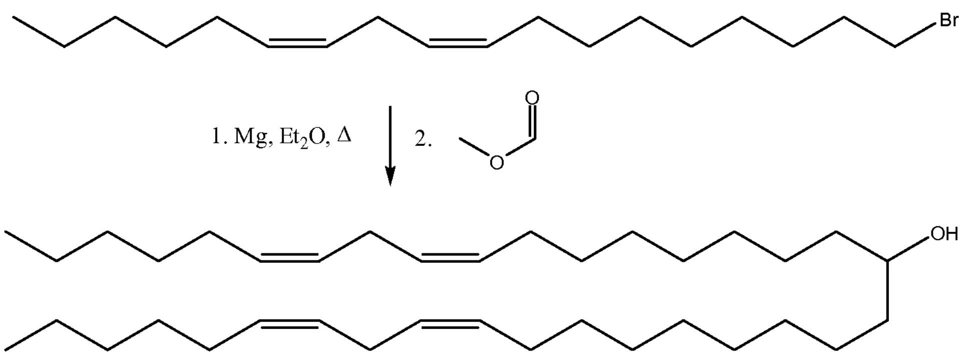


A solution of linoleyl bromide (78g, 0.237 mol) in anhydrous ether (500mL) was added drop-wise to a stirred suspension of magnesium turnings (6.9g, 0.284 mol) with a crystal of iodine in anhydrous ether (1000mL) at room temperature under a nitrogen atmosphere. The resulting mixture was refluxed for 10 hours and then cooled to room temperature. Methyl formate (14.5g, 0.241 mol) was added drop-wise to the grey mixture and the reaction continued overnight. Sulfuric acid (5%, 1000mL) was added carefully to the reaction mixture. The ethereal phase was separated and the aqueous layer was washed with diethyl ether. The combined organic phase was washed with water and brine, dried with sodium sulfate, and concentrated under reduced pressure. The resulting oil was purified by flash chromatography eluting with 0-5% ether in hexanes to afford dilinoleyl methyl formate (42g).

A mixture of dilinoleyl methyl formate (42g) and potassium hydroxide (9g) was stirred in 85% ethanol (250mL) at room temperature for 2 hours. The solvent was removed *in vacuo* and the aqueous residue was neutralized with 2M hydrochloric acid. The aqueous residue was extracted with ether. The combined organic layer was dried with sodium sulfate, filtered and concentrated under reduced pressure to give dilinoleyl methanol (38g) as pale yellow oil.

Synthesis of Dilinoleyl ketone

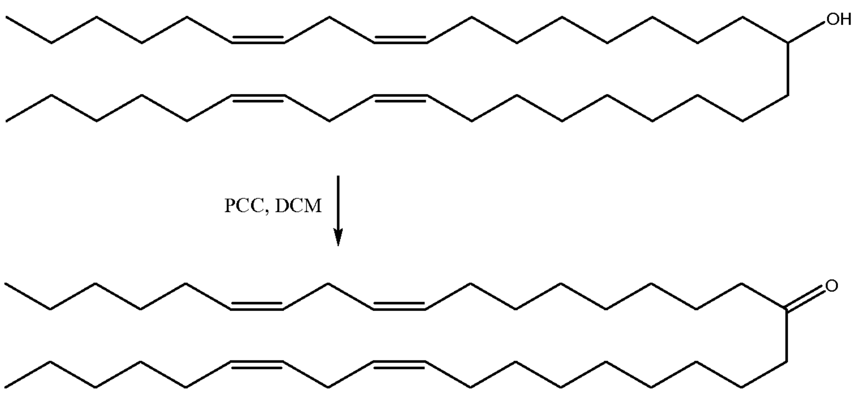


Pyridinium chlorochromate (46.3g, 0.2155mol) was added portion-wise to a stirred mixture of dilinoleyl methanol (38g, 0.0718 mol) in dichloromethane (750 mL) at room temperature for 2 hours. Ether was added to quench the reaction. The resulting brown mixture was filtered through Florisil eluting with ether. The solvent was removed under reduced pressure to afford dilinoleyl ketone (36g) as pale yellow oil.

Synthesis of 2,2-Dilinoleyl-4-chloromethyl-[1,3]-dioxolane

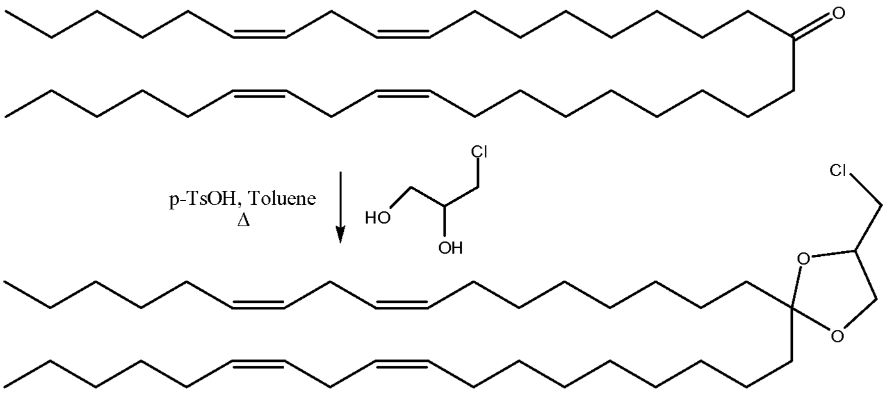


(S)-2,2-Dilinoleyl-4-chloromethyl-[1,3]-dioxolane

A mixture of dilinoleyl ketone (7g), (S)-(+)-3-chloro-1,2-propanediol (5g), p-toluenesulfonic acid (0.05g), and toluene (200mL) was heated to reflux for 20 hours using a Dean-Stark apparatus. The reaction mixture was cooled to room temperature and washed with sat. sodium bicarbonate and brine. The solvent was removed under *in vacuo* and the residue was purified by flash chromatography eluting with 2% ethyl acetate in hexanes. The product was isolated as pale yellow oil (7g).

(R)-2,2-Dilinoleyl-4-chloromethyl-[1,3]-dioxolane

A mixture of dilinoleyl ketone (8g), (R)-(-)-3-chloro-1,2-propanediol (5g), p-toluenesulfonic acid (0.05g), and toluene (200mL) was heated to reflux for 20 hours using a Dean-Stark apparatus. The reaction mixture was cooled to room temperature and washed with sat. sodium bicarbonate and brine. The solvent was removed under reduced pressure and the residue was purified by flash chromatography eluting with 2% ethyl acetate in hexanes. The product was isolated as pale yellow oil (8g)

Synthesis of Chiral DLin-K-DMA

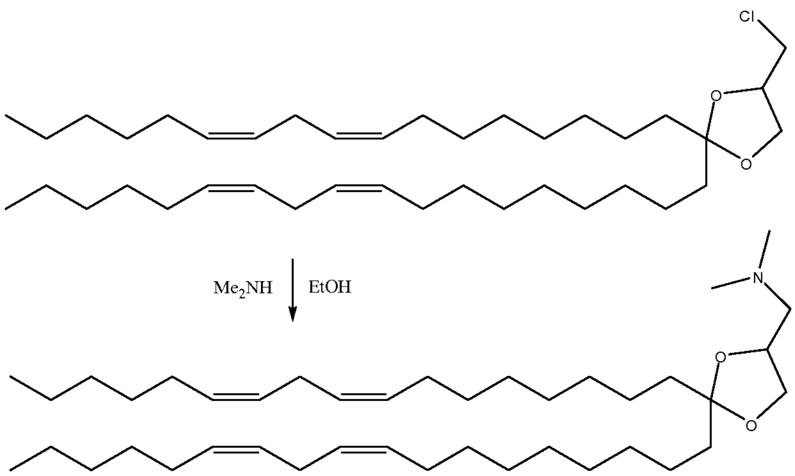


Synthesis of (R)- DLin-K-DMA

A solution of the above (S)-ketal (7g) and dimethylamine (33% in EtOH, 500mL) in THF (50mL) was heated at 90°C under 30psi of pressure for 1 week. The solution was removed under reduced pressure and the residue was purified by flash chromatography eluting with 3-75% ethyl acetate in hexanes. (R)-DLin-K-DMA was isolated as pale brown liquid (6g).

Synthesis of (S)- DLin-K-DMA

A solution of the above (R)-ketal (3.5g) and dimethylamine (33% in EtOH, 500mL) in THF (50mL) was heated at 85°C under 30psi of pressure for 1 week. The solution was removed *in vacuo* and the residue was purified by flash chromatography eluting with 3-75% ethyl acetate in hexanes. (S)- DLin-K-DMA was isolated as pale brown liquid (2g).

EXAMPLE 19

SYNTHESIS OF MPEG2000-1,2-DI-*O*-ALKYL-*SN*3-CARBOMOYLGLYCERIDE

The PEG-lipids, such as mPEG2000-1,2-Di-*O*-Alkyl-*sn*3-Carbomoylglyceride (PEG-C-DOMG) were synthesized as shown in the schematic and described below.

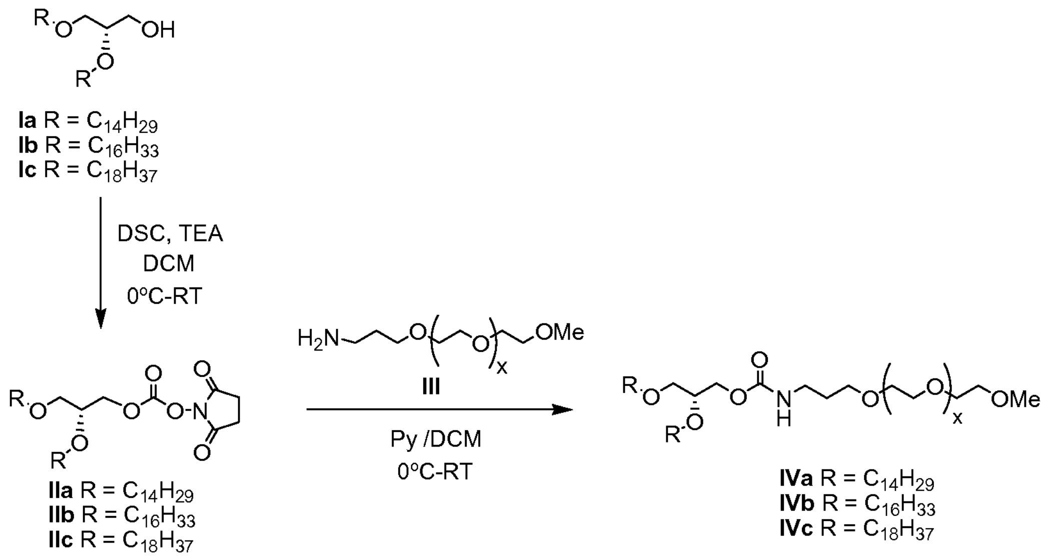


**Ia** R = $C_{14}H_{29}$
**Ib** R = $C_{16}H_{33}$
**Ic** R = $C_{18}H_{37}$

**IIa** R = $C_{14}H_{29}$
**IIb** R = $C_{16}H_{33}$
**IIc** R = $C_{18}H_{37}$

**IVa** R = $C_{14}H_{29}$
**IVb** R = $C_{16}H_{33}$
**IVc** R = $C_{18}H_{37}$

Synthesis of IVa

1,2-Di-*O*-tetradecyl-*sn*-glyceride **Ia** (30 g, 61.80 mmol) and *N,N'*-succinimidylcarboante (DSC, 23.76 g, 1.5eq) were taken in dichloromethane (DCM, 500 mL) and stirred over an ice water mixture. Triethylamine (TEA, 25.30 mL, 3 eq) was added to the stirring solution and subsequently the reaction mixture was allowed to stir overnight at ambient temperature. Progress of the reaction was monitored by TLC. The reaction mixture was diluted with DCM (400 mL) and the organic layer was washed with water (2X500 mL), aqueous $NaHCO_3$ solution (500 mL) followed by standard work-up. The residue obtained was dried at ambient temperature under high vacuum overnight. After drying, the crude carbonate **IIa** thus obtained was dissolved in dichloromethane (500 mL) and stirred over an ice

bath. To the stirring solution, $mPEG_{2000}$-$NH_2$ (**III**, 103.00 g, 47.20 mmol, purchased from NOF Corporation, Japan) and anhydrous pyridine (Py, 80 mL, excess) were added under argon. In some embodiments, the x in compound **III** has a value of 45-49, preferably 47-49, and more preferably 49. The reaction mixture was then allowed to stir at ambient temperature overnight. Solvents and volatiles were removed under vacuum and the residue was dissolved in DCM (200 mL) and charged on a column of silica gel packed in ethyl acetate. The column was initially eluted with ethyl acetate and subsequently with gradient of 5-10 % methanol in dichloromethane to afford the desired PEG-Lipid **IVa** as a white solid (105.30g, 83%). $^1$H NMR ($CDCl_3$, 400 MHz) $\delta$ = 5.20-5.12(m, 1 H), 4.18-4.01 (m, 2H), 3.80-3.70(m, 2H), 3.70-3.20(m, -O-$CH_2$-$CH_2$-O-, PEG-$CH_2$), 2.10-2.01 (m, 2H), 1.70-1.60 (m, 2H), 1.56-1.45(m, 4H), 1.31-1.15(m, 48H), 0.84(t, J= 6.5Hz, 6H). MS range found: 2660-2836.

Synthesis of IVb

1,2-Di-*O*-hexadecyl-*sn*-glyceride **Ib** (1.00 g, 1.848 mmol) and DSC (0.710 g, 1.5eq) were taken together in dichloromethane (20 mL) and cooled down to 0°C in an ice water mixture. Triethylamine (1.00 mL, 3eq) was added and the reaction was stirred overnight. The reaction was followed by TLC, diluted with DCM, washed with water (2 times), $NaHCO_3$ solution and dried over sodium sulfate. Solvents were removed under reduced pressure and the resulting residue of **IIb** was maintained under high vacuum overnight. This compound was directly used for the next reaction

without further purification. $MPEG_{2000}$-$NH_2$ **III** (1.50g, 0.687 mmol, purchased from NOF Corporation, Japan) and **IIb** (0.702g, 1.5eq) were dissolved in dichloromethane (20 mL) under argon. In some embodiments, the x in compound **III** has a value of 45-49, preferably 47-49, and more preferably 49. The reaction was cooled to 0°C. Pyridine (1 mL, excess) was added and the reaction stirred overnight. The reaction was monitored by TLC. Solvents and volatiles were removed under vacuum and the residue was purified by chromatography (first ethyl acetate followed by 5-10% MeOH/DCM as a gradient elution) to obtain the required compound **IVb** as a white solid (1.46 g, 76 %). $^1$H NMR ($CDCl_3$, 400 MHz) $\delta$ = 5.17(t, J= 5.5Hz, 1 H), 4.13(dd, J= 4.00Hz, 11.00 Hz, 1 H), 4.05(dd, J= 5.00Hz, 11.00 Hz, 1 H), 3.82-3.75(m, 2H), 3.70-3.20(m, -O-$CH_2$-$CH_2$-O-, PEG-$CH_2$), 2.05-1.90(m, 2H), 1.80-1.70 (m, 2H), 1.61-1.45(m, 6H), 1.35-1.17(m, 56H), 0.85(t, J= 6.5Hz, 6H). MS range found: 2716-2892.

Synthesis of IVc

1,2-Di-*O*-octadecyl-*sn*-glyceride **Ic** (4.00 g, 6.70 mmol) and DSC (2.58 g, 1.5eq) were taken together in dichloromethane (60 mL) and cooled down to 0°C in an ice water mixture. Triethylamine (2.75 mL, 3eq) was added and the reaction was stirred overnight. The reaction was followed by TLC, diluted with DCM, washed with water (2 times), $NaHCO_3$ solution, and dried over sodium sulfate. Solvents were removed under reduced pressure and the residue was maintained under high vacuum overnight. This compound was directly used for the next reaction without further purification. $MPEG_{2000}$-$NH_2$ **III** (1.50g, 0.687 mmol, purchased from NOF Corporation, Japan) and **IIc** (0.760g, 1.5eq) were dissolved in dichloromethane (20 mL) under argon. In some embodiments, the x in compound **III** has a value of 45-49, preferably 47-49, and more preferably 49. The reaction was cooled to 0°C. Pyridine (1 mL, excess) was added and the reaction was stirred overnight. The reaction was monitored by TLC. Solvents and volatiles were removed under vacuum and the residue was purified by chromatography (ethyl acetate followed by 5-10% MeOH/DCM as a gradient elution) to obtain the desired compound **IVc** as a white solid (0.92 g, 48 %). $^1$H NMR ($CDCl_3$, 400 MHz) $\delta$ = 5.22-5.15(m, 1 H), 4.16(dd, J= 4.00Hz, 11.00 Hz, 1 H), 4.06(dd, J= 5.00Hz, 11.00 Hz, 1 H), 3.81-3.75(m, 2H), 3.70-3.20(m, -O-$CH_2$-$CH_2$-O-, PEG-$CH_2$), 1.80-1.70 (m, 2H), 1.60-1.48(m, 4H), 1.31-1.15(m, 64H), 0.85(t, J= 6.5Hz, 6H). MS range found: 2774-2948.

EXAMPLE 20

PREPARATION AND CHARACTERIZATION OF NUCLEIC ACID-LIPID PARTICLES

Nucleic acid lipid particles containing a siRNA that targets Factor VII were prepared and characterized as described below.

Materials and Methods:

Lipids

Distearoylphosphatidylcholine (DSPC), sphingomyelin (SM), and palmitoyloleoylphosphatidylcholine (POPC) were purchased from Northern Lipids (Vancouver, Canada). 1,2-dioleoyloxy-3-dimethylammoniumpropane (DODAP) was purchased from Avanti Polar Lipids (Alabaster, AL). Cholesterol was purchased from Sigma Chemical Company (St. Louis, Missouri, USA) or Solvay Pharmaceuticals (Weesp, The Netherlands). PEG-C-DOMG was synthesized as described herein. The PEG-S-DMG and PEG-DMA were synthesized as described in Heyes et al. (2006) Synthesis and Charactierization of Novel Poly(ethylene glycol)-lipid Conjugates Suitable for Use in Drug Delivery, J. Controlled Release 112:280-290.

Buffers and Solvents

Ethanol (100%), methanol, chloroform, citric acid monohydrate, sodium citrate dehydrate, HEPES, NaCl and phosphate-buffered saline (PBS) were all purchased from commercial suppliers.

siRNA

siRNAs were chemically synthesized as described in John *et al*. (John et al., Nature advance online publication, 26 September 2007 (DOI:10.1038/nature06179). Sequences of siRNAs used in these studies were as follows:

si-FVII sense, 5'-GGAUCAUCUCAAGUCUUACTT-3' (SEQ ID NO:34);
si-FVII antisense, 5'-GUAAGACUUGAGAUGAUCCTT-3' (SEQ ID NO:35);
si-Luc sense, 5'-cuuAcGcuGAGuAcuucGATT-3' (SEQ ID NO:36);

si-Luc antisense, 5'-UCGAAGuACUcAGCGuAAGTT-3' (SEQ ID NO:37);
Lower-case letters denote 2'-*O*-Me-modified nucleotides; bold letters denote 2'-*F*-modified nucleotides. All siRNAs contained phosphorothioate linkages between the two thymidines (T) at the 3' end of each strand.

Preparation of Liposomal siRNA Formulations

Liposomal siRNA formulations comprising various cationic lipids in combination with DSPC, cholesterol and PEG-C-DOMG at an approximate ratio (mol%) of 40% cationic lipid:10% DSPC:40% cholesterol:10% PEG-C-DOMG were prepared as described in Maurer et al. (Biophys J., 2001), with modifications. Stock solutions of each lipid were prepared in absolute ethanol. Alternatively, lipids were weighed on an analytical balance, mixed in the desired ratio in an RNase-free container, and absolute ethanol was added to dissolve the lipids. In some instances, warming (e.g., 50°C) was required to completely dissolve the lipids or lipid mixtures. Once the lipids were dissolved in ethanol, the appropriate volume of lipids was added, with mixing, to 50 mM citrate, pH4.0 to form liposomes with a lipid concentration of 8-10 mM and a final ethanol concentration of 30-40% by volume (typically 34%).

These pre-formed vesicles (PFV) were extruded 3 times through two stacked 80 nm filters as described previously (Hope *et al.*, 1986). In some instances, depending on the lipid composition, warming was required to extrude the liposomes. The mean particle size of the PFVs was determined by QELS and was generally 50-120 nm (more typically, 70-80 nm), depending on the lipid composition and formulation conditions used.

Stock solutions of siRNA were dissolved in 10 mM citrate, 30 mM NaCl, pH 6.0 and stored at 4°C until use. Immediately prior to formulation, an aliquot of the siRNA stock solution was added to a mixture of ethanol and 50 mM citrate, pH 4.0 to achieve a final ethanol concentration that was equivalent to that used in the specific PFV composition, typically 34% ethanol by volume.

After preparing the siRNA, both the siRNA and PFV were equilibrated for 10 minutes at the desired incubation temperature (25-45°C, depending on the lipid composition used) prior to mixing. The siRNA was then added quickly, with continual mixing, to the PFVs and the resulting mixture was incubated for 30 minutes at the selected temperature (mixing continually). At the completion of the incubation, the sample was typically diluted 2-3 fold in 50 mM citrate or PBS (or HBS), pH 7.4, concentrated to its original volume by tangential flow diafiltration and then washed with 10-15 volumes of PBS (or HBS), pH 7.4 to remove residual ethanol and exchange the external buffer. In some instances, generally involving small formulation volumes, the incubation mixtures were placed in pre-washed dialysis tubing (100K MWt cutoff) and the samples were dialyzed overnight against PBS (or HBS), pH 7.4. After completion of the buffer exchange and ethanol removal, samples were concentrated to the desired siRNA concentration by tangential flow dialfiltration.

Particle Size Analysis

The size distribution of liposomal siRNA formulations was determined using a NICOMP Model 380 Sub-micron particle sizer (PSS NICOMP, Particle Sizing Systems, Santa Barbara, CA). Mean particle diameters were generally in the range 50-120 nm, depending on the lipid composition used. Liposomal siRNA formulations were generally homogeneous and had standard deviations (from the mean particle size) of 20-50 nm, depending on the lipid composition and formulation conditions used.

Ion Exchange Chromatography to Determine Non-encapsulated (Free) siRNA

Anion exchange chromatography, using either DEAE Sepharose columns or commercial centrifugation devices (Vivapure D Mini columns, catalogue number VS-IX01 DH24), was used to measure the amount of free siRNA in the liposome formulations. For the DEAE Sepharose columns, siRNA-containing formulations were eluted through columns (~2.5 cm bed height, 1.5 cm diameter) equilibrated with HBS (145 mM NaCl, 20 mM HEPES, pH 7.5). Aliquots of the initial and eluted samples were assayed for lipid and siRNA content by HPLC and A260, respectively. The percent encapsulation was calculated based on the relative siRNA-to-lipid ratios of the pre and post column samples.

For the Vivapure centrifugal devices, an aliquot (0.4 mL, <1.5 mg/mL siRNA) of the siRNA-containing formulation was eluted through the positively charged membrane by centrifugation (2000 xg for 5 min). Aliquots of the pre and post column samples were analyzed as described above to determine the amount of free siRNA in the sample.

Determination of siRNA Concentration

siRNA concentration was determined by measuring the absorbance at 260 nm after solubilization of the lipid. The lipid was solubilized according to the procedure outlined by Bligh and Dyer (Bligh, et al., Can. J. Biochem. Physiol. 37:911-917 (1959). Briefly, samples of liposomal siRNA formulations were mixed with chloroform/methanol at a volume

ratio of 1:2.1:1 (aqueous sample:methanol:chloroform). If the solution was not completely clear (*i.e.,* a single, clear phase) after mixing, an additional 50-100 mL (volume recorded) of methanol was added and the sample was remixed. Once a clear monophase was obtained, the sample was assayed at 260 nm using a spectrophotometer. siRNA concentration was determined from the A260 readings using a conversion factor of approximately 45 μg/mL = 1.0 OD, using a 1.0 cm path length. The conversion factor in the chloroform/methanol/water monophase varies (35-50 μg/mL = 1.0 OD) for each lipid composition and is determined empirically for each novel lipid formulation using a known amount of siRNA.

Determination of Lipid Concentrations and Ratios

Cholesterol, DSPC, PEG-lipid (e.g., PEG-S-DMG) and cationic lipid (e.g., DLin-K-DMA) were measured against reference standards using a Waters Alliance HPLC system consisting of an Alliance 2695 Separations Module (autosampler, HPLC pump, and column heater), a Waters 2424 Evaporative Light Scattering Detector (ELSD), and Waters Empower HPLC software (version 5.00.00.00, build number 1154; Waters Corporation, Milford, MA, USA). Samples (15 μL) containing 0.8 mg/mL total lipid in 90% ethanol were injected onto a reversed-phase XBridge C18 column with 2.5 μm packing, 2.1 mm x 50 mm (Waters Corporation, Milford, MA, USA) heated at 55°C and chromatographed with gradient elution at a constant flow rate of 0.5 mL/min. The mobile phase composition changed from 10 mM $NH_4HCO_3$:methanol (20:80) to THF:10 mM $NH_4HCO_3$:methanol (16:4:80) over 16 minutes. The gas pressure on the ELSD was set at 25 psi, while the nebulizer heater-cooler set point and drift tube temperature set point were set at 100% and 85°C respectively. Measured lipid concentrations (mg/mL) were converted to molar concentrations, and relative lipid ratios were expressed as mol% of the total lipid in the formulation.

Determination of Encapsulation Efficiency

Trapping efficiencies were determined after removal of external siRNA by tangential flow diafiltration or anion exchange chromatography. siRNA and lipid concentrations were determined (as described above) in the initial formulation incubation mixtures and after tangential flow diafiltration. The siRNA-to-lipid ratio (wt/wt) was determined at both points in the process, and the encapsulation efficiency was determined by taking the ratio of the final and initial siRNA-to-lipid ratio and multiplying the result by 100 to obtain a percentage.

The results of these studies are provided in Table 5.

EXAMPLE 21

REGULATION OF MAMMALIAN GENE EXPRESSION USING NUCLEIC ACID-LIPID PARTICLES

Factor VII (FVII), a prominent protein in the coagulation cascade, is synthesized in the liver (hepatocytes) and secreted into the plasma. FVII levels in plasma can be determined by a simple, plate-based colorimetric assay. As such, FVII represents a convenient model for determining siRNA-mediated downregulation of hepatocyte-derived proteins, as well as monitoring plasma concentrations and tissue distribution of the nucleic acid lipid particles and siRNA.

Factor VII Knockdown in Mice

FVII activity was evaluated in FVII siRNA-treated animals at 24 hours after intravenous (bolus) injection in C57BL/6 mice. FVII was measured using a commercially available kit (Biophen FVII Kit™; Aniara Corp., Mason, OH), following the manufacturer's instructions at a microplate scale. FVII reduction was determined against untreated control mice, and the results were expressed as % Residual FVII. Four dose levels (2, 5, 12.5, 25 mg/kg FVII siRNA) were used in the initial screen of each novel liposome composition, and this dosing was expanded in subsequent studies based on the results obtained in the initial screen.

Determination of Tolerability

The tolerability of each novel liposomal siRNA formulation was evaluated by monitoring weight change, cageside observations, clinical chemistry and, in some instances, hematology. Animal weights were recorded prior to treatment and at 24 hours after treatment. Data was recorded as % Change in Body Weight. In addition to body weight measurements, a full clinical chemistry panel, including liver function markers, was obtained at each dose level (2, 5, 12.5 and 25 mg/kg siRNA) at 24 hours post-injection using an aliquot of the serum collected for FVII analysis. Samples were sent to the Central Laboratory for Veterinarians (Langley, BC) for analysis. In some instances, additional mice were included in the treatment group to allow collection of whole blood for hematology analysis.

Determination of Therapeutic Index

Therapeutic index (TI) is an arbitrary parameter generated by comparing measures of toxicity and activity. For these studies, TI was determined as:

$$TI = MTD\ (\text{maximum tolerated dose}) / ED_{50}\ (\text{dose for 50\% FVII knockdown})$$

The MTD for these studies was set as the lowest dose causing >7% decrease in body weight and a >200-fold increase in alanine aminotransferase (ALT), a clinical chemistry marker with good specificity for liver damage in rodents. The $ED_{50}$ was determined from FVII dose-activity curves.

Determination of siRNA plasma levels

Plasma levels of Cy3 fluorescence were evaluated at 0.5 and 3 h post-IV injection in C57BL/6 mice using a fluorescently labeled siRNA (Cy-3 labeled luciferase siRNA). The measurements were done by first extracting the Cy3-siRNA from the protein-containing biological matrix and then analyzing the amount of Cy-3 label in the extract by fluorescence. Blood was collected in EDTA-containing Vacutainer tubes and centrifuged at 2500 rpm for 10 min at 2-8°C to isolate the plasma. The plasma was transferred to an Eppendorf tube and either assayed immediately or stored in a -30°C freezer. An aliquot of the plasma (100 μL maximum) was diluted to 500 μL with PBS (145 mM NaCl, 10 mM phosphate, pH 7.5), then methanol (1.05 mL) and chloroform (0.5 mL) were added, and the sample vortexed to obtain a clear, single phase solution. Additional water (0.5 mL) and chloroform (0.5 mL) were added and the resulting emulsion sustained by mixing periodically for a minimum of 3 minutes. The mixture was centrifuged at 3000 rpm for 20 minutes and the aqueous (top) phase containing the Cy-3-label was transferred to a new tube. The fluorescence of the solution was measured using an SLM Fluorimeter at an excitation wavelength of 550 nm (2 nm bandwidth) and emission wavelength of 600 nm (16 nm bandwidth). A standard curve was generated by spiking aliquots of plasma from untreated animals with the formulation containing Cy-3-siRNA (0 to 15 μg/mL) and the sample processed as indicated above. Data was expressed as Plasma Cy-3 concentration (μg/mL).

Determination of siRNA Biodistribution

Tissue (liver and spleen) levels of Cy3 fluorescence were evaluated at 0.5 and 3 h post-IV injection in C57BL/6 mice for each novel liposomal siRNA formulation. One portion of each tissue was analyzed for total fluorescence after a commercial phenol/chloroform (Trizol® reagent) extraction, while the other portion was evaluated by confocal microscopy to assess intracellular delivery. Upon collection, each tissue was weighed and divided into 2 pieces.

Sections (400 - 500 mg) of liver obtained from saline-perfused animals were accurately weighed into Fastprep tubes and homogenized in 1 mL of Trizol using a Fastprep FP120 instrument. An aliquot of the homogenate (typically equivalent to 50 mg of tissue) was transferred to an Eppendorf tube and additional Trizol was added to achieve 1 mL final volume. Chloroform (0.2 mL) was added and the solution was mixed and incubated for 2-3 min before being centrifuged for 15 min at 12,000xg. An aliquot (0.5 mL) of the aqueous (top) phase containing Cy3 was diluted with 0.5 mL of PBS and the fluorescence of the sample measured as described above.

Spleens from saline-perfused treated animals were homogenized in 1 mL of Trizol using Fastprep tubes. Chloroform (0.2 mL) was added to the homogenate, incubated for 2-3 min and centrifuged for 15 min at 12 000 ×g at 2-8°C. An aliquot of the top aqueous phase was diluted with 0.5 mL of PBS and the fluorescence of the sample was measured as described above. The data was expressed as the % of the Injected Dose (in each tissue) and Tissue Cy-3 Concentration (μg/mL).

In preparation for confocal microscopy, whole or portions of tissues recovered from saline-perfused animals were fixed in commercial 10% neutral-buffered formalin. Tissues were rinsed in PBS, pH 7.5 and dissected according to RENI Guide to Organ Trimming, available at (http://www.item.fraunhofer.de/reni/trimming/index.php). The specimens were placed cut side down in molds filled with HistoPrep (Fisher Scientific, Ottawa ON, SH75-125D) and frozen in 2-methylbutane that had been cooled in liquid Nitrogen until the equilibration point was reached. Next, the frozen blocks were fastened to the cryomicrotome (CM 1900; Leica Instruments, Germany) in the cryochamber (-18°C) and trimmed with a disposable stainless steel blade (Feather S35, Fisher Scientific, Ottawa ON), having a clearance angle of 2.5°. The sample was then cut at 10μm thickness and collected on to Superfrost/Plus slides (Fisher Scientific, Ottawa ON, 12-550-15) and dried at room temperature for 1 minute and stored at -20°C. Slides were rinsed 3 times in PBS to remove HistoPrep, mounted with Vectorshield hard set (Vector Laboratories, Inc. Burlingame CA, H-1400) and frozen pending

microscopy analysis. In some instances, TOTO-3 (1:10,000 dilution) was used to stain nuclei.

Fluorescence was visualized and images were captured using a Nikon immunofluorescence confocal microscope C1 at 10x and 60x magnifications using the 488-nm (green) 568-nm (red) and 633-nm (blue) laser lines for excitation of the appropriate fluorochromes. Raw data were imported using ImageJ.1.37v to select and generate Z-stacked multiple (2-3) slices, and Adobe Photoshop 9.0 to merge images captured upon excitation of fluorochromes obtained different channels.

The results of these experiments are provided in Table 6. Treatments that demonstrate utility in the mouse models of this invention are excellent candidates for testing against human disease conditions, at similar dosages and administration modalities.

EXAMPLE 22

EFFECTS OF LOADING CONDITIONS ON NUCLEIC ACID LOADING AND PARTICLE STABILITY

The effects of various loading conditions, including ethanol concentration, time, temperature, and nucleic acid:lipid ratio, on oligonucleotide loading and vesicle stability were determined.

Effect of ethanol concentration on oligonucleotide loading and vesicle stability

The presence of ethanol during the encapsulation process is needed to facilitate lipid rearrangement and encapsulation of the polynucleotide. However, the amount of ethanol required varies for different lipid compositions as too high of a concentration of ethanol can also lead to membrane instability.

The effect of using 32, 34 and 36 % ethanol to encapsulate a 16mer phosphodiester oligonucleotide (ODN) in DLinDMA/DSPC/CH/PEG-S-DMG (40:10:48:2 mole ratio) vesicles is shown in Figure 1A. After a 30 min incubation at 23°C, the 32 and 34 % ethanol-containing mixture resulted in 75 - 85 % encapsulation whereas the mixture containing 36 % ethanol had only 28 % encapsulation and this did not increase by 60 min (Figure 1A). The low encapsulation seen with the 36 % ethanol sample correlated with a large vesicle size increase as measured by quasi-elastic light scattering using a Nicomp particle sizer (Figure 1 B), suggesting that the vesicle membrane had destabilized and significant inter-vesicle fusion had occurred. This vesicle instability can also occur when the incubation time is extended to 60 min with the 32 and 34 % ethanol-containing samples (Figure 1 B) and correlated with a loss of ODN encapsulation (Figure 1A).

Effect of time and temperature on oligonucleotide loading vesicle stability

The effect of temperature on the extent and kinetics of ODN encapsulation was characterized using DLinDMA/DSPC/CH/PEG-S-DMG (40:10:42:8, mole ratio) vesicles. Vesicles were incubated with ODN at an initial ratio of 0.06 (wt/wt) in 50 mM citrate, pH 4 buffer containing 34 % ethanol. Using an incubation temperature of 30°C, a maximum encapsulation of 70 % was obtained at 30 min after which the encapsulation efficiency remained unchanged within the error of the measurements (Figure 2A). At 40°C, 80 - 90 % encapsulation efficiency was observed over a 15 to 60 min time course (Figure 2A). Changes in vesicle size were also monitored by quasi-elastic light scattering. At both 30 (data not shown) and 40°C (Figure 3B), the vesicle size remained stable.

Effect of siRNA to lipid ratio and the formulation process on encapsulation efficiency

The amount of siRNA that can be encapsulated by cationic lipid-containing vesicles (measured as the encapsulated siRNA to lipid ratio) can reach a saturation level for a given lipid composition and/or formulation process.

Using the pre-formed vesicle method (PFV), a maximum encapsulated siRNA to lipid ratio was observed at ~0.050 (wt/wt). As shown in Table 1, when an initial siRNA to lipid ratio of 0.061 (wt/wt) was used in the incubation mixture, a final encapsulated ratio of 0.049 was obtained with DLinDMA/DSPC/CH/PEG-S-DMG (40:10:40:10 mole ratio) vesicles, correlating to 80 % encapsulation. However, at a higher initial siRNA/lipid ratio of 0.244, a similar final encapsulated ratio of 0.052 was observed, correlating to 21 % encapsulation. The maximum siRNA/lipid ratio obtained can be limited by the amount of positive charge available to interact with the negatively charged backbone of the siRNA. However, at a siRNA/lipid ratio of 0.060 there is still a ~3-fold excess of positive to negative charge, suggesting that the encapsulation under these conditions is not limited by charge interactions.

A higher encapsulated siRNA/lipid ratio was obtained using an alternative formulation method ("classic method") as described in Semple, S.C. et al., Biochim Biophys Acta 1510:152-66 (2001) and Semple, S.C., et al., Methods Enzymol 313:322-41 (2000). Briefly, instead of incubating cationic vesicles with the siRNA to induce lipid rearrangement and siRNA encapsulation (the PFV method), the lipids are solubilized in 100 % ethanol and added directly to an aqueous solution containing the siRNA at pH 4 (34 % ethanol final). Using this method, a progressive increase in encapsulated

siRNA/lipid ratio was observed when higher incubation siRNA/lipid ratios were used (Table 1). At initial siRNA/lipid ratios of 0.060 and 0.120, nearly complete encapsulation was observed; however, at an initial siRNA/lipid ratio of 0.240, only 61 % encapsulation was obtained suggesting that a plateau was being reached. This plateau may reflect saturation in the positive charges (*i.e.,* cationic lipid) available to interact with the anionic backbone of the siRNA. The 0.147 siRNA/lipids ratio (wt/wt) obtained (Table 3) is near the theoretical charge neutralization ratio of 0.178.

Using the PFV technique, the siRNA/lipid ratio was not increased by increasing the mole% of cationic lipid in a formulation composed of DLinDMA/CH/PEG-S-DMG, and at 70 mole% DLinDMA the siRNA to lipid ratio was significantly reduced from that obtained at 50 and 60 mole% DLinDMA (Table 4).

Table 3

| Formulation method | siRNA/lipid ratio (wt/wt) | | % Encapsulation |
|---|---|---|---|
| | Initial | Final | |
| PFV | 0.061 | 0.049 | 80 % |
| PFV | 0.244 | 0.052 | 21 % |
| Classic | 0.060 | 0.060 | 100 % |
| Classic | 0.120 | 0.113 | 94 % |
| Classic | 0.240 | 0.147 | 61 % |

Table 4

| Lipid composition | Lipid mole ratio | siRNA/lipid ratio (wt/wt) | | % Encapsulation |
|---|---|---|---|---|
| | | Initial | Final | |
| DLinDMA/CH/PEG-S-DMG | 50:40:10 | 0.077 | 0.040 | 52 |
| DLinDMA/CH/PEG-S-DMG | 60:28:12 | 0.089 | 0.044 | 50 |
| DLinDMA/CH/PEG-S-DMG | 70:16:14 | 0.089 | 0.028 | 31 |

## EXAMPLE 23

### EFFECT OF CATIONIC LIPID ON PHARMACOKINETICS, BIODISTRIBUTION, AND BIOLOGICAL ACTIVITY OF NUCLEIC ACID-LIPID PARTICLES

The effect of different cationic lipid formulations on the *in vivo* characteristics of various nucleic acid-lipid particles was examined in using the Factor VII siRNA in C57BL/6 mice, essentially as described in Example 21. The various lipid compositions tested are described in Table 5.

Formulations were generated at a nominal lipid ratio of 40/10/40/10 (mol% aminolipid/DSPC/Chol/PEG-S-DMG). Cy-3 fluorescence in plasma, liver and spleen was assessed as described in Example 21. In general, with a few exceptions, formulations with the lowest plasma levels and highest liver levels of Cy-3 fluorescence at 0.5 h post-IV injection showed the highest activity in the Factor VII model when formulated with a FVII-specific siRNA. The results of these studies are summarized in Table 6.

The ability of various lipid formulations to knockdown Factor VII expression was determined in a liver model using Factor VII siRNA, in order to evaluate the impact of aminolipid linker chemistry. The structure of the headgroup in each lipid was the same. Each formulation was generated at a nominal lipid ratio of 40/10/40/10 (mol% aminolipid/DSPC/Chol/PEG-S-DMG). Samples were injected intravenously into C57BL/6 mice at the doses indicated in Figure 3. Factor VII levels in serum were measured against control mice at 24 hours post-injection. The dose (mg/kg) to achieve 50% Factor VII reduction was improved approximately 10-fold using the ketal linkage (DLin-K-DMA lipid) as compared with the ether linkage (DLinDMA lipid), and approximately 100-fold as compared with the ester linkage (DLinDAP lipid; Figure 3).

Table 5 - Formulation Characteristics of Novel Lipid Formulations Tested In Vivo.

| Lipid Composition[1] | Nominal siRNA-to-Lipid Ratio (wt/wt)[2] | Incubation Conditions | Diafiltration Buffer | Formulation Characteristics | | | |
|---|---|---|---|---|---|---|---|
| | | | | Particle Size (nm) | Final siRNA-to-Lipid Ratio (wt/wt) | Encapsulation (%) | siRNA Recovery (%) |
| **DLinTMA**/DSPC/Chol/PEG-S-DMG | 0.061 | RT/15 min | PBS | 185 ± 72 | NA | NA | 84 |
| **DLinTAP**/DSPC/Chol/PEG-S-DMG | 0.060 | RT/15 min | PBS | 127 ± 41 | NA | NA | 81 |
| **DOTAP**/DSPC/Chol/PEG-S-DMG | 0.060 | 31°C130 min | PBS | 71 ± 22 | NA | NA | 47 |
| **DODMA**/DSPC/Chol/PEG-S-DMG | 0.062 | 31°C/30 min | PBS | 66 ± 17 | 0.054 | 99 | 77 |
| **DLinDMA**/DSPC/Chol/PEG-S-DMG | 0.063 | 31°C/30 min | PBS | 72 ± 23 | NA | NA | 77 |
| **DLinDAC**/DSPC/Chol/PEG-S-DMG | 0.061 | RT/30 min | PBS | 74 ± 26 | NA | NA | 60 |
| **DLin-C-DAP**/DSPC/Chol/PEG-S-DMG | 0.060 | 31°C/30 min | PBS | 73 ± 25 | NA | NA | 58 |
| **DLin-2-DMAP**/DSPC/Chol/PEG-S-DMG | 0.062 | 31°C/30 min | PBS | 79 ± 26 | NA | NA | 42 |
| **DLin-S-DMA**/DSPC/Chol/PEG-S-DMG | 0.062 | 31°C/30 min | PBS | 69 ± 25 | NA | NA | 46 |
| **DLinMA**/DSPC/Chol/PEG-S-DMG | 0.061 | 31°C/30 min, pH3 | PBS | 78 ± 35 | NA | NA | 46 |
| **DLinAP**/DSPC/Chol/PEG-S-DMG | 0.064 | 31°C/30 min | PBS | 75 ± 40 | NA | NA | 32 |
| **DLinDAP**/DSPC/Chol/PEG-S-DMG | 0.061 | 31°C/30 min | HBS | 73 ± 35 | NA | NA | NA |

(continued)

| Lipid Composition[1] | Nominal siRNA-to-Lipid Ratio (wt/wt)[2] | Incubation Conditions | Diafiltration Buffer | Formulation Characteristics | | | |
|---|---|---|---|---|---|---|---|
| | | | | Particle Size (nm) | Final siRNA-to-Lipid Ratio (wt/wt) | Encapsulation (%) | siRNA Recovery (%) |
| **DLin-EG-DMA**/DSPC/Chol/PEG-S-DMG | 0.061 | 31°C/30 min | PBS | 76 ± 27 | NA | NA | 64 |
| **DLinMPZ**/DSPC/Chol/PEG-S-DMG | 0.061 | 31°C/30 min | PBS | 75 ± 20 | 0.061 | 103 | 88 |
| **DLin-K-DMA**/DSPC/Chol/PEG-S-DMG | 0.062 | RT/30 min | PBS | 73 ± 20 | 0.060 | 100 | 76 |

1 The nominal lipid ratio (mol%) for each formulation was 40/10/40/10 (mol% aminolipid/DSPC/Chol/PEG-S-DMG)
2 The nominal siRNA-to-lipid ratio expressed as μg siRNA/μmol total lipid was 0.0466 unless otherwise noted; variation on a wt/wt basis results from different molecular weights of the different aminolipids

Table 6 - Pharmacokinetics, Biodistribution and Activity of Selected Novel Lipid Formulations Tested In Vivo.

| Lipid Composition[1] | Plasma Cy3 Concentration (μg equiv/mL) | | Liver Cy3 Concentration (% Injected Dose) | | Spleen Cy3 Concentration (% Injected Dose) | | Factor VII Activity |
|---|---|---|---|---|---|---|---|
| | 0.5 | 3 h | 0.5 h | 3 h | 0.5 h | 3 h | |
| **DLin-K-DMA**/DSPC/Chol/PEG-S-DMG | 1.1 | 0.4 | 32.0 | 4.0 | ND | ND | +++++++ |
| **DLinDMA**/DSPC/Chol/PEG-S-DMG | 15.3 | 0.7 | 50.0 | 17.0 | 0.79 | 0.17 | ++++ |
| **DLinMPZ**/DSPC/Chol/PEG-S-DMG | 20.3 | 0.4 | 52.0 | 37.5 | 1.53 | 0.15 | +++ |
| **DLinDAC**/DSPC/Chol/PEG-S-DMG | 27.1 | 0.3 | 29.0 | 6.5 | 0.23 | 0.13 | ++ |
| **DLin-2-DMAP**/DSPC/Chol/PEG-S-DMG | 17.5 | 8.8 | 20.5 | 2.5 | 0.34 | 0.11 | ++ |
| **DLinAP**/DSPC/Chol/PEG-S-DMG | 86.2 | 23.1 | 11.5 | 5.0 | 0.37 | 0.24 | ++ |
| **DLin-C-DAP**/DSPC/Chol/PEG-S-DMG | 69.4 | 19.0 | 28.5 | 13.5 | 0.79 | 0.12 | + |
| **DLin-S-DMA**/DSPC/Chol/PEG-S-DMG | 10.7 | 5.4 | 2.5 | 0.0 | 0.02 | 0.04 | + |
| **DLinMA**/DSPC/Chol/PEG-S-DMG | 20.2 | 0.4 | 10.5 | 4.5 | 0.12 | 0.32 | + |
| **DLinDAP**/DSPC/Chol/PEG-S-DMG | 46.6 | 3.3 | 20.5 | 16.5 | 0.74 | 0.22 | + |

[1]Factor VII scoring system based on < 50% Factor VII knockdown at the following doses: +, 25 mg/kg; ++, 12.5 mg/kg, +++, 5 mg/kg; ++++, 2 mg/kg; +++++, 0.8 mg/kg; ++++++, 0.32 mg/kg; +++++++, 0.128 mg/kg

EXAMPLE 24

EFFECT OF CATIONIC LIPID ON TOLERABILITY AND THERAPEUTIC INDEX OF NUCLEIC ACID-LIPID PARTICLES

Various nucleic acid-lipid formulations were prepared as outlined in Example 20. The nominal lipid ratios for each formulation was 40/10/40/10 (mol% aminolipid/DSPC/Chol/PEG-S-DMG), and the nominal siRNA-to-lipid ratio was 0.0466 (μg siRNA siRNA/μmol total lipid). Mortality/morbidity, weight change and alanine aminotransferase (ALT; a plasma marker for liver damage), were measured for various siRNA doses (2, 5, 12.5 and 25 mg/kg) at 24 hours post-IV injection in C57BL/6 mice. Formulations were sorted based on mean weight loss at a 25 mg/kg siRNA dose. The results of these studies are summarized in Table 7.

Therapeutic index estimates were determined for certain formulations by deriving from $ED_{50}$ values in Factor VII dose response curves (e.g., Example 23, Figure 2) and tolerability assessments to determine maximum tolerated doses (MTDs). The MTD for these formulations was set as the lowest dose to cause ≥7% weight loss, a 200-fold increase in ALT and no severe clinical signs. The results of these studies are shown in Table 8.

Table 7 - Tolerability Information for Novel Lipid Formulations - Body Weight, Liver Enzymes, Mortality.

| Formulation | Δ Body Weight (%) | | | | Δ ALT[1] (-fold increase) | | | | Comments/Mortalities |
|---|---|---|---|---|---|---|---|---|---|
| | 2 mg/kg | 5 mg/kg | 12.5 mg/kg | 25 mg/kg | 2 mg/kg | 5 mg/kg | 12.5 mg/kg | 25 mg/kg | |
| **DLinTMA**/DSPC/Chol/PEG-S-DMG | +0.1 | -4.6 | **DEAD** | **DEAD** | NC | NC | **DEA D** | **DEA D** | |
| **DLinAP**/DSPC/Chol/PEG-S-DMG | +6.9 | +4.7 | **-7.5** | **DEAD** | NC | NC | **4** | **DEA D** | |
| **DLinMPZ**/DSPC/Chol/PEG-S-DMG | +2.3 | +4.0 | **-0.7** | **-14.7** | NC | NC | **3** | **183** | Sick at 25 mg/kg |
| **DODMA**/DSPC/Chol/PEG-S-DMG | +3.8 | -0.7 | **-12.8** | **-10.3** | NC | NC | **159** | **384** | 1 death (n=3) at 25 mg/kg |
| **DLin-K-DMA**/DSPC/Chol/PEG-S-DMG | +2.9 | +1.4 | **-10.4** | **-9.1** | NC | NC | **192** | **200** | Sick at 25 mg/kg. 3 deaths at 25 mg/kg (n=12) |
| **DLin-C-DA**P/DSPC/Chol/PEG-S-DMG | +6.2 | +7.1 | +1.4 | **-9.0** | NC | NC | **3** | **562** | Sick at 25 mg/kg |
| **DLinDMA**/DSPC/Chol/PEG-S-DMG | +4.2 | +2.9 | **-10.2** | **-8.6** | NC | NC | **209** | **587** | High dose is 18.75. Multiple deaths ≥ 18.75 |
| **DLin-S-DMA**/DSPC/Chol/PEG-S-DMG | +5.2 | +4.9 | +2.2 | **-8.5** | NC | NC | **2** | **68** | |
| **DLin-EG-DMA**/DSPC/Chol/PEG-S-DMG | +2.9 | +2.5 | +0.2 | **-4.6** | NC | NC | **2** | **100** | |
| **DLin-2-DMAP**/DSPC/Chol/PEG-S-DMG | +2.0 | +3.7 | +3.0 | +6.5 | NC | NC | NC | NC | |
| **DLinMA**/DSPC/Chol/PEG-S-DMG | +2.6 | +1.9 | +2.1 | +1.2 | NC | NC | NC | 2 | |
| **DLinTAP**/DSPC/Chol/PEG-S-DMG | +2.3 | +0.2 | +3.1 | +0.9 | NC | NC | NC | NC | |
| **DOTAP**/DSPC/Chol/PEG-S-DMG | +2.9 | +4.4 | +2.3 | +0.7 | NC | NC | NC | NC | High dose is 17.5 |
| DLinDAC/DSPC/Chol/PEG-S-DMG | +9.0 | +4.3 | +6.0 | +0.5 | NC | NC | NC | NC | |
| DLinDAP/DSPC/Chol/PEG-S-DMG | ND | +5.4 | -1.0 | +0.4 | ND | ND | NC | 2 | |

[1]Increase in ALT versus untreated control animals. NC = no change; ND = not done

Table 8: Therapeutic Index (TI) Comparison of Lipid Particle Formulations

| Lipid Composition | ED50 (mg/kg) | MTD (mg/kg) | TI |
|---|---|---|---|
| **DLinDAP**/DSPC/Chol/PEG-S-DMG | ~15 | >60 | >4.0 |
| **DLinDMA**/DSPC/Chol/PEG-S-DMG | ~1.0 | 12.5 | 12.5 |
| **DLin-K-DMA**/DSPC/Chol/PEG-S-DMG | ~0.1 | 15 | 150 |
| TI=MTD/ED50; ED50 = lowest dose to achieve 50% FVII knockdown. | | | |

EXAMPLE 25

ENHANCED TOLERABILITY OF LIPOSOMAL SIRNA FORMULATIONS COMPRISING PEG-C-DOMG

The activity and tolerability of liposomal formulations comprising various combinations of vationic lipid and PEG-lipid were tested. Liposomal formulations comprising either DLin-DMA or DLin-K-DMA in combination with PEG-S-DMG, PEG-c-DOMG, or PEG-DMA (also referred to as PEG-c-DMA) were prepared and evaluated as described in Examples 20 and 21. The composition and characteristics of the specific formulations evaluated is summarized in Table 9.

Table 9. Liposomal Formulations

| | | | | | | | |
|---|---|---|---|---|---|---|---|
| DP-0342 Summary (DLinDMA, PEG-s-DMG, PEG-c-DOMG, PEG-c-DMA) | | | | | | | |
| | | | | | | | |
| Sample | Particle Size | Total Lipid | Lipid Ratio | Total siRNA | Free siRNA | % Free | siRNA : Lipid |
| | (nm) | (mg/mL) | | (mg/mL) | (mg/mL) | siRNA | (Encapsulated siRNA) |
| Final A (PEG-s-DMG) | 67.5 | 117.05 | 39.0 : 10.4 : 41.1 : 9.6 | 5.902 | 0.029 | 0.5 | 0.050 |
| Final B (PEG-c-DOMG) | 70.4 | 80.72 | 38.0 : 10.7 : 41.2 : 10.1 | 4.022 | 0.138 | 3.4 | 0.048 |
| Final C (PEG-c-DMA) | 72.5 | 84.90 | 39.7 : 10.4 : 40.2 : 9.6 | 4.507 | 0.134 | 3.0 | 0.052 |
| | | | | | | | |
| | | | | | | | |
| | | | | | | | |
| DP-0343 Summary (DLin-K-DMA, PEG-s-DMG, PEG-c-DOMG, PEG-c-DMA) | | | | | | | |
| | | | | | | | |
| Sample | Particle Size | Total Lipid | Lipid Ratio | Total siRNA | Free siRNA | % Free | siRNA: lipid |
| | (nm) | (mg/mL) | | (mg/mL) | (mg/mL) | siRNA | (Encapsulated siRNA) |
| Final A (PEG-s-DMG) | 65.0 | 72.32 | 39.8 : 10.5 : 39.9 : 9.8 | 5.944 | 0.103 | 1.7 | 0.081 |

(continued)

| Sample | Particle Size | Total Lipid | Lipid Ratio | Total siRNA | Free siRNA | % Free | siRNA: lipid |
|---|---|---|---|---|---|---|---|
| | (nm) | (mg/mL) | | (mg/mL) | (mg/mL) | siRNA | (Encapsulated siRNA) |
| Final B (PEG-c-DMA) | 62.0 | 72.26 | 39.7 : 10.4 : 40.2 : 9.8 | 4.656 | 0.069 | 1.5 | 0.063 |
| Final C (PEG-c-DOMG) | 61.0 | 168.58 | 40.0 : 10.5 : 39.8 : 9.7 | 10.143 | 0.028 | 0.3 | 0.060 |

The ability of these various formulations to reduce FVII levels and their tolerability was examined as described in Example 21.

As shown in Figure 4, formulations comprising the cationic lipid DLin-DMA in combination with any of the PEG-lipids tested resulted in a similar dose-dependent reduction in FVII. Specifically, formulations comprising DLin-DMA had approximately equal ED50 for all three PEG-lipids tested.

In contrast, as shown in Figure 5, formulations comprising DLin-K-DMA showed greater activity in combination with PEG-C-DOMG, and lesser activity in combination with either PEG-S-DMG or PEG-DMA (PEG-C-DMA).

However, dramatic differences were observed in the toxicity of the various formulations. As shown in Figure 6, formulations comprising DLin-K-DMA were less toxic than equivalent DLin-DMA formulations, with the following rank order of toxicity:

DMA formulation: PEG-S-DMG » PEG-C-DMA > PEG-C-DOMG

KDMA formulation: PEG-S-DMG » PEG-C-DMA = PEG-C-DOMG. On further comparison, DLin-K-DMA formulations comprising PEG-C-DOMG exhibitied significantly greater tolerability than DLin-K-DMA formulations comprising PEG-S-DMG, as shown in Figures 7A and 7B.

## Claims

**1.** An amino lipid having the following structure (I):

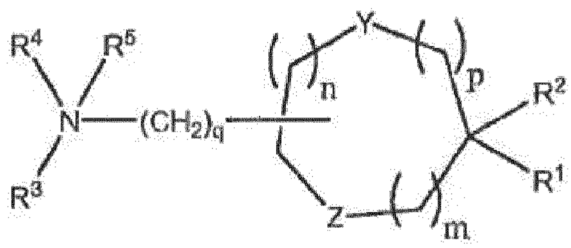

(I)

wherein

$R^1$ and $R^2$ are either the same or different and independently substituted $C_{12}$-$C_{24}$ alkyl, optionally substituted $C_{12}$-$C_{24}$ alkenyl, optionally substituted $C_{12}$-$C_{24}$ alkynyl, or optionally substituted $C_{12}$-$C_{24}$ acyl;

$R^3$ and $R^4$ are either the same or different and independently optionally substituted $C_1$-$C_6$ alkyl, optionally substituted $C_1$-$C_6$ alkenyl, or optionally substituted $C_1$-$C_6$ alkynyl or $R^3$ and $R^4$ may join to form an optionally substituted heterocyclic ring of 4 to 6 carbon atoms and 1 or 2 heteroatoms chosen from nitrogen end oxygen;

$R^5$ is either absent or present and when present is hydrogen $C_1$-$C_6$ alkyl;

m, n and p are either the same or different and independently either 0 or 1 with the proviso that m, n, and p are not simultaneously 0;

q is 0, 1, 2, 3, or 4; and

Y and Z are either the same or different and independently O, S or NH;

wherein the optional substituents are chosen from oxo, halogen, heterocycle, -CN,-$OR^x$, -$NR^xR^y$, -$NR^xC(=O)R^y$, -$NR^xSO_2R^y$, -$C(=O)R^x$, -$C(=O)OR^x$, -$C(=O)NR^xR^y$, -$SO_nR^x$ and-$SO_nNR^xR^y$, wherein n is 0, 1 or 2, $R^x$ and $R^y$ are the same or different and independently hydrogen, alkyl or heterocycle, and each or said alkyl and heterocycle substituents may be further substituted with one or more of oxo, halogen, -OH, -CN, alkyl, -$OR^x$, heterocycle,-$NR^xR^y$, $NR^xC(=O)R^y$, $NR^xSO_2R^y$, -$C(=O)R^x$, -$C(=O)OR^x$, -$C(=O)NR^xR^y$, -$SO_nR^x$ and -$SO_nNR^xR^y$;
wherein halogen is chosen from fluoro, chloro, bromo and iodo.

**2.** The amino lipid of claim 1, wherein the amino lipid has the structure:

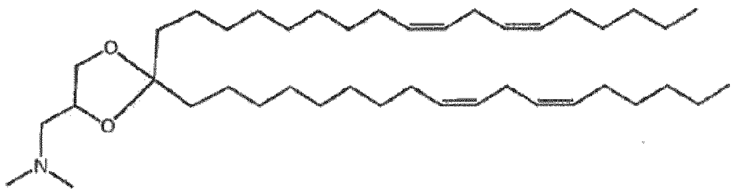

(DLin-K-DMA).

**3.** A lipid particle comprising an amino lipid of any one of claims 1-2, preferably comprising the amino lipid of claim 2.

**4.** The lipid particle of claim 3, wherein the particle further comprises a neutral lipid and a lipid capable of reducing particle aggregation, wherein preferably the particle further comprises essentially of:

(i) DLin-K-DMA;
(ii) a neutral lipid selected from DSPC, POPC, DOPE, and SM;
(iii) cholesterol; and
(iv) PEG-S-DMG, PEG-C-DOMG or PEG-DMA;

in a molar ratio of 20-60% DLin-K-DMA : 5-25% neutral lipid : 25-55% Chol: 0.5-1 5% PEG-S-DMG, PEG-C-DOMG or PEG-DMA.

**5.** A lipid particle, wherein the lipid particle comprises:

(i) one or more amino lipids;
(ii) one or more neutral lipids selected from DSPC, POPC, DOPE, and SM;
(iii) cholesterol; and
(iv) PEG-C-DOMG,

in a molar ratio of 20-60% amino lipid : 5-25% neutral lipid : 25-55% cholesterol : 0.5-15% PEG-C-DOMG, wherein the amino lipid is an amino lipid of any one of claims 1-2.

**6.** The lipid particle of any one of claims 3-5, further comprising a therapeutic agent, wherein preferably the therapeutic agent is a nucleic acid, which is further preferably a plasmid, an imunostimulatory oligonucleotide, or is selected from the group constisting of: a siRNA, a microRNA, an antisense oligonucleotide, and a ribozyme, preferably a siRNA.

**7.** A pharmaceutical composition comprising a lipid particle of claim 6 and a pharmaceutically acceptable excipient, carrier, or diluent.

**8.** The pharmaceutical composition of claim 7 for use in therapy.

**9.** The pharmaceutical composition of claim 7 for use in a method of treating a disease or disorder **characterized by** overexpression of a poypeptide in a subject, the method comprising providing to the subject the pharmaceutical composition, wherein the therapeutic agent is selected from a siRNA, a microRNA, an antisense oligonucleotide, a plasmid capable of expressing a siRNA, a microRNA, and an antisense oligonucleotide, and wherein the siRNA,

microRNA, or antisense RNA comprises a polynucleotide that specifically binds to a polynucleotide that encodes the polypeptide, or a complement thereof.

**10.** The pharmaceutical composition of claim 7 for use in a method of treating a disease or disorder **characterized by** underexpression of a polypeptide in a subject, the method comprising providing to the subject the pharmaceutical composition, wherein the therapeutic agent is a pasmid that encodes the polypeptide or a functional variant or fragment thereof.

**11.** The pharmaceutical composition of claim 7 for use in a method of inducing an immune response in a subject comprising providing to the subject the pharmaceutical composition 7, wherein the therapeutic agent is an immunostimulatory oligonucleotide.

**12.** The pharmaceutical composition for use in claim 11, wherein the pharmaceutical composition is suitable to be provided to the patient in combination with a vaccine or antigen.

**13.** A vaccine comprising the lipid particle of claim 6, wherein the therapeutic agent is an immunostimulatory oligonucleotide and an antigen associated with a disease or pathogen.

**14.** The vaccine of claim 13, wherein said antigen is a tumor antigen, a viral antigen, a bacterial antigen, or a parasitic antigen.

**15.** The lipid particle of claim 6 for use in therapy, preferably for use in modulating the expression of a polypeptide by a cell, wherein optionally and preferably:

a) the therapeutic agent is selected from a siRNA, a microRNA, an antisense oligonucleotide, a plasmid capable of expressing a siRNA, a microRNA, and an antisense oligonucleotide, and wherein the siRNA, microRNA, or antisense RNA comprises a polynucleotide that specifically binds to a polynucleotide that encodes the polypeptide, or a complement thereof, such that the expression of the polypeptide is reduced; or
b) the nucleic acid is a plasmid that encodes the polypeptide or a functional variant or fragment thereof, such that expression of the polypeptide or the functional variant or fragment thereof is increased.

## Patentansprüche

**1.** Aminolipid mit der folgenden Struktur (I):

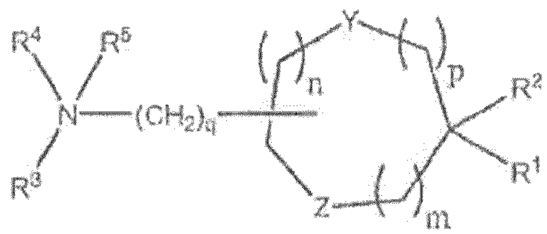

(I)

wobei

$R^1$ und $R^2$ entweder dasselbe oder jeweils ein unterschiedliches und unabhängig substituiertes $C_{12}$-$C_{24}$-Alkyl, optional substituiertes $C_{12}$-$C_{24}$-Alkenyl, optional substituiertes $C_{12}$-$C_{24}$-Alkynyl oder optional substituiertes $C_{12}$-$C_{24}$-Acyl sind;
$R^3$ und $R^4$ entweder dasselbe oder jeweils ein unterschiedliches und unabhängig optional substituiertes $C_1$-$C_6$-Alkyl, optional substituiertes $C_1$-$C_6$-Alkenyl oder optional substituiertes $C_1$-$C_6$-Alkynyl sind oder $R^3$ und $R^4$ sich verbinden können, um einen optional substituierten heterocyclischen Ring aus 4 bis 6 Kohlenstoffatomen und 1 oder 2 aus Stickstoff und Sauerstoff ausgewählten Heteroatomen zu bilden;
$R^5$ entweder fehlt oder Wasserstoff oder $C_1$-$C_6$-Alkyl ist;

m, n und p entweder dasselbe oder unterschiedlich und unabhängig entweder 0 oder 1 sind mit der Maßgabe, dass m, n, und p nicht gleichzeitig 0 sind;
q 0, 1, 2, 3, oder 4 ist; und
Y und Z entweder dasselbe oder verschieden und unabhängig O, S oder NH sind;

wobei die optionalen Substituenten aus Oxo, Halogen, Heterocyclus, -CN, $-OR^x$,$-NR^xR^y$, $-NR^xC(=O)R^y$, $-NR^XSO_2R^y$, $-C(=O)R^x$, $-C(=O)OR^x$, $-C(=O)NR^xR^y$, $-SO_nR^x$ und - $SO_nNR^xR^y$ ausgewählt werden, wobei n 0, 1 oder 2 ist, $R^x$ und $R^y$ dasselbe oder verschieden und unabhängig Wasserstoff, Alkyl oder Heterocyclus sind und jeder von besagten Alkyl- und Heterocyclus-Substituenten weiter mit einem oder mehreren aus Oxo, Halogen, -OH, - CN, Alkyl, $-OR^x$, Heterocyclus, $-NR^xR^y$, $NR^xC(=O)R^y$, $NR^xSO_2R^y$, $-C(=O)R^x$, $-C(=O)OR^x$,$-C(=O)NR^xR^y$, $-SO_nR^x$ und $-SO_nNR^xR^y$ substituiert werden kann;
wobei Halogen aus Fluoro, Chloro, Bromo und Jodo ausgewählt wird.

**2.** Aminolipid nach Anspruch 1, wobei das Aminolipid die folgende Struktur aufweist:

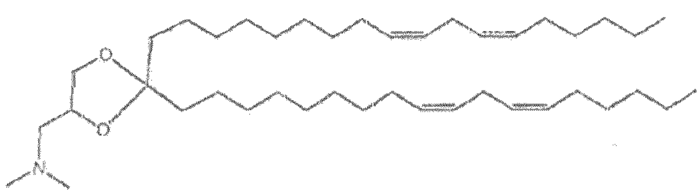

(DLin-K-DMA).

**3.** Lipidpartikel umfassend ein Aminolipid nach irgendeinem der Ansprüche 1-2, vorzugsweise umfassend das Aminolipid nach Anspruch 2.

**4.** Lipidpartikel nach Anspruch 3, wobei das Partikel ferner ein neutrales Lipid und ein Lipid, das zur reduzierenden Partikelaggregation imstande ist, umfasst, wobei vorzugsweise das Partikel ferner grundsätzlich Folgendes umfasst:

(i) DLin-K-DMA;
(ii) ein aus DSPC, POPC, DOPE und SM ausgewähltes neutrales Lipid;
(iii) Cholesterin; und
(iv) PEG-S-DMG, PEG-C-DOMG oder PEG-DMA;

in einem Molverhältnis von 20-60 % DLin-K-DMA:5-25 % neutrales Lipid:25-55 % Chol:0,5-15 % PEG-S-DMG, PEG-C-DOMG oder PEG-DMA.

**5.** Lipidpartikel, wobei das Lipidpartikel Folgendes umfasst:

(i) ein oder mehr Aminolipide;
(ii) ein oder mehr aus DSPC, POPC, DOPE und SM ausgewählte neutrale Lipide;
(iii) Cholesterin; und
(iv) PEG-C-DOMG,

in einem Molverhältnis von 20-60 % Aminolipid:5-25 % neutrales Lipid:25-55 % Cholesterin:0,5-15 % PEG-C-DOMG, wobei das Aminolipid ein Aminolipid nach irgendeinem der Ansprüche 1-2 ist.

**6.** Lipidpartikel nach irgendeinem der Ansprüche 3-5, ferner umfassend ein Therapeutikum, wobei vorzugsweise das Therapeutikum eine Nukleinsäure ist, welche ferner vorzugsweise ein Plasmid oder ein immunstimulierendes Oligonukleotid ist oder aus der aus Folgendem bestehenden Gruppe ausgewählt wird: einer siRNA, einer mikroRNA, einem Antisense-Oligonukleotid und einem Ribozym, vorzugsweise einer siRNA.

**7.** Pharmazeutische Zusammensetzung umfassend ein Lipidpartikel nach Anspruch 6 und einen pharmazeutisch annehmbaren Hilfsstoff, Träger oder Verdünner.

**8.** Pharmazeutische Zusammensetzung nach Anspruch 7 zur therapeutischen Verwendung.

**9.** Pharmazeutische Zusammensetzung nach Anspruch 7 zur Verwendung bei einem Verfahren zur Behandlung einer Krankheit oder Störung, die durch Überexpression eines Polypeptids bei einem Probanden gekennzeichnet ist, wobei das Verfahren die Versorgung des Probanden mit der pharmazeutischen Zusammensetzung umfasst, wobei das Therapeutikum aus einer siRNA, einer mikroRNA, einem Antisense-Oligonukleotid und einem Plasmid mit Befähigung zur Expression einer siRNA, einer mikroRNA und eines Antisense-Oligonukleotids ausgewählt wird und wobei die siRNA, mikroRNA oder Antisense-RNA ein Polynukleotid umfasst, das sich spezifisch an ein Polynukleotid bindet, das das Polypeptid oder ein Komplement davon codiert.

**10.** Pharmazeutische Zusammensetzung nach Anspruch 7 zur Verwendung bei einem Verfahren zur Behandlung einer Krankheit oder Störung, die durch Unterexpression eines Polypeptids bei einem Probanden gekennzeichnet ist, wobei das Verfahren die Versorgung des Probanden mit der pharmazeutischen Zusammensetzung umfasst, wobei das Therapeutikum ein Plasmid ist, das das Polypeptid oder eine funktionelle Variante oder ein Fragment davon codiert.

**11.** Pharmazeutische Zusammensetzung nach Anspruch 7 zur Verwendung bei einem Verfahren zur Induzierung einer Immunantwort bei einem Probanden, umfassend die Versorgung des Probanden mit der pharmazeutischen Zusammensetzung 7, wobei das Therapeutikum ein immunstimulierendes Oligonukleotid ist.

**12.** Pharmazeutische Zusammensetzung zur Verwendung in Anspruch 11, wobei die pharmazeutische Zusammensetzung dafür geeignet ist, dem Patienten in Kombination mit einem Vakzin oder Antigen bereitgestellt zu werden.

**13.** Vakzin umfassend das Lipidpartikel nach Anspruch 6, wobei das Therapeutikum ein immunstimulierendes Oligonukleotid und ein mit einer Krankheit oder einem Pathogen assoziiertes Antigen ist.

**14.** Vakzin nach Anspruch 13, wobei besagtes Antigen ein Tumorantigen, ein virales Antigen, ein bakterielles Antigen oder ein parasitäres Antigen ist.

**15.** Lipidpartikel nach Anspruch 6 zur therapeutischen Verwendung, vorzugsweise zur Verwendung beim Modulieren der Expression eines Polypeptids durch eine Zelle, wobei optional und vorzugsweise:

a) das Therapeutikum aus einer siRNA, einer mikroRNA, einem Antisense-Oligonukleotid und einem Plasmid mit Befähigung zur Expression einer siRNA, einer mikroRNA und eines Antisense-Oligonukleotids ausgewählt wird und wobei die siRNA, mikroRNA oder Antisense-RNA ein Polynukleotid umfasst, das sich spezifisch an ein Polynukleotid bindet, das das Polypeptid oder ein Komplement davon codiert, sodass die Expression des Polypeptids reduziert wird; oder
b) die Nukleinsäure ein Plasmid ist, das das Polypeptid oder eine funktionelle Variante oder ein Fragment davon codiert, sodass die Expression des Polypeptids oder der funktionellen Variante oder des Fragments davon erhöht wird.

**Revendications**

**1.** Lipide aminé présentant la structure suivante (I) :

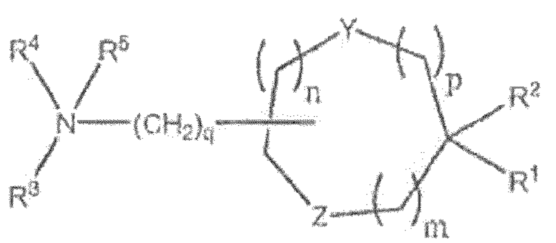

(I)

dans laquelle

$R^1$ et $R^2$ sont identiques ou différents et indépendamment un groupe alkyle en $C_{12}$-$C_{24}$ substitué, un groupe alcényle en $C_{12}$-$C_{24}$ éventuellement substitué, un groupe alcynyle en $C_{12}$-$C_{24}$ éventuellement substitué, ou un groupe acyle en $C_{12}$-$C_{24}$ éventuellement substitué ;
$R^3$ et $R^4$ sont identiques ou différents et indépendamment un groupe alkyle en $C_1$-$C_6$ éventuellement substitué, un groupe alcényle en $C_1$-$C_6$ éventuellement substitué, ou un groupe alcynyle en $C_1$-$C_6$ éventuellement substitué, ou $R_3$ et $R_4$ peuvent se joindre pour former un hétérocycle éventuellement substitué de 4 à 6 atomes de carbone et 1 ou 2 hétéroatomes choisis parmi l'azote et l'oxygène ;
$R^5$ est absent ou présent, et s'il est présent, est de l'hydrogène ou un groupe alkyle en $C_1$-$C_6$ ;
m, n et p sont identiques ou différents et valent indépendamment 0 ou 1, sous réserve que m, n et p ne valent pas simultanément 0 ;
q est 0,1,2,3 ou 4 ; et
Y et Z sont identiques ou différents et indépendamment O, S ou NH ;

dans laquelle les substituants facultatifs sont choisis parmi un groupe oxo, un halogène, un hétérocycle, -CN, -$OR^x$, -$NR^xR^y$, -$NR^xC(=O)R^y$, -$NR^xSO_2R^y$, -$C(=O)R^x$,-$C(=O)OR^x$, -$C(=O)NR^xR^y$, -$SO_nR^x$ et - $SO_nNR^xR^y$, où n est 0, 1 ou 2, $R^x$ et $R^y$ sont identiques ou différents et indépendamment de l'hydrogène, un groupe alkyle ou un hététerocycle, et ledit ou chacun desdits substituants alkyles et hétérocycles peut être davantage substitué par un ou plusieurs groupes choisis parmi un groupe oxo, un halogène, -OH, -CN, un groupe alkyle, -$OR^x$, un hétérocycle, - $NR^xR^y$, $NR^xC(=O)R^y$, $NR^xSO_2R^y$, -$C(=O)R^x$, -$C(=O)OR^x$, -$C(=O)NR^xR^y$, -$SO_nR^x$ et-$SO_nNR^xR^y$ ;
dans laquelle l'halogène est choisi parmi un groupe fluoro, chloro, bromo et iodo.

**2.** Lipide aminé selon la revendication 1, présentant la structure :

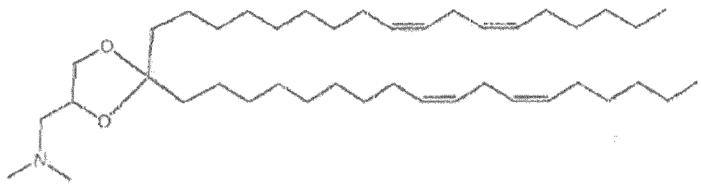

(DLin-K-DMA).

**3.** Particule lipidique comprenant un lipide aminé selon l'une quelconque des revendications 1 et 2, comprenant de préférence le lipide aminé selon la revendication 2.

**4.** Particule lipidique selon la revendication 3, la particule comprenant en outre un lipide neutre et un lipide capable de réduire l'agrégation de particules, la particule consistant de préférence en :

(i) DLin-K-DMA ;
(ii) un lipide neutre choisi parmi DSPC, POPC, DOPE et SM ;
(iii) du cholestérol ; et
(iv) PEG-S-DMG, PEG-C-DOMG ou PEG-DMA ;

selon un rapport molaire de 20 à 60 % de DLin-K-DMA sur 5 à 25 % de lipide neutre sur 25 à 55 % de cholestérol sur 0,5 à 15 % de PEG-S-DMG, PEG-C-DOMG ou PEG-DMA.

**5.** Particule lipidique comprenant :

(i) un ou plusieurs lipides aminés ;
(ii) un ou plusieurs lipides neutres choisis parmi DSPC, POPC, DOPE et SM ;
(iii) du cholestérol ; et
(iv) PEG-C-DOMG,

selon un rapport molaire de 20 à 60 % de lipide aminé sur 5 à 25 % de lipide neutre sur 25 à 55 % de cholestérol sur 0,5 à 15 % de PEG-C-DOMG, le lipide aminé étant un lipide aminé selon l'une quelconque des revendications 1 et 2.

**6.** Particule lipidique selon l'une quelconque des revendications 3 à 5, comprenant en outre un agent thérapeutique,

l'agent thérapeutique étant de préférence un acide nucléique, qui est de préférence également un plasmide, un oligonucléotide immunostimulatoire ou choisi dans le groupe constitué d'un ARNic, d'un microARN, d'un oligonucléotide antisens et d'un ribozyme, de préférence un ARNic.

**7.** Composition pharmaceutique comprenant une particule lipidique selon la revendication 6 et un excipient, un support ou un diluant pharmaceutiquement acceptable.

**8.** Composition pharmaceutique selon la revendication 7, destinée à être utilisé dans une thérapie.

**9.** Composition pharmaceutique selon la revendication 7 destinée à être utilisée dans un procédé de traitement d'un maladie ou d'un trouble **caractérisé par** une surexpression d'un polypeptide chez un sujet, le procédé consistant à fournir au sujet la composition pharmaceutique, dans laquelle l'agent thérapeutique est choisi parmi un ARNic, un microARN, un oligonucléotide antisens, un plasmide capable d'exprimer un ARNic, un microARN et un oligonucléotide antisens, et dans laquelle l'ARNic, le microARN ou l'ARN antisens comprend un polynucléotide qui se lie de manière spécifique à un polynucléotide codant le polypeptide, ou un complément correspondant.

**10.** Composition pharmaceutique selon la revendication 7, destinée à être utilisée dans un procédé de traitement d'une maladie ou d'un trouble **caractérisé par** la sous-expression d'un polypeptide chez un sujet, le procédé consistant à administrer au sujet la composition pharmaceutique, dans laquelle l'agent thérapeutique est un plasmide codant le polypeptide ou une variante ou un fragment fonctionnel correspondant.

**11.** Composition pharmaceutique selon la revendication 7, destinée à être utilisée dans un procédé d'induction d'une réponse immunitaire chez un sujet consistant à administrer au sujet la composition pharmaceutique selon la revendication 7, dans laquelle l'agent thérapeutique est un oligonucléotide immunostimulatoire.

**12.** Composition pharmaceutique destinée à être utilisée selon la revendication 11, la composition pharmaceutique étant appropriée pour être administrée au patient en combinaison avec un vaccin ou un antigène.

**13.** Vaccin comprenant la particule lipidique selon la revendication 6, dans lequel l'agent thérapeutique est un oligonucléotide immunostimulatoire et un antigène associé à une maladie ou un pathogène.

**14.** Vaccin selon la revendication 13, dans lequel ledit antigène est un antigène tumoral, un antigène viral, un antigène bactérien ou un antigène parasitaire.

**15.** Particule lipidique selon la revendication 6 destinée à être utilisée dans un traitement thérapeutique, destinée à être utilisée de préférence dans la modulation de l'expression d'un polypeptide par une cellule, dans laquelle éventuellement et de préférence :

l'agent thérapeutique est choisi parmi un ARNic, un microARN, un oligonucléotide antisens, un plasmide capable d'exprimer un ARNic, un microARN et un oligonucléotide antisens, et dans lequel l'ARNic, le microARN ou l'ARN antisens comprend un polynucléotide qui se lie de manière spécifique à un polynucléotide codant le polypeptide, ou un complément correspondant, de telle sorte que l'expression du polypeptide soit réduite ; ou l'agent thérapeutique est un plasmide qui code le polypeptide, ou une variante ou un fragment fonctionnel correspondant, de telle sorte que l'expression du polypeptide ou de la variante ou du fragment fonctionnel correspondant soit augmentée.

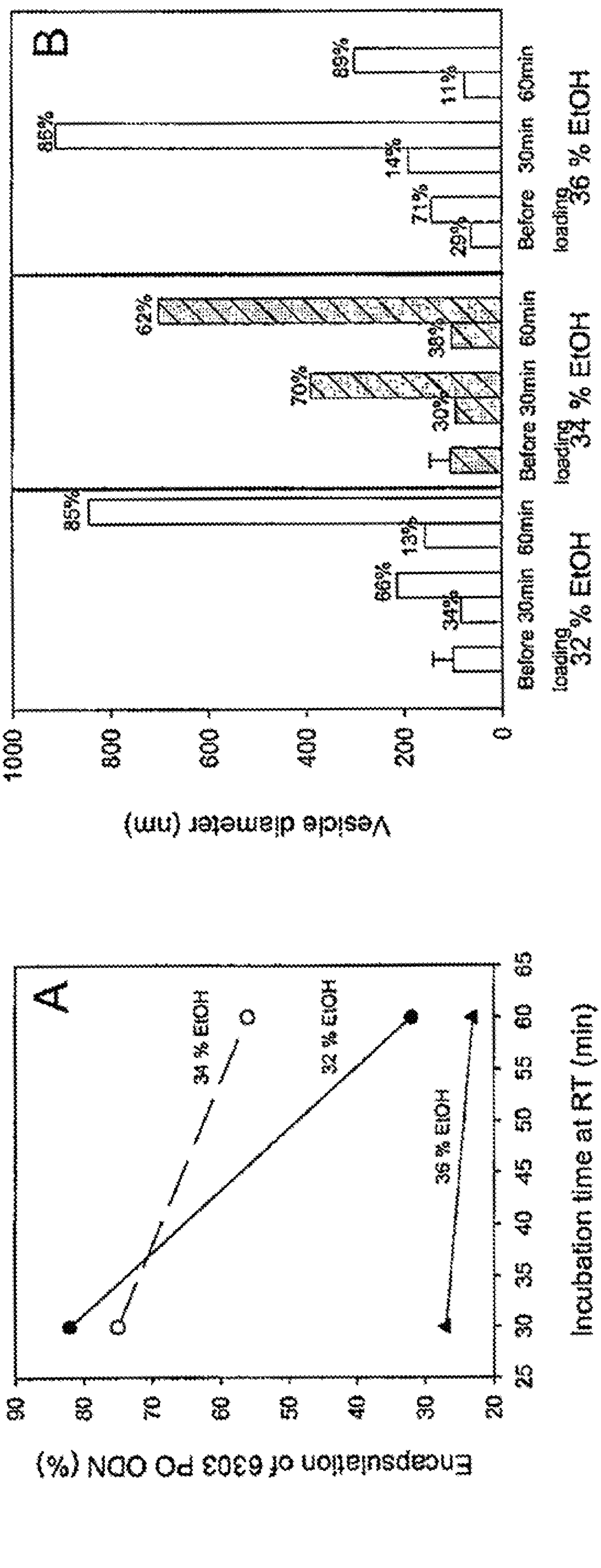
A
Encapsulation of 6303 PO ODN (%)
90
80
70
60
50
40
30
20
25
30
35
40
45
50
55
60
65
Incubation time at RT (min)
34 % EtOH
32 % EtOH
36 % EtOH
B
Vesicle diameter (nm)
1000
800
600
400
200
0
34%
66%
13%
85%
30%
70%
38%
62%
29%
71%
14%
85%
11%
89%
Before loading
30min
60min
32 % EtOH
Before loading
30min
60min
34 % EtOH
Before loading
30min
60min
36 % EtOH

*FIG. 1*

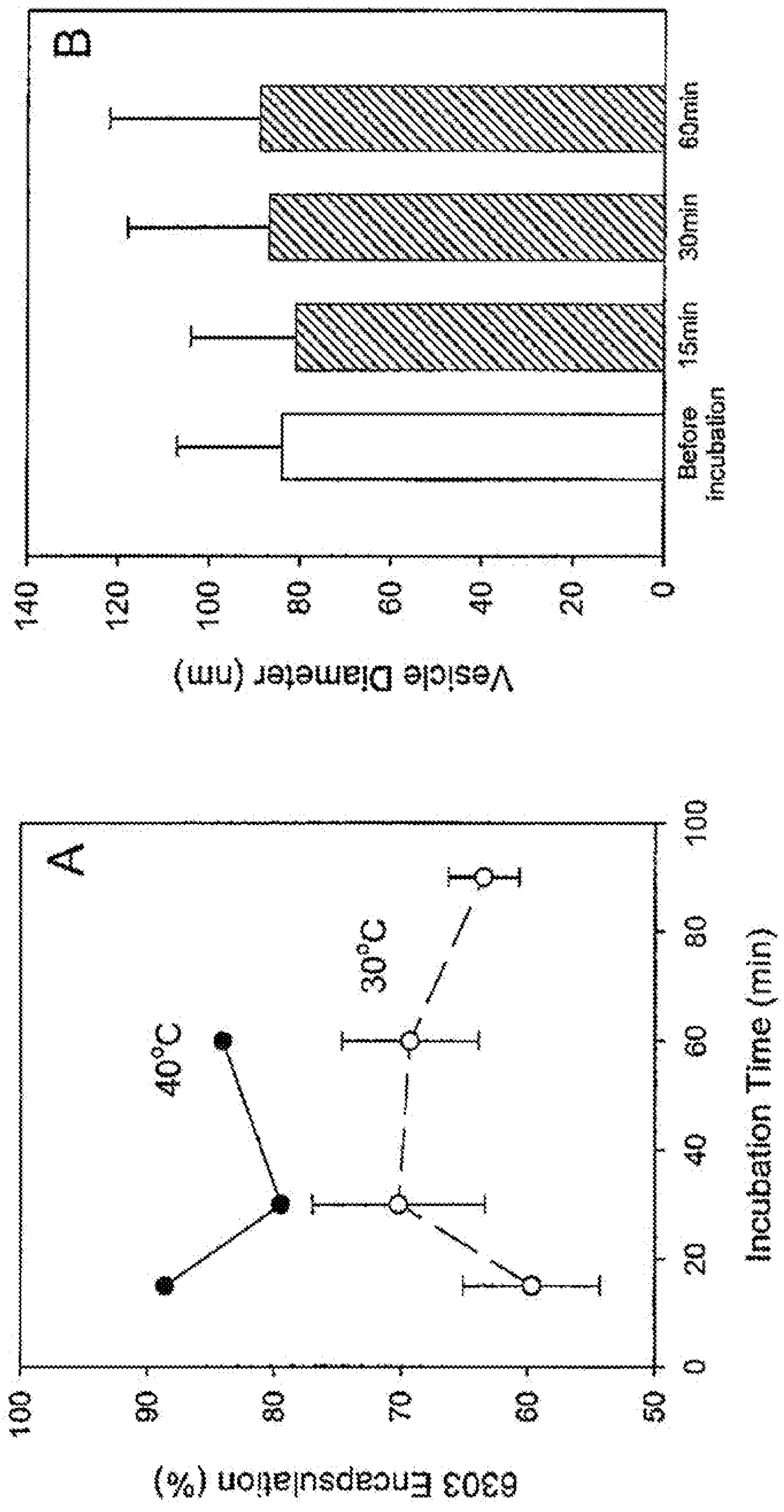
A
40°C
30°C
6303 Encapsulation (%)
Incubation Time (min)
100
90
80
70
60
50
0
20
40
60
80
100
B
Vesicle Diameter (nm)
140
120
100
80
60
40
20
0
Before incubation
15min
30min
60min

FIG. 2

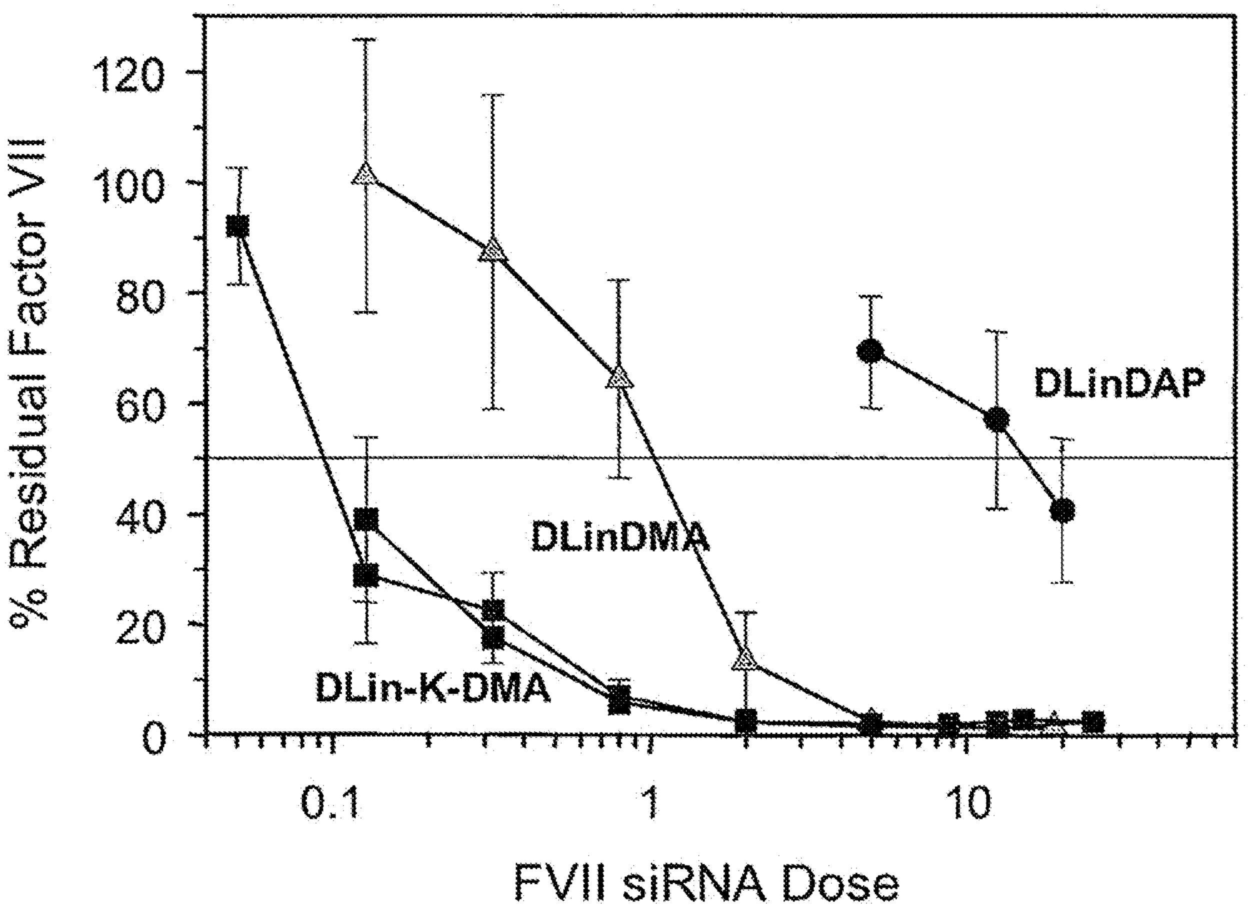
% Residual Factor VII
120
100
80
60
40
20
0
0.1
1
10
FVII siRNA Dose
DLinDAP
DLinDMA
DLin-K-DMA

*FIG. 3*

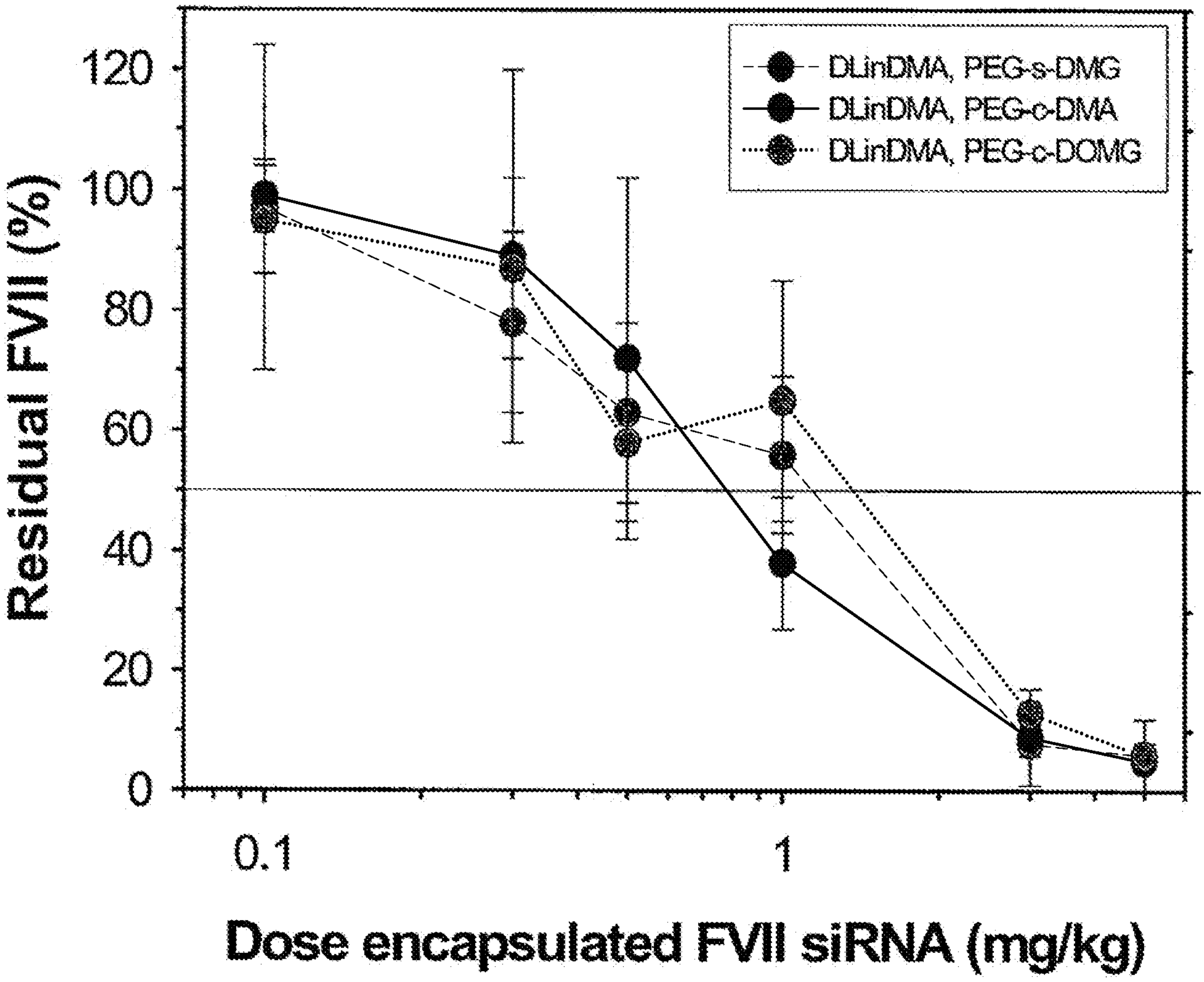
DLinDMA, PEG-s-DMG
DLinDMA, PEG-c-DMA
DLinDMA, PEG-c-DOMG
Residual FVII (%)
120
100
80
60
40
20
0
0.1
1
Dose encapsulated FVII siRNA (mg/kg)

*FIG. 4*

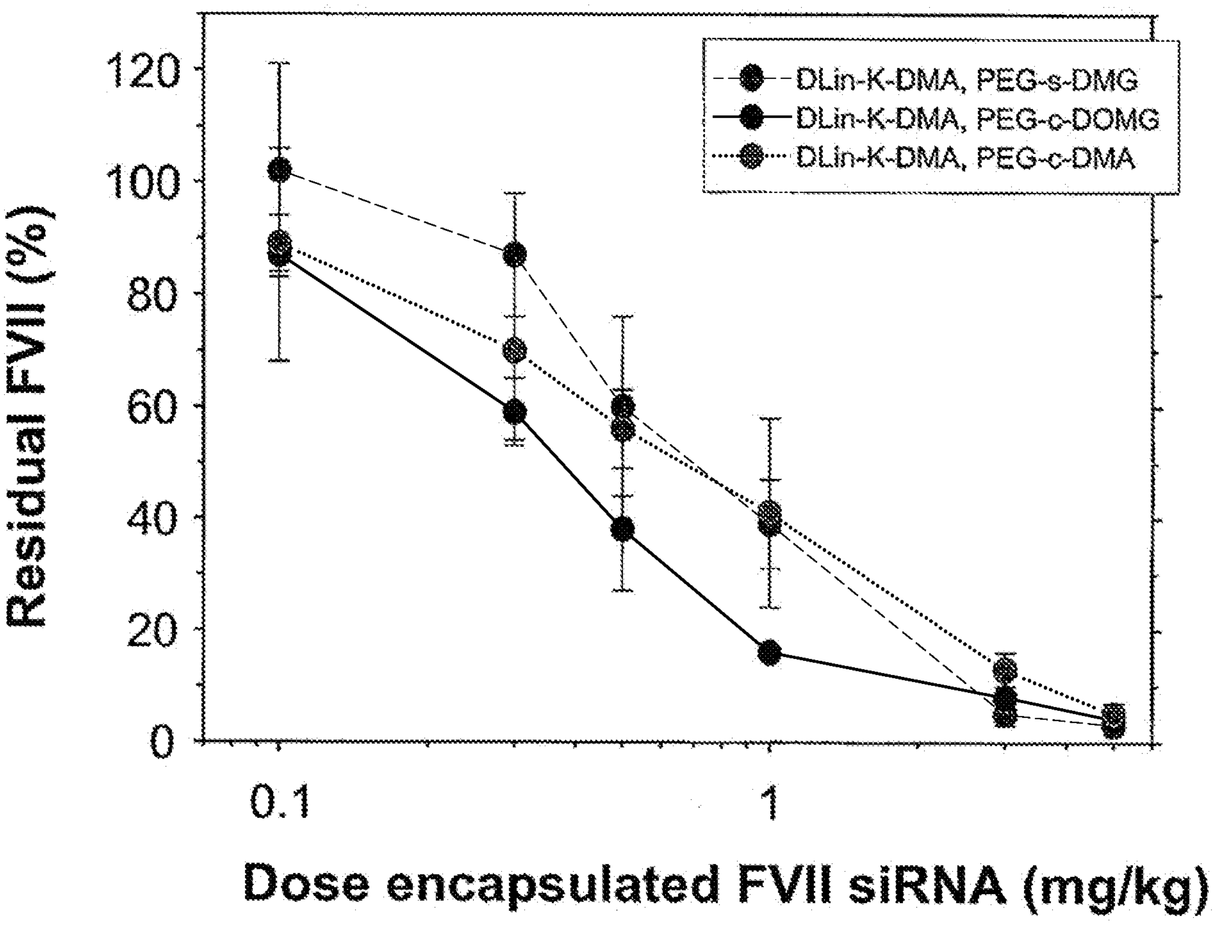
DLin-K-DMA, PEG-s-DMG
DLin-K-DMA, PEG-c-DOMG
DLin-K-DMA, PEG-c-DMA
Residual FVII (%)
0
20
40
60
80
100
120
0.1
1
Dose encapsulated FVII siRNA (mg/kg)

*FIG. 5*

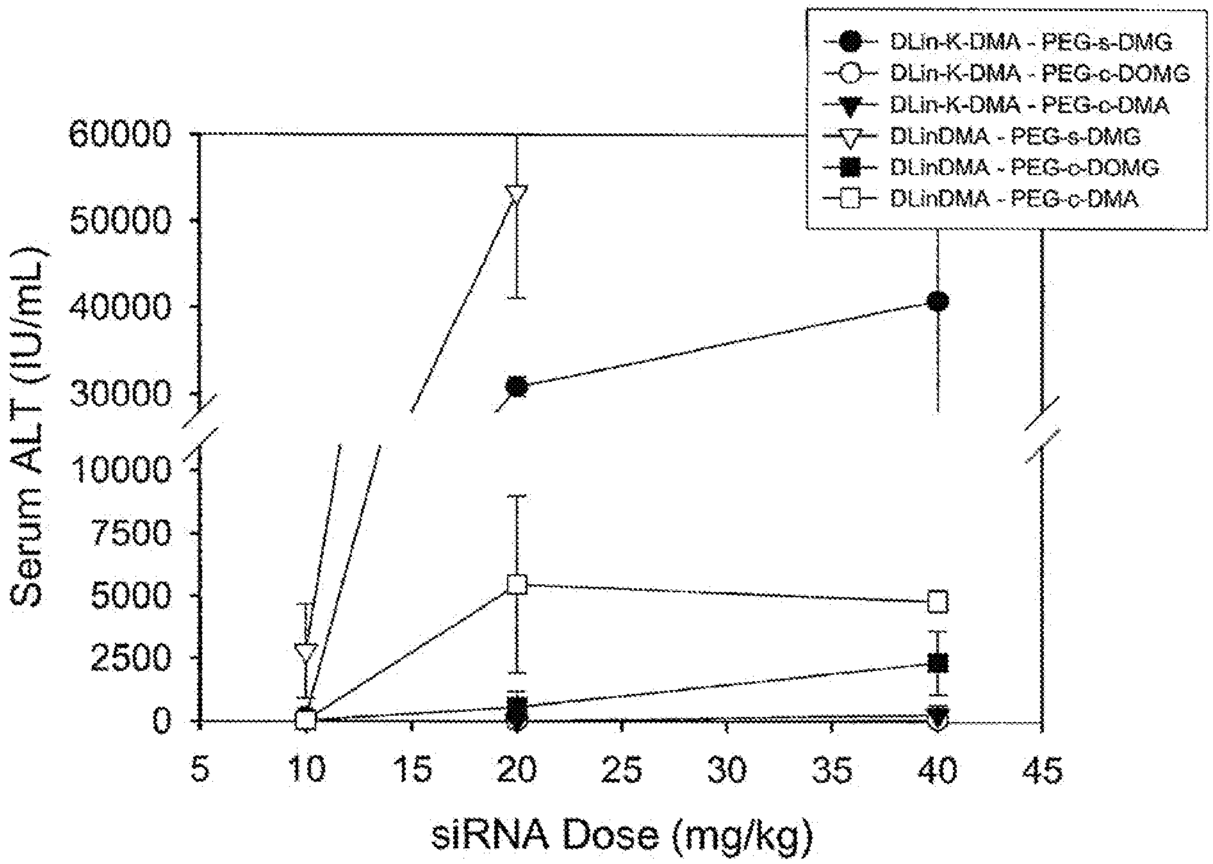
DLin-K-DMA - PEG-s-DMG
DLin-K-DMA - PEG-c-DOMG
DLin-K-DMA - PEG-c-DMA
DLinDMA - PEG-s-DMG
DLinDMA - PEG-c-DOMG
DLinDMA - PEG-c-DMA
Serum ALT (IU/mL)
60000
50000
40000
30000
10000
7500
5000
2500
0
5
10
15
20
25
30
35
40
45
siRNA Dose (mg/kg)

*FIG. 6*

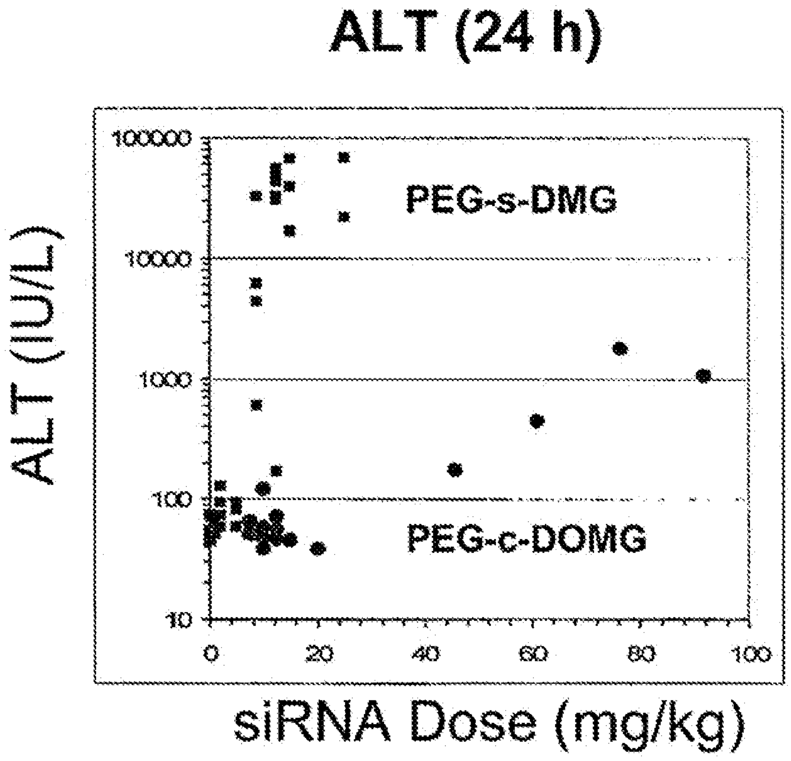
ALT (24 h)
ALT (IU/L)
100000
10000
1000
100
10
0
20
40
60
80
100
PEG-s-DMG
PEG-c-DOMG
siRNA Dose (mg/kg)

FIG. 7A

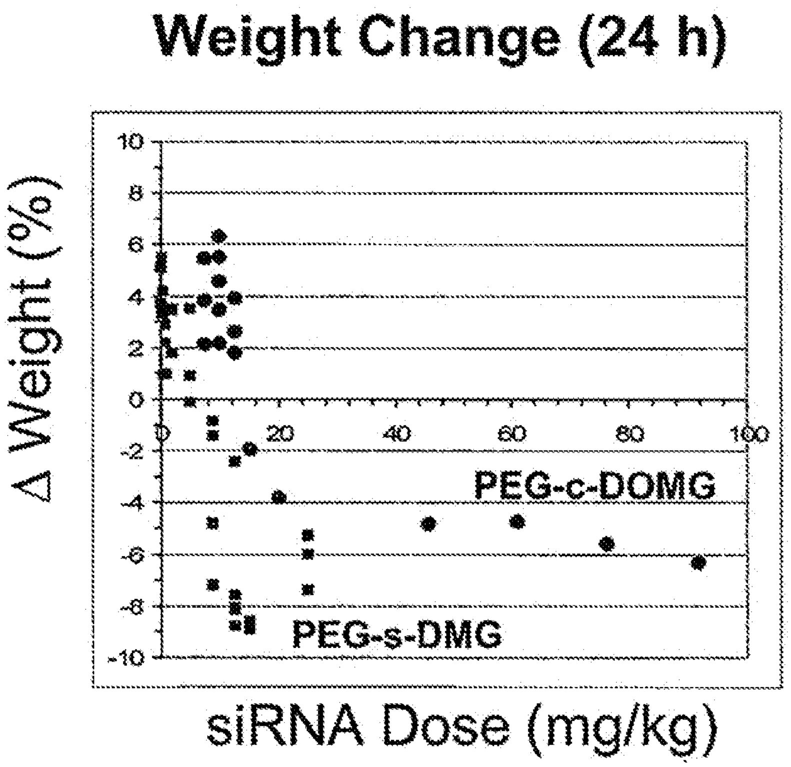
Weight Change (24 h)
Δ Weight (%)
10
8
6
4
2
0
-2
-4
-6
-8
-10
20
40
60
80
100
PEG-c-DOMG
PEG-s-DMG
siRNA Dose (mg/kg)

FIG. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5532130 A
- WO 2006074546 A1
- US 6320017 B
- US 5885613 A
- US 5820873 A
- US 5534499 A
- US 08486214 B
- US 486214 A
- US 4957773 A
- US 4603044 A
- US 5013556 A
- US 6027726 A
- US 5739119 A
- US 5759829 A
- US 5801154 A
- US 5789573 A
- US 5718709 A
- US 5610288 A
- US 5747470 A
- US 5591317 A
- US 5783683 A
- EP 0360257 A
- US 5631359 A
- US 4987071 A
- WO 9323569 A
- WO 9402595 A
- WO 9207065 A
- WO 9315187 A
- WO 9103162 A
- EP 92110298 A
- US 5334711 A
- WO 9413688 A
- WO 9611266 A
- US 379343 P
- US 649527 A
- WO 02069369 A
- WO 0115726 A
- US 6406705 B
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5489677 A
- US 5541307 A
- US 5561225 A
- US 5596086 A
- US 5602240 A
- US 5610289 A
- US 5608046 A
- US 5618704 A
- US 5623070 A
- US 5663312 A
- US 5633360 A
- US 5677437 A
- US 5677439 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A

- US 5552540 A
- US 5587469 A
- US 5594121 A
- US 5596091 A
- US 5614617 A
- US 5681941 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5700920 A
- US 5539082 A
- US 5714331 A
- US 5719262 A
- US 6287591 B
- US 6858225 B
- US 5976567 A
- US 4737323 A
- US 5286634 A, Stadler
- US 3993754 A, Rahman
- US 4145410 A
- US 4235871 A, Papahadjopoulos
- US 4224179 A, Schneider
- US 4522803 A, Lenk
- US 4588578 A, Fountain


**Non-patent literature cited in the description**


- **AGRAWAL.** *Trends in Biotech.,* 1996, vol. 14, 376-387
- **ZELPHATI, O. et al.** *Antisense. Res. Dev.,* 1993, vol. 3, 323-338
- **THIERRY, A.R. et al.** Gene Regulation: Biology of Antisense RNA and DNA. Raven Press, 1992, 147-161
- Antisense: Chemical Modifications. **UHLMANN E. et al.** Encyclopedia of Cancer. Academic Press Inc, 1997, vol. X, 64-81
- **VLASSOV et al.** *Biochim. Biophys. Acta,* 1994, vol. 1197, 95-1082
- **GALBRAITH et al.** *Antisense Nucl. Acid Drug Des.,* 1994, vol. 4, 201-206
- **ZELPHATI, O ; SZOKA, F.C.** *J. Contr. Rel.,* 1996, vol. 41, 99-119
- **ZIMMERMANN et al.** *Nature,* 2006, vol. 441, 111-114
- **GREEN, T.W.** PROTECTIVE GROUPS IN ORGANIC SYNTHESIS. Wiley-Interscience, 1999
- **SAPRA, P. ; ALLEN, TM.** *Prog. Lipid Res.,* 2003, vol. 42 (5), 439-62
- **ABRA, RM et al.** *J. Liposome Res.,* 2002, vol. 12, 1-3
- **ALLEN et al.** *Biochimica et Biophysica Acta,* 1995, vol. 1237, 99-108
- **DEFREES et al.** *Journal of the American Chemistry Society,* 1996, vol. 118, 6101-6104
- **BLUME et al.** *Biochimica et Biophysica Acta,* 1993, vol. 1149, 180-184
- **KLIBANOV et al.** *Journal of Liposome Research,* 1992, vol. 2, 321-334
- **ZALIPSKY.** *Bioconjugate Chemistry,* 1993, vol. 4, 296-299
- **ZALIPSKY.** *FEBS Letters,* 1994, vol. 353, 71-74
- **ZALIPSKY.** Stealth Liposomes. CRC Press, 1995
- **KIRPOTIN et al.** *FEBS Letters,* 1996, vol. 388, 115-118
- **RENNEISEN et al.** *J. Bio. Chem.,* 1990, vol. 265, 16337-16342
- **LEONETTI et al.** *Proc. Natl. Acad. Sci. (USA),* 1990, vol. 87, 2448-2451
- Covalent Attachment of Proteins to Liposomes. **HEATH.** Methods in Enzymology. Academic Press, Inc, 1987, vol. 149, 111-119
- **DE FOUGEROLLES, A. et al.** *Nature Reviews,* 2007, vol. 6, 443-453
- **LAMBERTON, J.S. ; CHRISTIAN, A.T.** *Molecular Biotechnology,* 2003, vol. 24, 111-119
- **ELSHABIR, S.M. et al.** *Nature,* 2001, vol. 411, 494-498
- **CAPLEN, N. et al.** *Proc. Natl. Acad. Sci. USA,* 2001, vol. 98, 9746-9747
- **BROWN, D. et al.** *TechNotes,* vol. 9 (1), 1-7
- **ELSHABIR, S.M. et al.** *EMBO,* 2001, vol. 20, 6877-6888
- **ELSHABIR, S. et al.** *Nature,* 2001, vol. 411, 494-498
- **ELSHABIR, S. et al.** *EMBO J.,* 2001, vol. 20, 6877-6888
- **PADDISON, P. et al.** *Genes Dev.,* 2002, vol. 16 (8), 948-58
- **PADDISON, P. et al.** *Proc. Natl. Acad. Sci. USA,* 2002, vol. 99 (3), 1443-1448
- **PADDISON et al.** *Genes & Dev.,* 2002, vol. 16 (8), 948-58

- **GRIFFITHS-JONES S ; GROCOCK RJ ; VAN DONGEN S ; BATEMAN A ; ENRIGHT AJ.** miRBase: microRNA sequences, targets and gene nomenclature. *NAR,* 2006, 34
- **GRIFFITHS-JONES S.** The microRNA Registry. *NAR,* 2004, vol. 32, D109-D111
- **JASKULSKI et al.** *Science,* 10 June 1988, vol. 240 (4858), 1544-6
- **VASANTHAKUMAR ; AHMED.** *Cancer Commun.,* 1989, vol. 1 (4), 225-32
- **PERIS et al.** *Brain Res Mol Brain Res.,* 15 June 1998, vol. 57 (2
- **ALTSCHUL et al.** *Nucleic Acids Res.,* 1997, vol. 25 (17), 3389-402
- **KIM ; CECH.** *Proc Natl Acad Sci USA.,* December 1987, vol. 84 (24), 8788-92
- **FORSTER ; SYMONS.** *Cell,* 24 April 1987, vol. 49 (2), 211-20
- **CECH et al.** *Cell,* December 1981, vol. 27 (3), 487-96
- **MICHEL ; WESTHOF.** *J Mol Biol.,* 05 December 1990, vol. 216 (3), 585-610
- **REINHOLD-HUREK ; SHUB.** *Nature,* 14 May 1992, vol. 357 (6374), 173-6
- **ROSSI et al.** *Nucleic Acids Res.,* 11 September 1992, vol. 20 (17), 4559-65
- **HAMPEL ; TRITZ.** *Biochemistry,* 13 June 1989, vol. 28 (12), 4929-33
- **HAMPEL et al.** *Nucleic Acids Res.,* 25 January 1990, vol. 18 (2), 299-304
- **PERROTTA ; BEEN.** *Biochemistry,* 01 December 1992, vol. 31 (47), 11843-52
- **GUERRIER-TAKADA et al.** *Cell,* December 1983, vol. 35 (3), 849-57
- **SAVILLE ; COLLINS.** *Cell,* 18 May 1990, vol. 61 (4), 685-96
- **SAVILLE ; COLLINS.** *Proc Natl Acad Sci USA.,* 01 October 1991, vol. 88 (19), 8826-30
- **COLLINS ; OLIVE.** *Biochemistry,* 23 March 1993, vol. 32 (11), 2795-9
- **YAMAMOTO S. et al.** *J. Immunol.,* 1992, vol. 148, 4072-4076
- **RANEY et al.** *Journal of Pharmacology and Experimental Therapeutics,* 2001, vol. 298, 1185-1192
- **KURRECK, J.** Antisense technologies. Improvement through novel chemical modifications. *Eur J Biochem,* 2003, vol. 270, 1628-44
- **KURRECK, J.** *Eur. J. Biochem.,* 2003, vol. 270, 1628-44
- **HALL et al.** *Nucleic Acids Res.,* 2004, vol. 32, 5991-6000
- **ZHANG, H.Y. ; DU, Q. ; WAHLESTEDT, C. ; LIANG, Z.** RNA Interference with chemically modified siRNA. *Curr Top Med Chem,* 2006, vol. 6, 893-900
- Antisense Research and Applications. CRC Press, Boca Raton, 1993, 276-278
- **MANOHARAN, M.** RNA interference and chemically modified small interfering RNAs. *Curr Opin Chem Biol,* 2004, vol. 8, 570-9
- **HOLEN, T. ; AMARZGUIOUI, M. ; BABAIE, E. ; PRYDZ, H.** Similar behaviour of single-strand and double-strand siRNAs suggests they act through a common RNAi pathway. *Nucleic Acids Res,* 2003, vol. 31, 2401-7
- **PRAKASH, T.P. ; ALLERSON, C.R. ; DANDE, P. ; VICKERS, T.A. ; SIOUFI, N. ; JARRES, R. ; BAKER, B.F. ; SWAYZE, E.E. ; GRIFFEY, R.H. ; BHAT, B.** Positional effect of chemical modifications on short interference RNA activity in mammalian cells. *J Med Chem,* 2005, vol. 48, 4247-53
- **MARTIN et al.** *Helv. Chim. Acta,* 1995, vol. 78, 486-504
- **NIELSEN et al.** *Science,* 1991, vol. 254, 1497-1500
- **KUNKEL et al.** *Brit. Med. Bull.,* 1989, vol. 45 (3), 630-643
- **GOODFELLOW.** *Nature,* 1989, vol. 341, 102-103
- **BENNETT et al.** *Mol. Pharm.,* 1992, vol. 41, 1023-1033
- **ZHU et al.** *Science,* 1993, vol. 261, 209-211
- **HYDE et al.** *Nature,* 1993, vol. 362, 250-256
- **BRIGHAM et al.** *Am. J. Med. Sci.,* 1989, vol. 298, 278-281
- METHODS IN ENZYMOLOGY. Academic Press, 1983, vol. 101, 512-527
- **MANNINO et al.** *Biotechniques,* 1988, vol. 6, 682-690
- **NICOLAU et al.** *Crit. Rev. Ther. Drug Carrier Syst.,* 1989, vol. 6, 239-271
- **BEHR.** *Acc. Chem. Res.,* 1993, vol. 26, 274-278
- **BRIGHAM et al.** *Am. J. Sci.,* 1989, vol. 298 (4), 278-281
- **CULVER.** Human Gene Therapy. MaryAnn Liebert, Inc., Publishers, 1994, 70-71
- **HEYES et al.** Synthesis and Charactierization of Novel Poly(ethylene glycol)-lipid Conjugates Suitable for Use in Drug Delivery. *J. Controlled Release,* 2006, vol. 112, 280-290
- **JOHN et al.** *Nature advance online publication,* 26 September 2007
- **MAURER et al.** *Biophys J.,* 2001
- **BLIGH et al.** *Can. J. Biochem. Physiol.,* 1959, vol. 37, 911-917
- **SEMPLE, S.C. et al.** *Biochim Biophys Acta,* 2001, vol. 1510, 152-66
- **SEMPLE, S.C. et al.** *Methods Enzymol,* 2000, vol. 313, 322-41